(19) Europäisches Patentamt
European Patent Office
Office européen des brevets

(11) **EP 3 578 984 A2**

(12) **EUROPEAN PATENT APPLICATION**

(43) Date of publication:
**11.12.2019 Bulletin 2019/50**

(51) Int Cl.:
***G01N 33/574*** (2006.01)

(21) Application number: **19183662.6**

(22) Date of filing: **28.10.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2013 JP 2013223738**

(62) Document number(s) of the earlier application(s) in accordance with Art. 76 EPC:
**14858507.8 / 3 064 940**

(71) Applicant: **Salivatech Co., Ltd.**
**Yamagata 997-0052 (JP)**

(72) Inventors:
• **Sugimoto, Masahiro**
**Tsuruoka-shi, Yamagata, 997-0017 (JP)**

• **Soga, Tomoyoshi**
**Tsuruoka-shi, Yamagata, 997-0017 (JP)**
• **Sunamura, Makoto**
**Tokyo, 160-8402 (JP)**

(74) Representative: **Grünecker Patent- und Rechtsanwälte**
**PartG mbB**
**Leopoldstraße 4**
**80802 München (DE)**

Remarks:
This application was filed on 01-07-2019 as a divisional application to the application mentioned under INID code 62.

(54) **SALIVARY BIOMARKERS FOR ORAL CANCER**

(57) Salivary biomarker characterized as low-molecular-weight compounds named metabolites or combinations these biomarkers are used for detecting cancers. As an example of a combination, creatinine, NI-acetylspermidine, α-aminoadipic acid, N-acetylneuraminic acid, and 1,3-diaminopropane is used. The use combination of these markers or single marker enable the early detection of pancreatic cancers, breast cancers, and oral cancers from a healthy subject regardness unstable concentration fluctuations of saliva.

EP 3 578 984 A2

**Description**

Technical Field

The present invention relates to salivary biomarkers for cancers, methods and devices for assaying the same, and methods for determining the salivary biomarkers for cancer. In particular, the present invention relates to salivary biomarkers for cancer that are suitably used for early detection of pancreatic cancer, intraductal papillary mucinous neoplasm (IPMN), breast cancer, and oral cancer, and methods and devices for assaying the same, and methods for determining the salivary biomarkers.

Background Art

A treatment for pancreatic cancer, which is intractable cancer, has not yet been sufficient, and the median survival year has not yet reached a year for a non-surgical excision example of pancreatic cancer, such as chemotherapy, radiotherapy, and the like. That is, in order to improve these treatment results of intractable cancer, cancer diagnosis at an early stage in which surgical excision is still possible is important. Thus, by using a biological sample(body fluid, etc.) that can be easily collected minimally or non-invasively, testing is frequently performed, and a method needs to be developed, which can detect cancer at an early stage or at a stage in which excision treatment is still possible at the latest.

In Patent Literature 1, one of the present inventors proposes a serum marker for determining a kidney disease. In Patent Literatures 2 and 3, he proposes a liver disease marker.

Additionally, for early pancreatic cancer detection, many procedures are also used, which detect a tumor diagnosis marker from blood (Patent Literatures 4 and 5). As a protein marker in blood related to cancer of a digestive system, carbohydrate antigen 19-9 (CA19-9) is used in a clinical field and is useful so as to detect pancreatic cancers and biliary tract cancers as well as to evaluate effects of chemotherapy. However, early cancer diagnosis is difficult, and the accuracy of screening cancer is insufficient (Non-Patent Literature 1). In addition, among negative people of the Lewis blood group system, there is a problem that false negatives are shown in which the level of CA19-9 is not elevated even if it is cancer. Additionally, detection of DUPAN-2 (pancreatic cancer associated antigen), CEA (CarcinoEmbryonic Antigen), and the like are also available. However, the former shows positive for biliary tract and liver cancers, and the latter also shows positive even for the cancers of the digestive system, such as esophageal cancer, gastric cancer, and the like. Therefore, these markers are not specific to pancreatic cancer. Further, these markers have not been widely used due to costs.

Polyamines, such as spermine (spermine), and acetylated polyamines, such as N8-acetylspermidine (N8-Acetylspermidine), N1-acetylspermidine (N1-Acetylspermidine), and N1-acetylspermine (N1-Acetylspermine) are known as markers for various cancers in blood and urine (Non-Patent Literature 2). In a metabolic pathway, arginine is metabolized to ornithine, and then metabolized through putrescine to polyamines. Biosynthesis of these is activated in a place in which a large amount of oxygen exists at a suface of a cancer tissue. In a hypoxic state at a center of cancer, uptake from outside of a cell increases. It is considered that as an entire cancer tissue, the concentration becomes high and flows into blood. For example, an increase in the concentration of spermidine in blood is known in breast cancers, prostate cancers and testis tumors (Non-Patent Literature 1). Furthermore, it is known through animal testing that the concentrations of spermine and spermidine in blood decrease in acute pancreatitis (Non-Patent Literature 3).

Citation List

Patent Literature

Patent Literature 1: Japanese Patent Application Laid-Open No. 2011-58863
Patent Literature 2: Japanese Patent Application Laid-Open No. 2011-232164
Patent Literature 3: WO2011/158590A1
Patent Literature 4: Japanese Patent Application Laid-Open No. 2011-247869
Patent Literature 5: Japanese Translation of PCT International Application No. 2009-508493
Patent Literature 6: Japanese Patent Application Laid-Open No. 2013-521763 Non-Patent Literature
Non-Patent Literature 1: Hamada S, Shimosegawa T., Biomarkers of pancreatic cancer, Pancreatology. 2011; 11: 14-9
Non-Patent Literature 2: Soda K (2011), The mechanisms by which polyamines accelerate tumor spread. Journal of Experimental & Clinical Cancer Research. 30(1): 95
Non-Patent Literature 3: Jin HT, Lamsa T, Merentie M, Hyvonen MT, Sand J, Raty S, Herzig KH, Alhonen L, Nordback I (2008). Polyamine levels in the pancreas and the blood change according to the severity of pancreatitis. Pancreatology. 8(1), 15-24

Non-Patent Literature 4: Zhang L, Farrell JJ, Zhou H, Elashoff D, Akin D, Park NH, Chia D, Wong DT. (2010), Salivary transcriptomic biomarkers for detection of resectable pancreatic cancer. Gastroenterology. 138(3): 949-57

Non-Patent Literature 5: Sugimoto M, Wong DT, Hirayama A, Soga T, Tomita M, (2010), Capillary electrophoresis mass spectrometry-based saliva metabolomics identified oral, breast and pancreatic cancer-specific profiles, Metabolomics, 6, 78-95

Non-Patent Literature 6: Soga, T., Baran, R., Suematsu M., Ueno, Y., Ikeda, S., Sakurakawa T., Kakazu, Y., Ishikawa, T., Robert, M., Nishioka, T., Tomita, M. (2006), Differential methabolomics reveals ophthalmic acid as an oxidative stress biomarker indicating hepatic glutathione sonsumption., Journal of Biological Chemistry, 281 (24): 16768-16776

Non-Patent Literature 7: Sugimoto et al. Capillary electrophoresis mass spectrometry-based saliva metabolomics identified oral, breast and pancreatic cancer-specific profiles, Metabolomics, 2010, 6, 78-95

Non-Patent Literature 8: Tsutsui et al, High-throughput LC-MS/MS based simultaneous determination of polyamines including N-acetylated forms in human saliva and the diagnostic approach to breast cancer patients, Anal Chem, 2013, 85, 11835-42

Non-Patent Literature 9: Wang Q et al. Investigation and identification of potential biomarkers in human saliva for the early diagnosis of oral squamous cell carcinoma., Clin Chim Acta. 2014; 427: 79-85

Summary of Invention

Technical Problem

Conventional detection of cancer using protein in blood is insufficient for screening for early pancreatic cancer detection. Additionally, although a blood test is a minimally invasive test, for a health examination or at a hospital, a blood sample is needed, which is collected by a healthcare professional, who uses a syringe. Thus, it is actually difficult to perform a test very frequently. In contrast, using saliva is non-invasive and does not cause any pain at all. There is an advantage that regardless of the location, even an individual subject can collect saliva and that it is possible to perform a test very frequently. For example, a salivary biomarker for detecting lung cancer isproposed in Patent Literature 6. In particular, it is said that early pancreatic cancer detection is difficult. It is important to detect a possibility of cancer promptly, using a test, which is performed much more frequently than a blood test.

As for a possibility of diagnosing pancreatic cancer by using saliva, Non-Patent Literature 4 discloses that pancreatic cancer can be diagnosed by using mRNA in saliva. Furthermore, there is also a report that pancreatic cancer can be diagnosed by using a metabolite in blood.

However, for mRNA quantitation, a step is needed in which an RNase inhibitor is added to saliva immediately after collecting saliva such that mRNA is not destroyed. Additionally, a complex step and time is required for measurement. Because of this, quantitative PCR is used for quantitation. However, in general, approximately several markers are only quantitated. In Non-Patent Literature 4 as well, 35 substances are only quantitated. Thus, trends of many molecules cannot be comprehensively and quantitatively understood by holding exhausitivity and quantitative property, and the saliva concentration cannot be corrected. Therefore, highly accurate prediction is impossible. Furthermore,, the step is complex, so there is a high possibility that artificial noise may be mixed with a quantitative value. In the past, there was no proposal that reports a marker search example with high reliability that also considers effects of a concentration fluctuation, which is generated in saliva, by minimizing a possibility of mixing noise with a quantitative value by means of a simpler processing, and comprehensively quantitating molecules. Additionally, a diagnosis method using such a salivary biomarker with high liability was not proposed, which was able to distinguish a healthy person from a person with cancer, particularly pancreatic cancer, intraductal papillary mucinous neoplasm (IPMN), breast cancer, and oral cancer.

The present invention was made to solve the above conventional problems. An object of the present invention is to be able to detect cancer, such as pancreatic cancer, breast cancer, oral cancer, and the like, at an early stage, using saliva.

Solution to the Problems

The present inventors identified multiple biomarkers that simultaneously quantitate low molecules (metabolites) in saliva of pancreatic cancer patients and discriminate pancreatic cancer patients from healthy controls by using CE-MS combining Capillary Electrophoresis (CE) and -Mass Spectrometry (MS) that can comprehensively measure the low molecules of biological samples. Additionally, they combined these and developed a discriminating method with higher accuracy than a single marker and also evaluated specificity by using saliva of cancer other than saliva of pancreatic cancer. A method of collecting saliva is made uniform, and the collection is performed by eliminating effects of diets and changes during the day, but even so, concentration variations cannot be completely eliminated. Thus, a marker was also searched, which suggests the total of metabolite concentrations, and algorithm was developed, which is combined with a marker that discriminates pancreatic cancer patients from healthy controls.

Further, a marker was found for breast cancer and oral cancer as well by the same method.

The present invention is made based on the aforementioned research results. The aforementioned problems are solved by a salivary biomarker for cancer whose feature is any of low molecule compounds that are metabolites of saliva samples, or the combination thereof.

Herein, the cancer may be any of pancreatic cancer, intraductal papillary mucinous neoplasm (IPMN), breast cancer, and oral cancer.

Furthermore, the following substances as the salivary biomarker for cancer that is used for detecting a pancreatic disease, or absolute concentrations of saliva of the combination thereof can be used: N-acetylputrescine (N-Acetylputrescine), adenosine (Adenosine), 3-phospho-D-glyceric acid (3PG), urea (Urea), o-acetylcarnitine (o-Acetylcarnitine), citric acid (Citrate), glycyl-glycine (Gly-Gly), 5-aminovaleric acid (5-Aminovalerate), 4-methyl 2-oxopentanoate (2-Oxoisopentanoate), malic acid (Malate), benzoate ester (Benzoate), fumaric acid (Fumarate), N-acetylaspartic acid (N-Acetylaspartate), inosine (Inosine), 3-methylhistidine (3-Methylhistidine), N1-acetylspermine (N1-Acetylspermine), creatine (Creatine), $\alpha$-aminoadipic acid (alpha-Aminoadipate), phosphorylcholine (Phosphorylcholine), 2-hydroxypentanoate (2-Hydroxypentanoate), xanthine (Xanthine), succinic acid (Succinate), 6-phosphogluconic acid (6-Phosphogluconate), butanoic acid (Butanoate), homovanillic acid (Homovanillate), O-phosphoserine (O-Phosphoserine), trimethylamine-N-oxide (Trimethylamine N-oxide), piperidine (Piperidine), cystine (Cystine), 2-isopropylmalic acid (2-Isopropylmalate), N8-acetylspermidine (N8-Acetylspermidine), N1-acetylspermidine (N1-Acetylspermidine), N-acetylneuraminic acid (N-Acetylneuraminate), glucosamine (Glucosamine), spermine (Spermine), agmatine (Agmatine), N-acetylhistamine (N-Acetylhistamine), methionine (Met), p-4-hydroxyphenylacetic acid (p-4-Hydroxyphenylacetate), N,N-dimethylglycine (N,N-Dimethylglycine), hypotaurine (Hypotaurine), glutamyl-glutamic acid (Glu-Glu), and N1,N12-diacetylspermine (N1,N12-Diacetylspermine).

Additionally, if a value is used that corrected a concentration in saliva, the following substances or the combination thereof can be used as a marker: N8-acetylspermidine (N8-Acetylspermidine), creatinine (Creatinine), spermine (Spermine), aspartic acid (Asp), N1-acetylspermidine (N1-Acetylspermidine), N1-acetylspermine (N1-Acetylspermine), cytidine (Cytidine), $\alpha$-aminoadipic acid (alpha-Aminoadipate), cytosine (Cytosine), betaine (Betaine), urea (Urea), homovanillic acid (Homovanillate), N-acetylneuraminic acid (N-Acetylneuraminate), cystine (Cystine), urocanic acid (Urocanate), fumaric acid (Fumarate), 1,3-diaminopropane (1,3-Diaminopropane), hypotaurine (Hypotaurine), nicotinic acid (Nicotinate), agmatine (Agmatine), valine (Val), 2-hydroxy-4-methylpentanoic acid (2-Hydroxy-4-methylpentanoate), alanyl-alanine (Ala-Ala), citric acid (Citrate), glucosamine (Glucosamine), carnosine (Carnosine), glycyl-glycine (Gly-Gly), 2-aminobutyric acid (2AB), arginine (Arg), N-acylglutamic acid (N-Acetylglutamate), glycerophosphoric acid (Glycerophosphate), phosphoenolpyruvic acid (PEP), isoleucine (Ile), adenosine (Adenosine), guanine (Guanine), dihydroxyacetonephosphoric acid (DHAP), and cadaverine (Cadaverine).

Additionally, as an example of a combination of the salivary biomarker for cancer that is used for detecting a pancreatic disease, prediction with high accuracy can be made by using a combination of creatinine, N1-acetylspermidine, $\alpha$-aminoadipic acid, N-acetylneuraminic acid and 1,3-diaminopropane. Prediction can also be made by using another combination or changing the methodology of the combination thereof.

Furthermore, the following substances as the salivary biomarker for cancer that is used for detecting breast cancer, or absolute concentrations of saliva of the combination thereof can be used. Choline (Choline), 2-hydroxybutyric acid (2-Hydroxybutyrate), $\beta$-alanine (beta-Ala), 3-methylhisdine (3-Methylhistidine), $\alpha$-aminobutyric acid (2AB), N-acetyl-$\beta$-alanine (N-Acetyl-beta-alanine), isethionic acid (Isethionate), N-acetylphenylalanine (N-Acetylphenylalanine), trimethyllysine (N6,N6,N6-Trimethyllysine), $\alpha$-aminoadipic acid (alpha-Aminoadipate), creatine (Creatine), $\gamma$-butyrobetaine (gamma-Butyrobetaine), sarcosine (Sarcosine), pyruvic acid (Pyruvate), urocanic acid (Urocanate), piperidine (Piperidine), serine (Ser), homovanillic acid (Homovanillate), 5-oxoproline (5-Oxoproline), GABA (GABA), 5-aminovaleric acid (5-Aminovalerate), trimethylamine-N-oxide (Trimethylamine N-oxide), 2-hydroxyvaleric acid (2-Hydroxyppentanoate), carnitine (Carnitine), isopropanolamine (Isopropanolamine), hypotaurine (Hypotaurine), lactic acid (Lactate), 2-hydroxy-4-methylpentanoic acid (2-Hydroxy-4-methylpentanoate), hydroxyproline (Hydroxyproline), butyric acid (Butanoate), adenine (Adenine), N6-acetyllysine (N-epsilon-Acetyllysine), 6-hydroxyhexanoic acid (6-Hydroxyhexanoate), propionic acid (Propionate), betaine (Betaine), N-acetylputrescine (N-Acetylputrescine), hypoxanthine (hypoxanthine), crotonic acid (Crotonate), tryptophan (Trp), citrulline (Citrulline), glutamine (Gln), proline (Pro), 2-oxoisopentanoic acid (2-Oxoisopentanoate), 4-methylbenzoate (4-Methylbenzoate), 3-(4-hydroxyphenyl)propionic acid (3-(4-Hydroxyphenyl)propionate), cysteic acid (Cysteate), azelaic acid (Azelate), ribulose-5-phosphoric acid (Ru5P), pipecolinic acid (Pipecolate), phenylalanine (Phe), O-phosphoserine (O-Phosphoserine), malonic acid (Malonate), hexanoic acid (Hexanoate), and p-hydroxyphenylacetic acid (p-Hydroxyphenylacetate).

The aforementioned substances that are significant substances and that are not publicly known are indicated in Table 7 below.

A combination of β-alanine, N-acetylphenylalanine, and citrulline can be used as one example of a combination of salivary biomarkers for cancer to detect breast cancer. The prediction can be performed by using a different combination or changing the methodology of combination.

When a value is used in which the concentration of saliva was corrected, the following substances or a combination thereof may be used as a marker: choline (Choline), β-alanine (beta-Ala), 3-methylhisdine (3-Methylhistidine), α-aminobutyric acid (2AB), N-acetyl-β-alanine (N-Acetyl-beta-alanine), isethionic acid (Isethionate), N-acetylphenylalanine (N-Acetylphenylalanine), trimethyllysine (N6,N6,N6-Trimethyllysine), urocanic acid (Urocanate), piperidine (Piperidine), 5-aminovaleric acid (5-Aminovalerate), trimethylamine-N-oxide (Trimethylamine N-oxide), isopropanolamine (Isopropanolamine), hypotaurine (Hypotaurine), hydroxyproline (Hydroxyproline), N6-acetyllysine (N-epsilon-Acetyllysine), 6-hydroxyhexanoic acid (6-Hydroxyhexanoate), N-acetylputrescine (N-Acetylputrescine), azelaic acid (Azelate), dihydroxyacetonephosphoric acid (DHAP), glycolic acid (Glycolate), 4-methyl-2-oxopentanoic acid (4-Methyl-2-oxopentanoate), N-acetylaspartic acid (N-Acetylaspartate), glycerophosphoric acid (Glycerophosphate), 3-hydroxybutyric acid (3-Hydroxybutyrate), benzoic acid (Benzoate), adipic acid (Adipate), 2-isopropylmalate (2-Isopropylmalate), phosphorylchlorine (Phosphorylcholine), N-acetylneuraminic acid (N-Acetylneuraminate), histamine (His), o-acetylcarnitine (o-Acetylcarnitine), N-acetylglucosamine 1-phosphate (N-Acetylglucosamine 1-phosphate), creatinine (Creatinine), arginine (Arg), and syringic acid (Syringate).

The aforementioned substances that are significant substances and that are not publicly known are indicated in Table 8 below.

A combination of N-acetylphenylalanine, N-acetylspermidine, and creatine can be used as one example of a combination of salivary biomarkers for cancer used to detect breast cancer. The prediction can be performed by using a different combination or changing the methodology of combination.

As the salivary biomarker for cancer used to detect oral cancer, the concentration of the following substances or a combination thereof in saliva can be used: Glycyl-glycine (Gly-Gly), citrulline (Citrulline), γ-butyrobetaine (gamma-Butyrobetaine), 3-phenyllactate (3-Phenyllactate), butyric acid (Butanoate), hexanoic acid (Hexanoate), methionine (Met), hypoxanthine (Hypoxanthine), spermidine (Spermidine), tryptophan (Trp), aspartic acid (Asp), isopropanolamine (Isopropanolamine), alanyl-alanine (Ala-Ala), N,N-dimethylglycine (N,N-Dimethylglycine), N1-acetylspermidine (N1-Acetylspermidine), N1-,N8-diacetylspermidine (N1,N8-Diacetylspermidine), N8-acetylspermidine (N8-Acetylspermidine), α-aminobutyric acid (2AB), trimethylamine-N-oxide (Trimethylamine N-oxide), N-acetylaspartic acid (N-Acetylaspartate), adenine (Adenine), 2-hydroxyvaleric acid (2-Hydroxyppentanoate), putrescine (Putrescine (1,4-Butanediamine)), 3-phosphoglycerate (3PG), 3-phenylpropionic acid (3-Phenylpropionate), serine (Ser), 1-methylnicotinamide (1-Methylnicotineamide), 3-hydroxy-3-methylglutaric acid (3-Hydroxy-3-methylglutarate), guanine (guanine), 3-(4-hydroxyphenyl)propionic acid (3-(4-Hydroxyphenyl)propionate), 4-methylbenzoate (4-Methylbenzoate), ribulose-5-phosphoric acid (Ru5P), α-aminoadipic acid (alpha-Aminoadipate), N6-acetyllysine (N-epsilon-Acetyllysine), glucosamine (Glucosamine), cystine (Cystine), carnosine (Carnosine), urocanic acid (Urocanate), phenylalanine (Phe), 2-deoxyribose-1-phosphoric acid (2-Deoxyribose 1-phosphate), cytidine disodium 5'-monophosphate (CMP), p-hydroxyphenylacetic acid (p-Hydroxyphenylacetate), polyhydroxybutyric acid (3-Hydroxybutyrate), N-acetylputrescine (N-Acetylputrescine), 7-methylguanine (7-Methylguanine), inosine (Inosine), lysine (Lys), dihydroxyacetonephosphoric acid (DHAP), 3-methylhisdine (3-Methylhistidine), carbamoylaspartic acid (Carbamoylaspartate), creatinine (Creatinine), N-methyl-2-pyrrolidone (1-Methyl-2-pyrrolidinone), pyruvic acid (Pyruvate), propionic acid (Propionate), 5-aminovaleric acid (5-Aminovalerate), N-acetylornithine (o-Acetylornithine), 5-oxoproline (5-Oxoproline), creatine (Creatine), homoserine (Homoserine), fumaric acid (Fumarate), glycine (Gly), and N1,N12-diacetylspermine (N1,N12-Diacetylspermine).

The aforementioned substances that are not publicly known are indicated in Table 9 below.

The present invention provides a method for assaying a salivary biomarker for cancer including the steps of: collecting a saliva sample; and detecting the aforementioned salivary biomarker for cancer in the collected saliva sample.

The present invention provides a device for assaying a salivary biomarker for cancer including means for collecting a saliva sample, and means for detecting the aforementioned salivary biomarker for cancer in the collected saliva sample.

The present invention further provides a method for determining a salivary biomarker for cancer including a procedure of performing ultrafiltration of a saliva sample, means for cyclopedically measuring ionic metabolites in the saliva sample after the ultrafiltration, and a procedure of selecting a substance having high ability of distinguishing a patient with a pancreatic disease from a healthy subject according to concentrations of the measured metabolites.

Here, when the salivary biomarker for cancer is selected, correlation values of the measured metabolites are calculated, and a concentration of each substance can be normalized, using a concentration of a substance that is correlated to the largest number of substances.

A combination of the salivary biomarkers for cancer can be determined using a mathematical model.

Advantageous Effects of Invention

According to the present invention, not only pancreatic cancer but also a pancreatic disease including IPMN and chronic pancreatitis, breast cancer, oral cancer, and the like can be detected early, using saliva that can be collected non-invasively and simply. In particular, a combination of polyamine with another novel substance makes a highly accurate prediction possible.

Brief Description of the Drawings

FIG. 1 is a flowchart illustrating the procedure for determining the biomarkers used in the Examples of the present invention.
FIG. 2 is a diagram illustrating a correlation network between metabolites in saliva used in the Examples.
FIG. 3 is a flowchart illustrating a procedure of developing a mathematical model used in the Examples.
FIG. 4 is a diagram illustrating a model of a decision tree that distinguishes a subject with pancreatic cancer from a healthy subject.
FIG. 5 is a diagram illustrating a receiver operating characteristic (ROC) curve of a mathematical model that distinguishes a subject with pancreatic cancer from a healthy subject using a metabolite concentration normalized with a concentration marker used in the Examples.
FIG. 6 is a diagram in which a risk of pancreatic cancer (PC) for a healthy subject (C), and subjects with pancreatic cancer (PC), chronic pancreatitis (CP), and IPMN is plotted in a model of classifying the healthy subject and the subject with pancreatic cancer in the Examples.
FIG. 7 is a diagram illustrating a stepwise forward selection method used for variable selection in an MLR model that distinguishes a subject with pancreatic cancer from a healthy subject when the absolute concentration of the concentration marker as used in the Examples.
FIG. 8 is a diagram illustrating a forward selection method used for variable selection in the MLR model that distinguishes a subject with pancreatic cancer from a healthy subject when the absolute concentration of the concentration marker as used in the Examples.
FIG. 9 is a diagram illustrating an example of a total concentration of amino acids in saliva used in the Examples.
FIG. 10 is a diagram illustrating an ROC curve in which variables in an MLR model that distinguishes a patient with breast cancer from a healthy subject are $\beta$-alanine, N-acetylphenylalanine, and citrulline.
FIG. 11 is a diagram illustrating a ROC curve in which the variables in the same MLR model are N-acetylphenylalanine, N1-acetylspermidine, and creatine.
FIG. 12 is a diagram of a network between metabolites for determination of a concentration-correcting substance for a biomarker for breast cancer.
FIG. 13 includes diagrams illustrating substances belonging to polyamines among substances that give a significant difference between the healthy subject and the patient with breast cancer.
FIG. 14 includes diagrams illustrating examples of substances other than polyamines among the substances that give a significant difference between the healthy subject and the patient with breast cancer.
FIG. 15 includes diagrams illustrating the top five substances that give a significant difference between the healthy subject and the patient with breast cancer and has a smaller p value regardless of the presence or absence of concentration correction, and an ROC curve thereof.
FIG. 16 is a correlation network diagram illustrating a reason for determining Gly to be a correction marker.
FIG. 17 includes diagrams illustrating the concentrations of metabolites in a cancer tissue sample obtained during surgery of oral cancer and a healthy tissue sample near the cancer tissue sample.
FIG. 18 includes diagrams illustrating a difference in the concentration of saliva of a patient with oral cancer from that of the healthy subject when a method of collecting saliva in the patient is changed.

Description of Embodiments

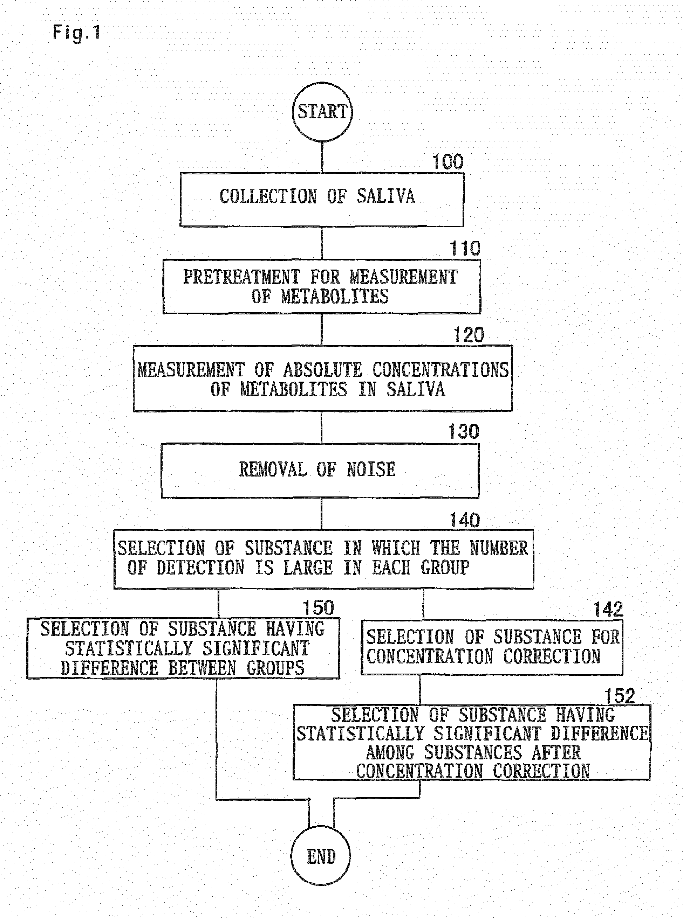

Hereinafter, an embodiment suitably implementing the present invention (hereinafter referred to as the embodiment) will be described in detail. The present invention is not limited to the contents described in the following embodiments and Examples. In addition, constituents in the following embodiments and Examples include those that can be easily assumed by those skilled in the art, those that are substantially the same, and those falling within the scope of the so-called doctrine of equivalents. Further, the constituents disclosed in the following embodiments and Examples may be used in appropriate combination or by appropriate selection.
A procedure of determining a biomarker for a pancreatic disease will be described with reference to FIG. 1.

1. Saliva donor

A total of 199 saliva samples was collected from (i) patients with pancreatic cancer at various stages, (ii) healthy controls, and (iii) patients with intraductal papillary mucinous neoplasm (IPMN) and chronic pancreatitis for evaluation of specificity. Table 1 lists the number of cases, the number of males and females, and age in each group. Cases in which patients never experienced chemotherapy before treatment began were subject to this procedure. The number of deficiencies of Table 1 shows the number of cases in which values are unknown.

Table 1

| DISEASE | STAGE | THE NUMBER OF CASES | SEX | | | AGE | | | |
|---|---|---|---|---|---|---|---|---|---|
| | | | MALE | FEMALE | THE NUMBER OF DEFECTS | MINIMUM | MEDIAN | MAXIMUM | THE NUMBER OF DEFECTS |
| HEALTHY | - | 63 | 50 | 13 | | 19 | 43 | 73 | |
| CHRONIC PANCREATITIS | - | 14 | 12 | 2 | | 32 | 49 | 79 | |
| IPMN | - | 7 | 6 | 1 | | 63 | 64 | 69 | 2 |
| PANCREATIC CANCER | I | 3 | 1 | 2 | | 61 | 68 | 76 | |
| | II | 3 | 1 | 1 | 1 | 67 | 67.5 | 68 | 1 |
| | III | 18 | 10 | 8 | | 51 | 69.5 | 95 | |
| | Iva | 37 | 14 | 16 | 7 | 45 | 69 | 85 | 7 |
| | Ivb | 54 | 30 | 22 | 2 | 43 | 72 | 86 | 3 |
| | SUBTOTAL OF PANCREATIC CANCER | 115 | 56 | 49 | 10 | 43 | 70 | 95 | 11 |

2. Method for collecting saliva (Step 100 in FIG. 1)

∘ With respect to collection date
Collection is performed on a day other than a surgery day as much as possible.
∘ With respect to diet
After 21:00 of the day before the collection, do not drink anything but water.
On the day of collection, do not eat breakfast.
∘ Notes before collection of saliva on the day
Collect saliva from AM 8:30 to 11:00 before breakfast.
Brush teeth without use of toothpaste 1 hour or more before the collection of saliva.
Do not strenuously exercise 1 hour before the collection of saliva.
Do not clean the inside of oral cavity (with a toothpick, etc.).
Do not smoke.
Do not drink anything but water.
∘ Method for collecting saliva
The mouth is rinsed with water before the collection of saliva, and non-irritant mixed saliva is collected.

Only saliva that runs spontaneously but is not volitionally generated is collected (sialemesis method). Alternatively, a straw is placed in the mouth when saliva is retained to some extent in the mouth (the time is about 3 minutes), and the saliva runs into a tube (passive drool method). When the face is turned down and saliva in the mouth is pushed into the straw that is vertically set, the saliva is likely to run spontaneously. However, when the saliva adheres to a middle of the straw and does not fall down, the saliva is sent out by the breath (in this case, saliva is easily collected by retaining saliva in the mouth to some extent and then pushing the saliva into the tube at one time as compared with opening of the mouth to the tube).

200 μL or more of saliva (as much as possible) is collected.

During the collection of saliva, the tube is placed on ice and kept at a low temperature as much as possible, and the collection is finished within 15 minutes (even when 200 μL of saliva is not collected, the collection is finished in 15 minutes).

Within 5 minutes, the saliva is cryopreserved on ice at -80°C or with dry ice for storage. The tube and the straw of collecting saliva is a tube and a straw made of a polypropylene material.

A method for collecting saliva is not limited to the aforementioned method, and another method may be used.

3. Pretreatment method for measurement of metabolites in saliva (Step 110 in FIG. 1)

400 μL of saliva sample is taken, placed in an ultrafiltration filter (molecular weight cutoff: 5,000 Da), and centrifuged at 4°C and 9,100 g for 3.5 hours. 45 μL of the filtrate and 5 μL of an aqueous solution in which the concentration of each of methionine sulfone (Methionine sulfone), 2-morpholinoethanesulfonic acid monohydrate (2-Morpholinoethanesulfonic acid, monohydrate), CSA (D-Camphor-10-sulfonic acid), sodium salt, 3-aminopyrrolidine (3-Aminopyrrolidine), and trimesic acid (Trimesate) is 2 mM are mixed to prepare 50 μL of a sample. Measurement was performed by the following method.

4. Measurement of absolute concentrations of metabolites in saliva by capillary electrophoresis-time-of-flight mass spectrometry (CE-TOFMS) (Step 120 in FIG. 1)

Ionic metabolites were identified and quantitatively determined from saliva by metabolome analysis using CE-MS.

A measurement method was performed in accordance with the method described in Non-Patent Literature 6. Hereinafter, the parameters will be described.

1) Cationic metabolite measurement mode

• HPCE

Capillary: fused silica, 50 μm in inner diameter × 100 cm in length

Buffer: 1 M formic acid (formate)
Voltage: positive, 30 kV
Temperature: 20°C
Injection: injection under a pressure of 50 mbar for 5 seconds (about 3 nL)
Washing before measurement: with 30 mM ammonium formate (Ammonium Formate) at a pH of 9.0 for 5 minutes, Milli-Q water for 5 minutes, and buffer for 5 minutes

• TOFMS

Polarity: positive
Capillary voltage: 4,000 V
Fragmentor voltage: 75 V
Skimmer voltage: 50 V
OCT RFV: 125 V
Drying gas: nitrogen ($N_2$), 10 L/min
Drying gas temperature: 300°C
Nebulizer gas pressure: 7 psig
Sheath liquid: 50% MeOH / 0.1 $\mu$M Hexakis (2,2-difluoroethoxy) phosphazene-containing water
Flow rate: 10 mL/min
Reference m/z: 2MeOH $^{13}$C isotope [M+H]+m/z 66.063061, Hexakis(2,2-difluoroethoxy)phosphazene [M+H]+m/z 622.028963

2) Anionic metabolite measurement mode

• HPCE

Capillary: COSMO (+), 50 $\mu$m in inner diameter $\times$ 10.6 cm in length
Buffer: 50 mM ammonium acetate, pH: 8.5
Voltage: negative, 30 kV
Temperature: 20°C
Injection: injection under a pressure of 50 mbar for 30 seconds (about 30 nL) Washing before measurement: with 50 mM ammonium acetate at a pH of 3.4 for 2 minutes, and 50 mM ammonium acetate at a pH of 8.5 for 5 minutes

• TOFMS

Polarity: negative
Capillary voltage: 3,500 V
Fragmentor voltage: 100 V
Skimmer voltage: 50 V
OCT RFV: 200 V
Drying gas: nitrogen ($N_2$), 10 L/min
Drying gas temperature: 300°C
Nebulizer gas pressure: 7 psig
Sheath liquid: 5 mM ammonium acetate and 50% MeOH / 0.1 $\mu$M Hexakis (2,2-difluoroethoxy) phosphazene-containing water
Flow rate: 10 mL/min
Reference m/z: 2[$CH_3COOH$] $^{13}$C isotope [M-H]-m/z 120.038339,
Hexakis(2,2-difluoroethoxy) phosphazene + $CH_3COOH$ [M-H]- 680.035541
ESI needle: platinum

The anionic metabolite measurement may be performed before the cationic metabolite measurement.

5. Removal of noise (Step 130 in FIG. 1)

Signals of a substance in which a value largely varies depending on a measurement day and a substance not derived from a metabolite are removed.

From measurement data, all peaks in which a signal noise ratio was 1.5 or more were first detected. A commercially available standard substance was measured before measurement of the saliva samples. A peak in which a value of mass to charge ratio (m/z) obtained by a mass spectrometer and a corresponding migration time were assigned to a substance name. Thus, identification was performed. In quantitative determination, the peak area of each peak was divided by the area of the peak of the internal standard substance, a fluctuation of measurement sensitivity of the mass spectrometer was corrected, and the specific peak area ratio was calculated. The absolute concentration was calculated from a ratio of the specific peak area in the saliva samples to the specific peak area of the standard substance.

6. Selection of substance detected highly frequently in each group (Step 140 in FIG. 1)

Only a substance in which the peak can be detected in 30% or more cases (for example, three out of ten) of each group was selected.

7. Selection of a substance having a statistically significant difference between groups (Step 150 in FIG. 1)

After a typical test (in this case, Mann-Whitney test) was performed, a P value was corrected using a false discovery rate (FDR) and a Q value was calculated. A substance having a significant difference of Q < 0.05 was selected.
The substance selected by this procedure is a substance described in Embodiment 3.

8. Selection of substance for presuming concentrations of all metabolites in saliva and performing concentration correction (Step 142 in FIG. 1)

In all the samples measured (including healthy, breast cancer, oral cancer, IPMN, and pancreatic cancer), correlation values between the metabolites were exhaustively calculated using the determined quantitative values of the metabolites. Combinations of substances satisfying a Pearson correlation coefficient (R) of $R \geq 0.8$ were listed. Of a metabolite group in which the most substances correlated with each other, a substance that correlated with the most substances was selected.

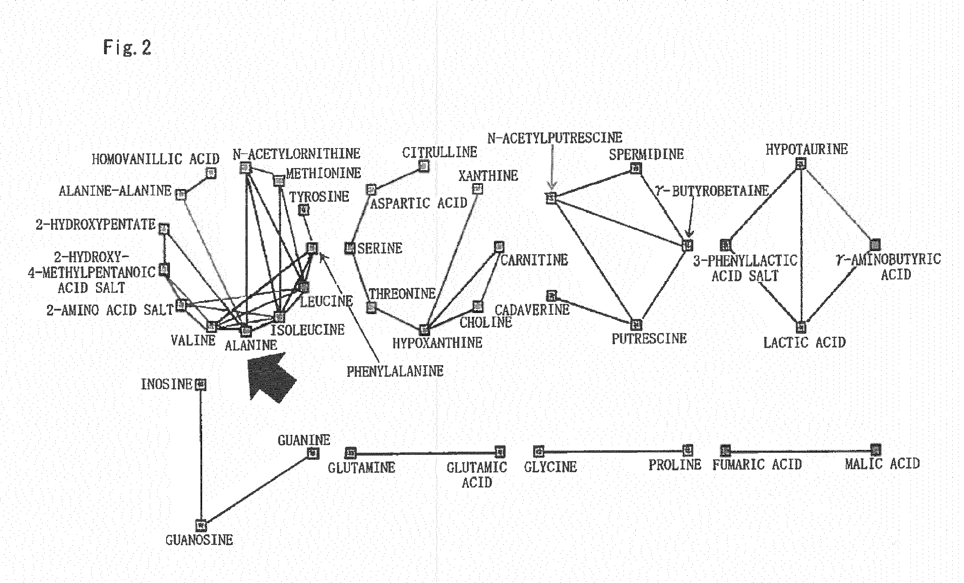

FIG. 2 shows one example of a correlation network diagram of the metabolites in saliva.

9. Selection of a substance having a statistically significant difference among the substances after concentration correction (Step 152 in FIG. 1)

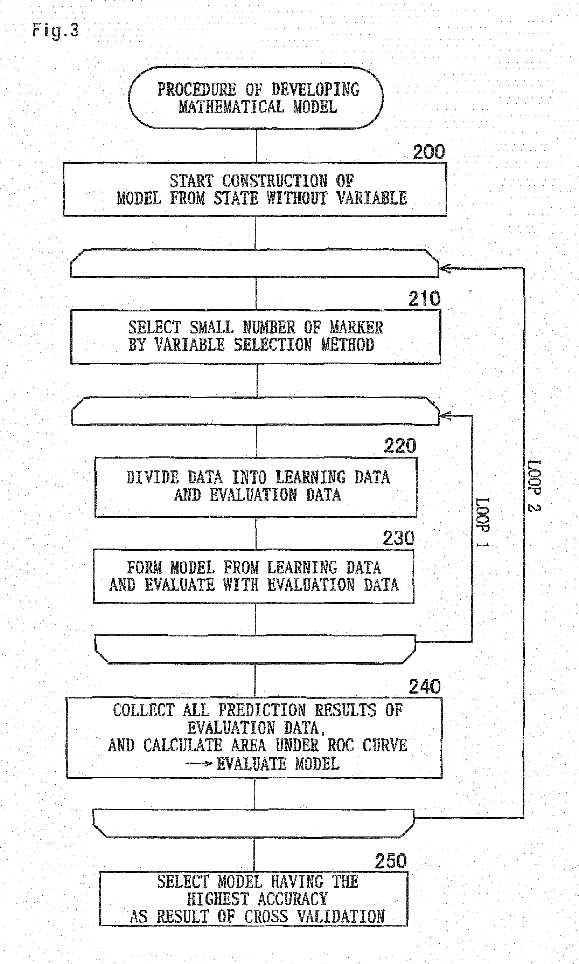

After the typical test (in this case, Mann-Whitney test) was performed using a value in which the concentration of each substance was corrected with the concentration of the substance selected at Step 142, a P value was corrected using the false discovery rate (FDR), and a Q value was calculated. A substance having a significant difference of Q < 0.05 was selected.
A procedure of developing a mathematical model of distinguishing the subjects with pancreatic cancer from the healthy subjects will be then described with reference to FIG. 3.
Using the marker selected at Step 150 or 152 in FIG. 1, a multiple logistic regression model (MLR model) that is a mathematical model was developed from a state in which a variable did not exist at Step 200. In the analysis of multiple logistic regression (MLR), a regression equation of P that is

$$\ln(P/1 - P) = b_0 + b_1 x_1 + b_2 x_2 + b_3 x_3 + \cdots + b_k x_k \ (1)$$

is determined using k description variables $x_1, x_2, x_3, \cdots,$ and $x_k$ for a ratio P as a target variable.
Specifically, a combination of the smallest independent variables that did not correlate with each other was selected at Step 210, for example, using a stepwise forward selection method of stepwise variable selection. A P value at which the variable was added was 0.05, a P value at which the variable was eliminated was 0.05, and a variable $x_i$ was selected.
At Step 220, the data were divided into learning data and evaluation data, and at Step 230, a model was formed from the learning data and evaluated using the evaluation data. In cross validation of Loop 1 in FIG. 3, Steps 220 and 230 were repeated.

At Step 240, receiver operating characteristic (ROC) analysis was performed using the selected model. An area under the ROC curve (AUC) and a 95% confidential interval (CI) were calculated, and the model was evaluated. In accordance with the ROC curve, a curve of $Y = X + \alpha$ ($\alpha$ is a constant) was drawn. When the value of $\alpha$ was decreased from 1 to 0, the value of $\alpha$ that first touched the ROC curve was determined. Thus, an optimal cut-off value was determined.

Next, the process proceeded to Step 250, and a model having the best accuracy as the result of cross validation was selected.

Herein, a stepwise method is used. The stepwise method includes three kinds of a forward selection method, a stepwise forward selection method, and a backward selection method. The threshold value may be adjusted to a threshold value of $P < 0.05$, and variable may be added. Therefore, the model having the best accuracy can be selected by forming a model many times at a larger loop 2 in FIG. 3.

Specifically, for evaluation of the MLR model, values of risk of pancreatic cancer (PC) with respect to saliva of breast cancer, oral cancer (CP), and IPMN were calculated. A group of the healthy subjects (C), and the subjects with CP and IPMN was formed. An AUC value that could identify pancreatic cancer from this group was calculated. The data were randomly divided into 10, a model was formed using 90% of the data, and the model was evaluated by the rest values of 10%. This operation was repeated 10 times. All the cases were selected once for evaluation, and cross validation (CV) of collecting the evaluation data and calculating the AUC value was performed.

10. Results of model of distinguishing pancreatic cancer from searched substance

FIG. 2 shows substances that exhibited high correlation values with the metabolites quantitatively determined at Step 120 in FIG. 1 at Step 142. In FIG. 2, a line is drawn between substances having $R \geq 0.8$. Eight clusters (groups of metabolites) are confirmed, but a cluster on the far left upper side in the drawing contains the most substances. In the cluster, alanine (Ala) forms the most networks with other substances. Therefore, alanine is determined as a metabolite for normalizing the concentration of the whole saliva. The metabolite used for normalization is not limited to the substance forming the most networks with other substances. For example, the total concentration of the metabolites, the sum of signals obtained during measurement of saliva by CE-MS (total ion electropherogram), or the area of a peak that is at a central order when all detected signals are sorted according to size may be used for normalization. The variable selection and the mathematical model are not limited to the stepwise method and the MLR model, respectively.

For example, for the variable selection, a correlation-based feature subset method (see M. A. Hall (1998). Correlation-based Feature Subset Selection for Machine Learning. Hamilton, New Zealand.), a relief method (see Marko Robnik-Sikonja, Igor Kononenko (1997). An adaptation of Relief for attribute estimation in regression. In: Fourteenth International Conference on Machine Learning, 296-304.), an SVM valiable selection method (see I. Guyon, J. Weston, S. Barnhill, V. Vapnik (2002). Gene selection for cancer classification using support vector machines. Machine Learning. 46(1-3): 389-422.), or the like may be applied.

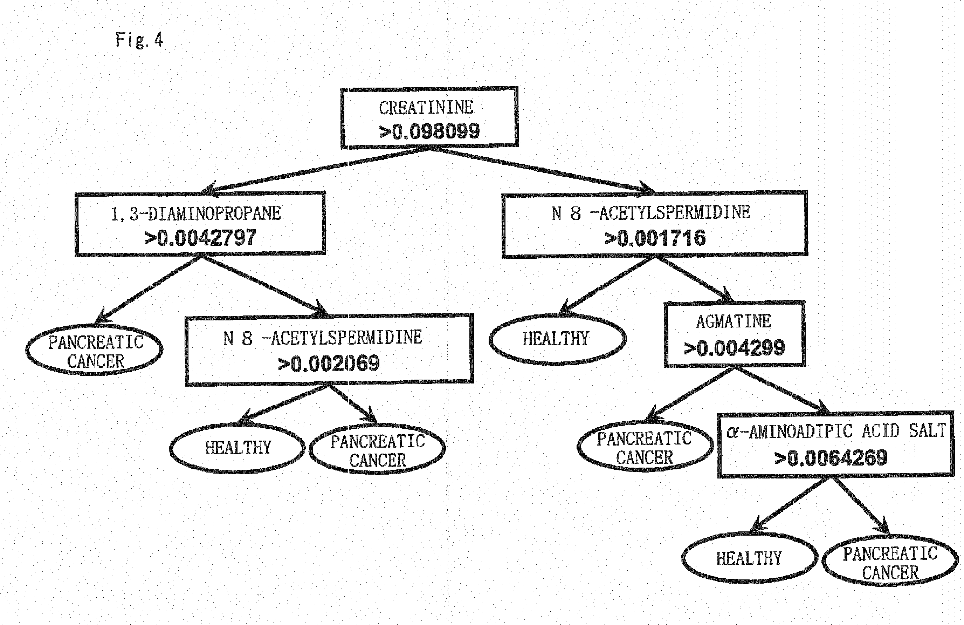

For the mathematical model, a mechanical learning method of dividing two groups may be applied. For example, Bayesian estimate (see Berger, James O (1985). Statistical Decision Theory and Bayesian Analysis. Springer Series in Statistics (Second ed.). Springer-Verlag. ISBN 0-387-96098-8.), neural network (ANN) (see D. E. Rumelhart, G. E. Hinton, and R. J. Williams, (1986): Learning representaions by back-propagating errors, Nature, 323-9, 533-536.), support vector machine (SVM) (see J. Platt (1998) Fast Training of Support Vector Machines using Sequential Minimal Optimization. In B. Schoelkopf and C. Burges and A. Smola, editors, Advances in Kernel Methods-Support Vector Learning), Alternative decision tree (ADTree) (see Yoav Freund and Llew Mason (1999) The Alternating Decision Tree Algorithm. Proceedings of the 16th International Conference on Machine Learning, 124-133, and Freund, Y., Mason, L. (1999) The alternating decision tree learning algorithm. In: Proceeding of the Sixteenth International Conference on Machine Learning, Bled, Slovenia, 124-133), decision tree (see Ross Quinlan (1993). C4.5: Programs for Machine Learning. Morgan Kaufmann Publishers, San Mateo, CA), PART model (see Eibe Frank, Ian H. Witten (1998) Generating Accurate Rule Sets Without Global Optimization. In: Fifteenth International Conference on Machine Learning, 144-151), Random forest, PLS discriminant analysis (see Partial least squares-discriminant analysis; PLS-DA)(Lindgren, F; Geladi, P; Wold, S (1993). The kernel algorithm for PLS. J. Chemometrics 7: 45-59. doi: 10.1002/cem.1180070104.), Orthogonal PLS discriminant analysis (OPLS-DA) (see Trygg, J., & Wold, S. (2002). Orthogonal projections to latent structures (O-PLS). Journal of Chemometrics, 16(3), 119-128, and Breiman, Leo (2001). Random Forests. Machine Learning 45 (1): 5-32. doi: 10.1023/A: 1010933404324.), or the like may be applied. Bootstrap method and Bagging method (see Breiman, Leo (1996) Bagging predictors. Machine, Learning24 (2): 123-140) in which prediction is performed using average and majority of predictive values of a plurality of mathematical models that are obtained by forming a plurality of mechanical learning methods of dividing two groups may be used. Further, separation may be performed using a principal component in principal component analysis (Principal Component Analysis; PCA) (see Hotelling, H. (1933). Analysis of a complex of statistical variables into principal components. Journal of Educational Psychology, 24, 417-441) that is unsupervised learning. FIG. 4 is a model of decision tree that distinguishes the subjects with pancreatic cancer from the healthy subjects. The concentrations of metabolites that were normalized with a concentration marker (in this case, Ala) were used. The area under the ROC curve was 0.856 and the area under the ROC curve during 10-fold cross validation was 0.653.

Substances having a high ability of distinguishing the subjects with pancreatic cancer from the healthy subjects at Step 152 of the above section 9 are shown in Table 2.

Table 2

| NAME OF SUBSTANCE | CONCENTRATION NORMALIZED WITH ALA (NO UNIT) | | | | DETECTION RATIO (%) | | MANN-WHITNEY TEST | | ROC CURVE | | |
| --- | --- | --- | --- | --- | --- | --- | --- | --- | --- | --- | --- |
| | HEALTHY SUBJECT AVERAGE | HEALTHY SUBJECT STANDARD DEVIATION | PANCREATIC CANCER AVERAGE | PANCREATIC CANCER STANDARD DEVIATION | HEALTHY SUBJECT | PANCREATIC CANCER | P VALUE | Q VALUE | AREA | 95% CI | P VALUE |
| N8-ACETYLSPERMIDINE | 0.0015 | 0.0015 | 0.0049 | 0.0071 | 70 | 90 | 1.55773E-06 | 0.000190043 | 0.7175 | 0.6414 to 0.7936 | 0.0001 |
| CREATININE | 0.1793 | 0.1349 | 0.1026 | 0.0946 | 100 | 99 | 1.74565E-05 | 0.001064849 | 0.695 | 0.6140 to 0.7759 | 0.0001 |
| SPERMINE | 0.0129 | 0.0222 | 0.0319 | 0.0477 | 63 | 83 | 3.99507E-05 | 0.001624661 | 0.6852 | 0.6036 to 0.7669 | 0.0001 |
| ASPARTIC ACID | 0.5559 | 0.2454 | 0.4290 | 0.2803 | 98 | 100 | 9.5103E-05 | 0.002320512 | 0.6772 | 0.5965 to 0.7578 | 0.0001 |
| N1-ACETYLSPERMIDINE | 0.0237 | 0.0147 | 0.0407 | 0.0351 | 97 | 99 | 0.000141561 | 0.002878407 | 0.6727 | 0.5929 to 0.7526 | 0.0001424 |
| N1-ACETYLSPERMINE | 0.0019 | 0.0045 | 0.0046 | 0.0060 | 30 | 60 | 7.37102E-05 | 0.002248162 | 0.6679 | 0.5868 to 0.7490 | 0.0002175 |
| CYTIDINE | 0.0232 | 0.0320 | 0.0107 | 0.0198 | 79 | 56 | 0.0001175296 | 0.00305516 | 0.6663 | 0.5828 to 0.7499 | 0.0002494 |
| α-AMINOADIPIC ACID SALT | 0.0381 | 0.0234 | 0.0689 | 0.0811 | 97 | 99 | 0.0007784335 | 0.010632096 | 0.6525 | 0.5712 to 0.7337 | 0.0007859 |
| CYTOSINE | 0.0042 | 0.0094 | 0.0105 | 0.0180 | 35 | 61 | 0.000495928 | 0.007562907 | 0.6489 | 0.5662 to 0.7316 | 0.001037 |
| BETAINE | 0.2236 | 0.3431 | 0.1548 | 0.4206 | 98 | 98 | 0.001209678 | 0.013416426 | 0.6469 | 0.5614 to 0.7325 | 0.001211 |
| UREA | 39.1920 | 43.7834 | 23.5791 | 42.6852 | 98 | 98 | 0.001345148 | 0.013675675 | 0.6455 | 0.5595 to 0.7316 | 0.001347 |
| HOMOVANILLIC ACID SALT | 0.0653 | 0.1133 | 0.0999 | 0.1260 | 46 | 75 | 0.00108606 | 0.013249934 | 0.645 | 0.5573 to 0.7327 | 0.001404 |
| N-ACETYLNEURAMINIC ACID SALT | 1.6138 | 1.8792 | 0.9916 | 1.3150 | 92 | 95 | 0.00158931 | 0.014559781 | 0.6433 | 0.5560 to 0.7307 | 0.001592 |
| CYSTINE | 0.0040 | 0.0062 | 0.0068 | 0.0071 | 43 | 70 | 0.001670795 | 0.014559781 | 0.6381 | 0.5526 to 0.7236 | 0.002351 |
| UROCANIC ACID SALT | 0.0965 | 0.0780 | 0.0696 | 0.0626 | 98 | 97 | 0.003836014 | 0.029249607 | 0.6313 | 0.5458 to 0.7167 | 0.003834 |
| FUMARIC ACID SALT | 0.0104 | 0.0209 | 0.0206 | 0.0294 | 35 | 59 | 0.00304094 | 0.024732975 | 0.6262 | 0.5418 to 0.7105 | 0.005451 |
| 1,3-DIAMINOPROPANE | 0.0516 | 0.0554 | 0.0304 | 0.0397 | 78 | 73 | 0.00511095 | 0.034103773 | 0.6261 | 0.5368 to 0.7154 | 0.005477 |
| HYPOTAURINE | 0.0322 | 0.0508 | 0.0498 | 0.0739 | 51 | 78 | 0.004991032 | 0.034103773 | 0.6255 | 0.5352 to 0.7157 | 0.005712 |
| NICOTINIC ACID SALT | 0.2649 | 1.1623 | 0.1163 | 0.5318 | 75 | 65 | 0.005311243 | 0.034103773 | 0.6246 | 0.5365 to 0.7127 | 0.006067 |
| AGMATINE | 0.0050 | 0.0050 | 0.0033 | 0.0039 | 81 | 69 | 0.005639892 | 0.034403344 | 0.6244 | 0.5377 to 0.7112 | 0.006123 |
| VALINE | 0.3794 | 0.2213 | 0.4894 | 0.3424 | 100 | 99 | 0.006972284 | 0.040119018 | 0.6225 | 0.5380 to 0.7070 | 0.006964 |
| 2-HYDROXY-4-METHYLPENTANOIC ACID SALT | 0.0645 | 0.0686 | 0.0949 | 0.0948 | 75 | 94 | 0.0073234577 | 0.040119018 | 0.6218 | 0.5333 to 0.7103 | 0.007288 |
| ALANYL-ALANINE | 0.0301 | 0.0175 | 0.0247 | 0.0139 | 89 | 96 | 0.0010929763 | 0.055559628 | 0.6155 | 0.5261 to 0.7049 | 0.01092 |
| CITRIC ACID SALT | 0.3689 | 0.5593 | 0.2466 | 0.5154 | 95 | 85 | 0.0014695008 | 0.067709323 | 0.6107 | 0.5265 to 0.6949 | 0.01474 |
| GLUCOSAMINE | 0.0124 | 0.0117 | 0.0078 | 0.0104 | 65 | 55 | 0.01143552 | 0.055805337 | 0.6106 | 0.5209 to 0.7004 | 0.0148 |
| CARNOSINE | 0.0029 | 0.0046 | 0.0016 | 0.0040 | 54 | 38 | 0.008103017 | 0.042981222 | 0.609 | 0.5195 to 0.6985 | 0.01629 |
| GLYCINE-GLYCINE | 0.0154 | 0.0158 | 0.0229 | 0.0202 | 56 | 80 | 0.015539845 | 0.067709323 | 0.6086 | 0.5203 to 0.6968 | 0.01677 |
| 2-AMINOBUTYRIC ACID | 0.0756 | 0.1971 | 0.0723 | 0.0719 | 95 | 100 | 0.017659224 | 0.074290531 | 0.6077 | 0.5190 to 0.6965 | 0.01762 |
| ARGININE | 0.5163 | 0.3841 | 0.4082 | 0.4195 | 100 | 99 | 0.019323999 | 0.078584264 | 0.6062 | 0.5197 to 0.6927 | 0.01928 |
| N-ACETYLGLUTAMIC ACID SALT | 0.0035 | 0.0057 | 0.0053 | 0.0060 | 40 | 63 | 0.015062166 | 0.067709323 | 0.6052 | 0.5172 to 0.6933 | 0.02041 |
| GLYCEROPHOSPHORIC ACID SALT | 0.3663 | 0.2880 | 0.3039 | 0.3335 | 100 | 99 | 0.020954571 | 0.082466378 | 0.6048 | 0.5208 to 0.6889 | 0.02091 |
| PHOSPHOENOLPYRUVIC ACID | 0.0130 | 0.0194 | 0.0222 | 0.0398 | 44 | 67 | 0.026459721 | 0.100877686 | 0.5972 | 0.5093 to 0.6852 | 0.03216 |
| ISOLEUCINE | 0.1286 | 0.0693 | 0.1629 | 0.1046 | 100 | 100 | 0.037465083 | 0.138507278 | 0.5945 | 0.5089 to 0.6800 | 0.03738 |
| ADENOSINE | 0.0126 | 0.0131 | 0.0093 | 0.0141 | 81 | 78 | 0.039739367 | 0.142594198 | 0.593 | 0.5040 to 0.6820 | 0.04054 |
| GUANINE | 0.0520 | 0.0403 | 0.0449 | 0.0543 | 94 | 91 | 0.04241365 | 0.147344023 | 0.5921 | 0.5065 to 0.6778 | 0.04236 |
| DIHYDROXYACETONEPHOSPHORIC ACID | 0.2462 | 0.1806 | 0.2041 | 0.1814 | 94 | 97 | 0.047321948 | 0.154153689 | 0.5901 | 0.5043 to 0.6758 | 0.04722 |
| CADAVERINE | 0.5943 | 0.7614 | 0.9336 | 1.2717 | 100 | 99 | 0.048015084 | 0.154153689 | 0.5898 | 0.5039 to 0.6757 | 0.0479 |

In Table 2, a detection ratio shows a ratio of cases in which a peak can be detected relative to all the cases in each group of the healthy subjects and the subjects with pancreatic cancer. A 95% confidential interval (CI) represents a value of 95% confidential interval.

A Mann-Whitney test that is a non-parametric two-group test for the healthy subject group and the pancreatic cancer group was performed between the healthy subjects and the subjects with pancreatic cancer, the p value of each of the metabolites was calculated, and the p value was corrected with the false discovery rate (FDR). A test of q value was performed.

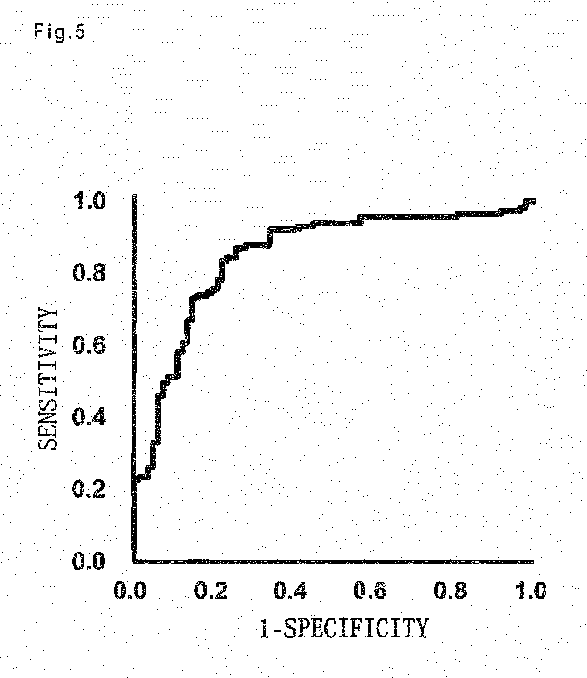

For evaluation of sensitivity and specificity of the substances that distinguish two groups of the subjects with pancreatic cancer (PC) and the healthy subjects (C), receiver operating characteristic (ROC) analysis was performed. The results are shown in FIG. 5. The concentrations of metabolites that were normalized with the concentration marker were used. The substances contained in the MLR model and a parameter and an odds ratio thereof are shown in Table 3.

Table 3

| ITEM | PARAMETER | P VALUE | 95% CI | | ODDS RATIO | 95% CI | |
|---|---|---|---|---|---|---|---|
| (INTERCEPT) | -0.0375549 | 0.9179 | -0.7551751 | 0.67987596 | - | - | - |
| CREATININE | 8.81954122 | <.0001 | 5.11051632 | 13.1970776 | 6765.16 | 165.7559 | 538788.1 |
| N1-ACETYLSPERMIDINE | -36.266806 | 0.0017 | -60.649929 | -14.849848 | 1.78E-16 | 4.57E-27 | 3.56E-07 |
| $\alpha$-AMINOADIPIC ACID | -25.286236 | 0.0039 | -43.368968 | -9.1982815 | 1.04E-11 | 1.46E-19 | 0.000101 |
| N-ACETYLNEURAMINIC ACID | 0.30754159 | 0.0219 | 0.05238651 | 0.58469864 | 1.360077 | 1.053783 | 1.79445 |
| 1.3-DIAMINOPROPANE | 11.309134 | 0.004 | 4.02635127 | 19.6651022 | 81563.25 | 56.056 | 3.47E+08 |

The area under the ROC curve in FIG. 5 was 0.8763 (95% CI: 0.8209 to 0.9317, p < 0.0001). The sensitivity of optimal cut-off value was 0.8348, and (1-specificity) was 0.2169.

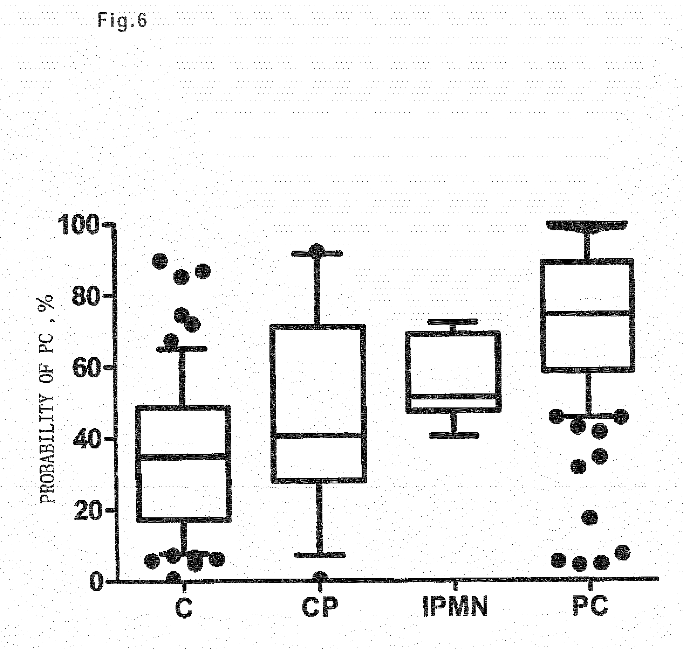

Using the MLR model that can distinguish the healthy subjects and the subjects with pancreatic cancer, values calculated as risk of pancreatic cancer (PC) of the healthy subjects (C) and the subjects with pancreatic cancer (PC) as well as the patients with breast cancer, oral cancer (CP), and IPMN are shown in FIG. 6.

FIG. 6 is a diagram in which the risk of pancreatic cancer (PC) of C, PC, breast cancer, oral cancer (CP), and IPMN is plotted by the model of classifying the healthy subjects (C) and the subjects with pancreatic cancer (PC). A boxplot represents values of 10%, 25%, 50%, 75%, and 90% from the top, and values under 10% and values beyond 90% are expressed as plots.

Table 4 shows an AUC value in which the specificity and general-purpose properties of the MLR model were evaluated.

Table 4

|  | DISTINGUISH PC FROM C | DISTINGUISH PC FROM C+CP+IPMN |
|---|---|---|
| WHEN ALL THE DATA ARE USED | 0.88 | 0.85 |
| IN CASE OF CV | 0.86 | 0.83 |

Herein, CV represents a case of cross validation.

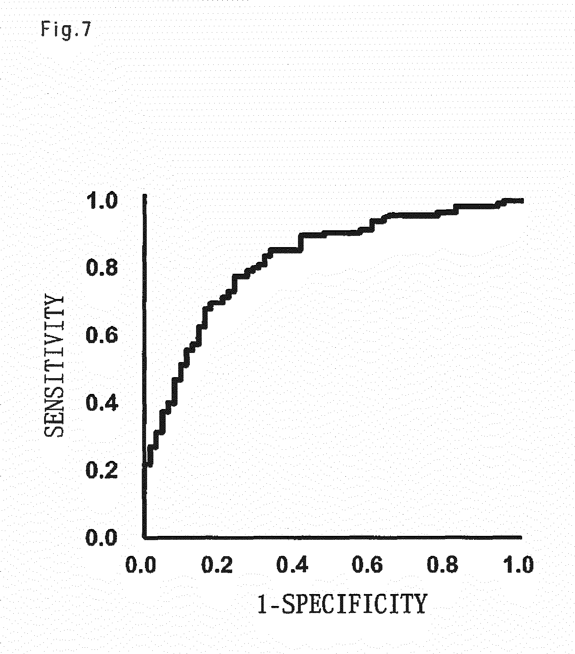

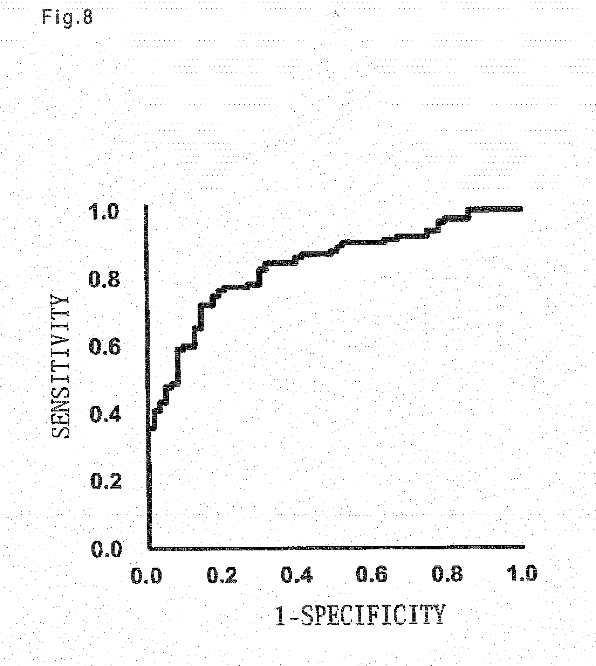

In FIG. 7, an ROC curve at which the MLR model was formed using the absolute concentration without concentration correction is shown. Table 5 shows selected markers and coefficients. The area under the ROC curve was 0.8264 (95% CI: 0.7619 to 0.8874, p < 0.0001). The accuracy was slightly decreased as compared with a case with concentration correction, but highly accurate prediction was possible. For formation of this model, a P value at which the variable was added was 0.05, and a P value at which the variable was eliminated was 0.05 using a stepwise forward selection method of stepwise variable selection. FIG. 8 shows an ROC curve at which the P value at which the variable was added was 0.05 using a forward selection method as a method of variable selection. Table 6 shows selected markers and coefficients. The area under the ROC curve was 0.8373 (95% CI: 0.7792 to 0.8954, p < 0.0001). Regardless of use of the markers and coefficients that were different from the model of FIG. 7, prediction accuracy of the same level could be achieved.

Table 5

| ITEM | PARAMETER | P VALUE | 95% CI | | ODDS RATIO | 95% CI | |
|---|---|---|---|---|---|---|---|
| (INTERCEPT) | 0.40019 | 0.162 | -0.15107 | 0.977355 | - | - | - |
| $\alpha$-AMINOADIPIC ACID | -0.64356 | <.0001 | -0.95539 | -0.39782 | 0.525421 | 0.384661 | 0.671782 |
| PHOSPHORYCHOLINE | -0.04641 | 0.0427 | -0.09647 | -0.00235 | 0.954646 | 0.90804 | 0.997649 |
| N-ACETYLNEURAMINIC ACID | 0.014466 | <.0001 | 0.007874 | 0.02224 | 1.014571 | 1.007905 | 1.022489 |

Table 6

| ITEM | PARAMETER | P VALUE | 95% CT | | ODDS RATIO | 95% CI | |
|---|---|---|---|---|---|---|---|
| (INTERCEPT) | 0.277503 | 0.3624 | -0.31567 | 0.884388 | - | - | - |
| 3PG | 0.166107 | 0.0028 | 0.063545 | 0.282987 | 1.1807 | 1.065608 | 1.327088 |
| N1-ACETYLSPERMINE | -2.26566 | 0.0127 | -4.28489 | -0.65321 | 0.103762 | 0.013775 | 0.520371 |
| $\alpha$-AMINOADIPIC ACID | -0.9899 | <.0001 | -1.47201 | -0.58351 | 0.371613 | 0.229465 | 0.557935 |
| N-ACETYLNEURAMINIC ACID | 0.012425 | 0.0007 | 0.005686 | 0.020249 | 1.012503 | 1.005702 | 1.020456 |
| 4-($\beta$-ACETYLAMINOETHYL) IMIDAZOLE | 4.469352 | 0.0041 | 1.397495 | 7.786111 | 87.30013 | 4.045055 | 2406.938 |

11. Consideration of searched substance

In the present invention, the concentrations of ionic metabolites contained in saliva were simultaneously measured, and markers having a high ability of distinguishing the subjects with pancreatic cancer from the healthy subjects were selected. Further, a model having higher accuracy (sensitivity and specificity) as compared with a single substance could be developed by combining the markers.

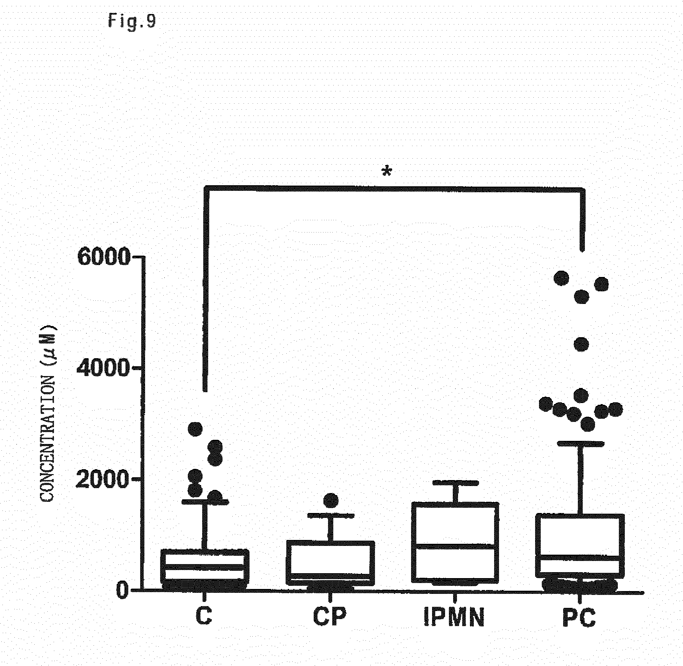

A problem involved in using saliva is that there is a greater variation of concentrations present in saliva as compared with blood. In this method, saliva was collected under unified conditions depending on the collection time and dietary restriction before the collection. Some of the samples had trends in which the concentrations of all the substances were clearly high or low. FIG. 9 shows a difference in the total concentration of amino acids in saliva of each disease. A boxplot has the same meanings as that of FIG. 6. A Kruskal-Wallis test that is a non-parametric multiplex test was performed, and the P value was 0.0138. After that, a Dunn's post test was performed. Only between C and PC, the P value was less than 0.05.

The concentration in the subjects with pancreatic cancer (PC) is significantly higher than that in the healthy subjects (C), and as an indication exhibiting a risk of pancreatic cancer, a high total concentration in saliva itself may be used. However, some of the samples of C have a concentration higher than PC, and in contrast, some of the samples of PC have a concentration lower than C. Therefore, when the samples are simply considered for a risk, the accuracy is low (from results of ROC analysis between C and PC in data of FIG. 8, AUC = 0.6282, and p = 0.004756).

Because of this, if these samples (the concentration is high in C and the concentration is low in PC) are excluded and only samples whose concentrations are within a certain range are subject to the test, the entire concentration can also be considered, but the number of cases that is subject to the test needs to be reduced. Therefore, the fluctuation of the entire concentration was offset by performing normalization using a substance that has a high correlation with the entire metabolite concentration of the saliva and can be detected in all the samples by the method shown in FIG. 2. The normalization may be omitted.

Among the substances as marker candidates in Table 2, polyamines such as spermine, and acetylated polyamines such as N8-acetylspermidine, N1-acetylspermidine, and N1-acetylspermine are each a substance that reflects on a state of the pancreatic tissues according to various changes in cancer. However, for example, in a case of spermine in urine, the concentration correction with creatinine is only considered. Therefore, spermine cannot achieve the accuracy that a tumor marker measured in a blood test can achieve. Because polyamines in blood are taken up by erythrocytes (see Fu NN, Zhang HS, MaM, Wang H. (2007) Quantification of polyamines in human erythrocytes using a new near-infrared cyanine 1-(epsilon-succinimidyl-hexanoate)-1'-methyl-3,3,3',3'-tetramethyl-indocarbocyanine-5,5'-dis ulfonate potassium with CE-LIF detection. Electrophoresis. 28(5): 822-9), the amount of polyamines in a free state is extremely small, and the concentration thereof in urine is extremely low. Even when polyamines in blood and urine are measured in breast cancer, the highest concentration of spermidine is about 140 nM (nanomol), and the concentration of N-acetylspermidine is about 64 nM (nanomol). Thus, concentrations that are much lower than the concentrations in saliva are reported (see Byun JA, Lee SH, Jung BH, Choi MH, Moon MH, Chung BC. (2008) Analysis of polyamines as carbamoyl derivatives in urine and serum by liquid chromatography-tandem mass spectrometry. Biomed Chromatogr. 22(1): 73-80). The quantitative determination of polyamines in erythrocytes requires a complicated step. Therefore, a diagnosis method found in the present invention has characteristics in which a highly accurate prediction can be achieved due to the contribution of the following three points, including (i) use of saliva capable of detecting the marker substances at high concentration, (ii) a decrease in dispersion generated at each measurement due to a simple treatment process for measurement, and (iii) use of the mathematical model in combination with the markers. A difference in mRNA in saliva between the patients with pancreatic cancer and the healthy subjects is already known (Non-Patent Literature 4). However, mRNA is completely different because a molecular group to which the present invention is directed is a metabolite. The variation of metabolites by themselves in saliva depending on pancreatic cancer is already known (Non-Patent Literature 5). However, substances that are not disclosed in known documents are used as a marker in the present invention, and a mathematical model for eliminating the effect of a specific concentration variation in saliva and identifying pancreatic cancer with high sensitivity and specificity can be developed.

With respect to four groups of healthy subjects, chronic pancreatitis, IPMN, and pancreatic cancer, a distribution of risk of pancreatic cancer that is predicted by the MLR model shows that the model exhibits high specificity for pancreatic cancer (FIG. 6). The results of cross validation (Table 4) and the results of a test for distinguishing the pancreatic cancer group from the groups other than the pancreatic cancer group also show that this model has high sensitivity and specificity that cannot be achieved by the conventional method.

In Examples, capillary electrophoresis-mass spectroscopy (CE-MS) is used to measure the concentrations of metabolites in saliva. However, high speed liquid chromatography (LC), gas chromatography (GC), chip LC, or chip CE, or GC-MS, LC-MS, and CE-MS methods in which they are combined with a mass spectrometer (MS), a measurement method for each MS alone, an NMR method, a measurement method for a metabolite substance that is derivatized into a fluorescent substance or a UV absorptive material, or an enzyme method in which an antibody is produced and measured by an ELISA method, may be used. Regardless of the measurement method, measurement may be performed by any analysis.

Next, a biomarker for breast cancer will be described.

Cases included healthy subjects (20 cases), and patients with breast cancer (90 cases) including patients with breast cancer before initiation of treatment (37 cases), patients with breast cancer that were treated with chemotherapy, hormonotherapy, or the like. In the breast cancer cases, one patient was male and the rest were female. In the patients with breast cancer before initiation of treatment, eight cases were DCIS, and 29 cases were invasive ductal carcinoma.

A method for collecting saliva, a method for measuring metabolites, and the like were the same as those used in the biomarker for pancreatic cancer.

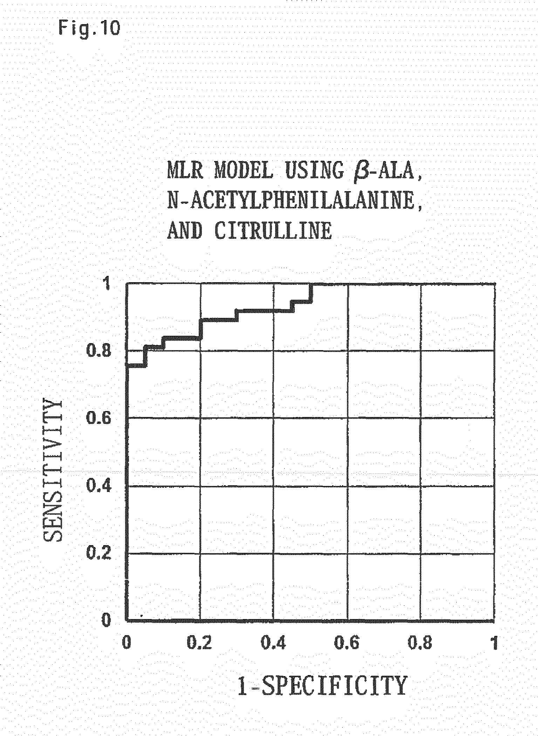

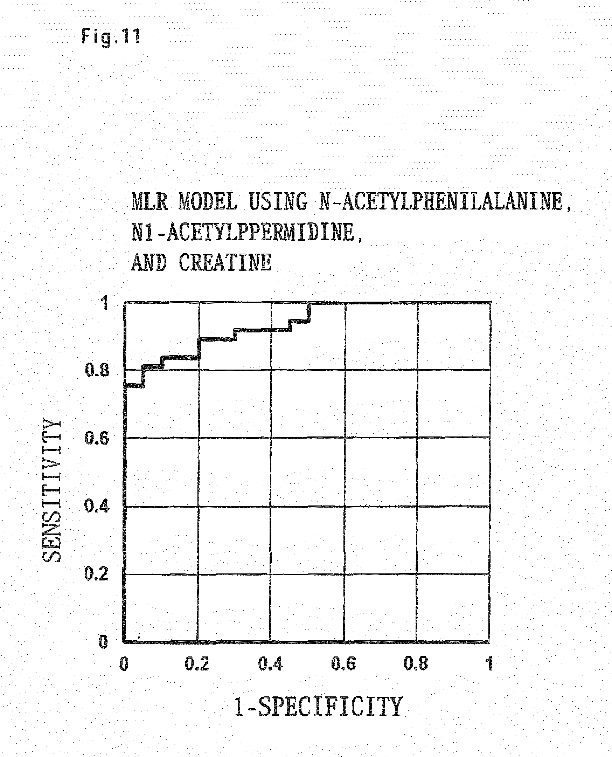

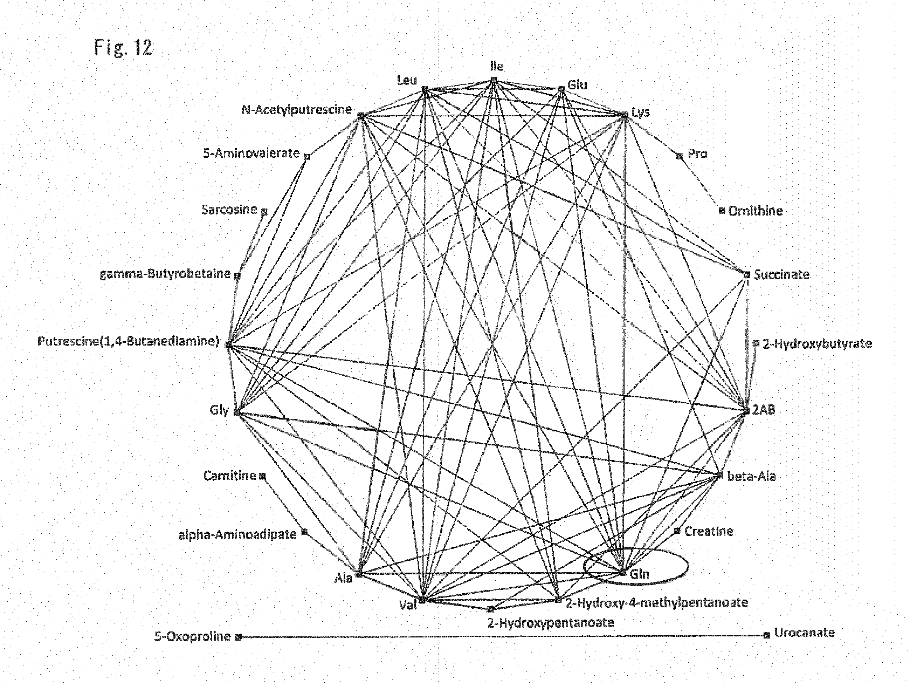

In a variable selection method performed during formation of multiple logistic regression model (MLR mode), only a substance of $Q \leq 0.05$ was used. In FIG. 10, $\beta$-alanine (Beta-Ala), N-acetylphenylalanine (N-Acetylphenylalanine), and citrulline (Citrulline) were used for addition of variable at $P \leq 0.05$ or elimination of variable at $P \geq 0.05$ by a stepwise forward selection method. In FIG. 11, N-acetylphenylalanine, N1-acetylspermidine, and creatine (Creatine), which include N1-acetylspermidine (N1-Acetylspermidine) that is a known marker, were used in a method for adding (increasing) a variable at $P \leq 0.05$ by a stepwise forward selection method. In the model of FIG. 10, as the ROC value of each substance, the ROC value of $\beta$-alanine is 0.8373, the ROC value of N-acetylphenylalanine is 0.7122, and the ROC value of citrulline is 0.698. By conversion of the substances into the MLR model, the ROC values are increased to 0.9622. Also in FIG. 12 including a known marker, the ROC value of N-acetylphenylalanine is 0.7122, the ROC value of N-acetylspermidine is 0.7811, and the ROC value of creatine is 0.7824. It was confirmed that the ROC values were increased to 0.9365 by combination of the substances using the MLR model.

Substances in which the absolute concentration exhibits a statistic significant difference ($p < 0.05$ in the Mann-Whitney test) between the patients with breast cancer and the healthy subjects are shown in Tables 7-1, 7-2, 7-3, and 7-4. Comparison was performed in 20 cases of the healthy subjects and all the cases (90 cases) including the patients with breast cancer before treatment without chemotherapy or hormonotherapy (37 cases). A Q value was calculated by the false discovery rate (FDR).

## Table 7-1

| NAME OF SUBSTANCE | | CONCENTRATION (μM) | | | | | | DETECTION RATIO (%) | | | MANN-WHITNEY TEST | | | | PUBLICLY KNOWN | SIGNIFICANT |
|---|---|---|---|---|---|---|---|---|---|---|---|---|---|---|---|---|
| | | HEALTHY SUBJECT | | ONLY BEFORE TREATMENT | | BREAST CANCER INCLUDING DURING TREATMENT | | HEALTHY SUBJECT | BREAST CANCER BEFORE TREATMENT | INCLUDING DURING TREATMENT | ONLY BEFORE TREATMENT vs HEALTHY SUBJECT | | INCLUDING DURING TREATMENT vs HEALTHY SUBJECT | | | |
| | | AVERAGE | STANDARD DEVIATION | AVERAGE | STANDARD DEVIATION | AVERAGE | STANDARD DEVIATION | | | | P VALUE | Q VALUE | P VALUE | Q VALUE | | |
| CHOLINE | Choline | 5.378 | 3.347 | 20.534 | 26.7069 | 18.249 | 21.29059 | 20 | 37 | 90 | 1.18E-05 | 0.001 | 3.07E-06 | 0.0002 | | 1 |
| 2-HYDROXYBUTYRIC ACID | 2-Hydroxybutyrate | 1.027 | 0.713 | 2.6841 | 4.1045 | 2.5884 | 3.078189 | 18 | 37 | 90 | 4.11E-05 | 0.002 | 4.39E-06 | 0.0002 | | 1 |
| β-ALANINE | beta-Ala | 1.233 | 0.919 | 5.4375 | 9.96858 | 4.5107 | 7.349677 | 17 | 36 | 87 | 3.34E-05 | 0.002 | 1.44E-05 | 0.0004 | | 1 |
| 3-METHYLHISDINE | 3-Methylhistidine | 0.085 | 0.180 | 1.2255 | 2.20901 | 1.0247 | 1.925432 | 6 | 28 | 70 | 6.88E-05 | 0.002 | 5.69E-06 | 0.0002 | | 1 |
| α-AMINOBUTYRIC ACID | 2AB | 0.791 | 0.703 | 6.2284 | 20.2287 | 5.3851 | 16.0949 | 16 | 35 | 86 | 8.39E-05 | 0.002 | 2.38E-05 | 0.0005 | | 1 |
| CADAVERINE | Cadaverine | 9.083 | 13.402 | 35.46 | 41.1071 | 39.765 | 55.12648 | 19 | 37 | 89 | 8.81E-05 | 0.002 | 2.13E-05 | 0.0005 | 78 | 1 |
| N-ACETYL-β-ALANINE | N-Acetyl-beta-alanine | 0.282 | 0.374 | 1.4267 | 2.0143 | 1.2751 | 1.666246 | 9 | 32 | 76 | 9.62E-05 | 0.002 | 4.60E-05 | 0.0008 | | 1 |
| ISETHIONIC ACID | Isethionate | 0.148 | 0.109 | 0.3581 | 0.27681 | 0.4044 | 0.804686 | 14 | 32 | 77 | 0.0001192 | 0.002 | 5.75E-05 | 0.0008 | | 1 |
| N-ACETYLPHENYLALANINE | N-Acetylphenylalanine | 0.086 | 0.116 | 0.0172 | 0.07346 | 0.0229 | 0.074225 | 10 | 3 | 10 | 0.0003692 | 0.004 | 7.54E-05 | 0.0009 | | 1 |
| TRIMETHYLLYSINE | N6,N6,N6-Trimethyllysine | 0.035 | 0.089 | 0.2699 | 0.36582 | 0.2515 | 0.342463 | 3 | 25 | 57 | 0.0003921 | 0.004 | 0.000271 | 0.0026 | | 1 |
| α-AMINOADIPIC ACID | alpha-Aminoadipate | 0.883 | 0.814 | 3.4689 | 5.42307 | 3.0669 | 6.266668 | 17 | 34 | 83 | 0.0004268 | 0.004 | 0.000311 | 0.0027 | | 1 |
| SPERMINE | Spermine | 0.065 | 0.119 | 1.3403 | 3.39481 | 1.0308 | 2.855626 | 6 | 28 | 60 | 0.0003262 | 0.004 | 0.001251 | 0.005 | 8 | 1 |
| N1-ACETYLSPERMIDINE | N1-Acetylspermidine | 0.327 | 0.345 | 1.289 | 1.74192 | 1.1973 | 1.595473 | 15 | 35 | 87 | 0.0005128 | 0.005 | 0.00023 | 0.0024 | 8 | 1 |
| CREATINE | Creatine | 10.968 | 6.522 | 34.274 | 63.8828 | 28.365 | 50.60692 | 20 | 37 | 90 | 0.0004883 | 0.005 | 0.000933 | 0.0043 | | 1 |
| γ-BUTYROBETAINE | gamma-Butyrobetaine | 1.914 | 1.542 | 7.3809 | 11.9304 | 6.1926 | 8.983804 | 20 | 36 | 89 | 0.0006073 | 0.005 | 0.000385 | 0.0028 | | 1 |
| SARCOSINE | Sarcosine | 5.133 | 4.294 | 13.808 | 18.2185 | 10.827 | 13.26566 | 20 | 37 | 90 | 0.0006649 | 0.005 | 0.002504 | 0.0087 | | 1 |
| PYRUVIC ACID | Pyruvate | 40.919 | 27.286 | 98.681 | 93.8728 | 105.45 | 133.2851 | 20 | 37 | 90 | 0.0008749 | 0.006 | 0.000421 | 0.0029 | | 1 |
| UROCANIC ACID | Urocanate | 0.995 | 1.082 | 5.054 | 13.3985 | 3.9576 | 8.875522 | 13 | 32 | 82 | 0.001326 | 0.009 | 0.000101 | 0.0011 | | 1 |
| PIPERIDINE | Piperidine | 0.073 | 0.152 | 0.3755 | 0.53702 | 0.7133 | 2.011631 | 7 | 25 | 67 | 0.0015033 | 0.01 | 5.83E-05 | 0.0008 | | 1 |
| SERINE | Ser | 10.300 | 9.622 | 34.007 | 67.4699 | 28.451 | 47.13016 | 20 | 37 | 90 | 0.0016657 | 0.01 | 0.000509 | 0.0033 | | 1 |
| HOMOVANILLIC ACID | Homovanillate | 1.721 | 0.906 | 3.8279 | 3.74161 | 4.2265 | 5.204739 | 20 | 37 | 88 | 0.0016108 | 0.01 | 0.000735 | 0.0041 | | 1 |
| 5-OXOPROLINE | 5-Oxoproline | 6.788 | 12.685 | 18.15 | 44.9634 | 14.684 | 30.06842 | 20 | 37 | 90 | 0.0018661 | 0.01 | 0.000369 | 0.0028 | | 1 |
| GABA | GABA | 1.286 | 1.299 | 2.5587 | 2.18752 | 4.4657 | 16.60331 | 20 | 36 | 89 | 0.0019086 | 0.01 | 0.000844 | 0.0042 | | 1 |
| 5-AMINOVALERIC ACID | 5-Aminovalerate | 195.795 | 209.963 | 827.22 | 1489.58 | 679.53 | 1052.043 | 20 | 37 | 89 | 0.002093 | 0.01 | 0.000323 | 0.0027 | | 1 |
| TRIMETHYLAMINE-N-OXIDE | Trimethylamine N-oxide | 0.091 | 0.195 | 0.4613 | 0.61489 | 0.4739 | 1.005668 | 4 | 24 | 61 | 0.0020797 | 0.01 | 0.000762 | 0.0041 | | 1 |
| 2-HYDROXYVALERIC ACID | 2-Hydroxypentanoate | 5.843 | 4.793 | 16.188 | 25.3406 | 15.137 | 19.67004 | 20 | 37 | 90 | 0.0023391 | 0.011 | 0.00086 | 0.0042 | | 1 |
| CARNITINE | Carnitine | 0.757 | 0.633 | 1.8914 | 2.68246 | 1.9392 | 4.54229 | 20 | 37 | 90 | 0.0025095 | 0.012 | 0.004876 | 0.0133 | | 1 |
| ISOPROPANOLAMINE | Isopropanolamine | 0.530 | 0.806 | 2.2374 | 4.13934 | 2.047 | 3.62564 | 12 | 31 | 77 | 0.0030194 | 0.012 | 0.000882 | 0.0042 | | 1 |
| THREONINE | Thr | 4.163 | 3.602 | 12.408 | 19.5441 | 12.486 | 19.3648 | 20 | 37 | 90 | 0.0029044 | 0.012 | 0.001147 | 0.0048 | 7 | 1 |
| HYPOTAURINE | Hypotaurine | 0.318 | 1.106 | 1.9495 | 2.79625 | 1.6802 | 2.564848 | 2 | 19 | 42 | 0.0028193 | 0.012 | 0.003926 | 0.0116 | | 1 |
| LACTIC ACID | Lactate | 128.842 | 80.253 | 348.15 | 495.025 | 394.62 | 807.0465 | 20 | 37 | 90 | 0.0029968 | 0.012 | 0.004292 | 0.0122 | | 1 |
| 2-HYDROXY-4-METHYLPENTANOIC ACID | 2-Hydroxy-4-methylpentanoate | 2.068 | 2.293 | 6.7127 | 12.7565 | 5.9735 | 9.612041 | 20 | 36 | 89 | 0.0033304 | 0.013 | 0.001146 | 0.0048 | | 1 |
| HYDROXYPROLINE | Hydroxyproline | 0.437 | 0.731 | 1.6485 | 3.26692 | 1.4398 | 2.683583 | 9 | 31 | 75 | 0.0035492 | 0.013 | 0.000991 | 0.0044 | | 1 |

Table 7-2

| NAME OF SUBSTANCE | | CONCENTRATION ($\mu$M) | | | | | | DETECTION RATIO (%) | | | MANN-WHITNEY TEST | | | | | |
|---|---|---|---|---|---|---|---|---|---|---|---|---|---|---|---|---|
| | | HEALTHY SUBJECT | | ONLY BEFORE TREATMENT | | BREAST CANCER INCLUDING DURING TREATMENT | | HEALTHY SUBJECT | BREAST CANCER | | ONLY BEFORE TREATMENT vs HEALTHY SUBJECT | | INCLUDING DURING TREATMENT vs HEALTHY SUBJECT | | PUBLICLY KNOWN | SIGNIFICANT |
| | | AVERAGE | STANDARD DEVIATION | AVERAGE | STANDARD DEVIATION | AVERAGE | STANDARD DEVIATION | | BEFORE TREATMENT | INCLUDING DURING TREATMENT | P VALUE | Q VALUE | P VALUE | Q VALUE | | |
| ALANINE | Ala | 21.390 | 19.430 | 67.526 | 117.162 | 69.232 | 120.485 | 20 | 37 | 90 | 0.00362 | 0.013 | 0.00148 | 0.006 | 7 | 1 |
| VALINE | Val | 11.610 | 15.718 | 49.326 | 129.44 | 41.908 | 95.743 | 20 | 37 | 90 | 0.00381 | 0.014 | 0.00064 | 0.004 | 7 | 1 |
| BUTYRIC ACID | Butanoate | 65.627 | 64.301 | 204.46 | 276.832 | 216.69 | 280.267 | 20 | 37 | 89 | 0.00424 | 0.015 | 0.00065 | 0.004 | | 1 |
| SPERMIDINE | Spermidine | 1.339 | 1.342 | 3.874 | 5.26322 | 3.3277 | 4.61563 | 20 | 36 | 88 | 0.00457 | 0.015 | 0.00821 | 0.02 | 8 | 1 |
| N8-ACETYLSPERMIDINE | N8-Acetylspermidine | 0.013 | 0.031 | 0.0715 | 0.0938 | 0.0737 | 0.11937 | 3 | 20 | 49 | 0.00497 | 0.016 | 0.00323 | 0.01 | 8 | 1 |
| ADENINE | Adenine | 0.673 | 0.482 | 1.4111 | 1.21709 | 1.2015 | 1.05126 | 16 | 34 | 81 | 0.00514 | 0.016 | 0.01372 | 0.03 | | 1 |
| PUTRESCINE | Putrescine(1,4-Butanediamine) | 39.277 | 40.268 | 182.54 | 417.293 | 137.74 | 287.799 | 20 | 37 | 89 | 0.00536 | 0.017 | 0.00201 | 0.007 | 78 | 1 |
| N6-ACETYLLYSINE | N-epsilon-Acetyllysine | 0.077 | 0.173 | 0.346 | 0.42637 | 0.3251 | 0.41918 | 4 | 22 | 51 | 0.00552 | 0.017 | 0.00453 | 0.013 | | 1 |
| 6-HYDROXYHEXANOIC ACID | 6-Hydroxyhexanoate | 0.949 | 1.156 | 0.2497 | 0.66804 | 0.4526 | 0.93174 | 9 | 5 | 19 | 0.00624 | 0.019 | 0.02908 | 0.058 | | 1 |
| PROPIONIC ACID | Propionate | 212.587 | 228.193 | 546.67 | 646.293 | 494.44 | 565.74 | 20 | 37 | 89 | 0.00642 | 0.019 | 0.00394 | 0.012 | | 1 |
| BETAINE | Betaine | 3.025 | 3.596 | 5.3352 | 5.90053 | 8.0909 | 19.3663 | 18 | 36 | 88 | 0.00688 | 0.02 | 0.00347 | 0.011 | | 1 |
| N-ACETYLPUTRESCINE | N-Acetylputrescine | 2.272 | 2.472 | 8.8389 | 21.6884 | 7.1087 | 15.0297 | 20 | 36 | 89 | 0.00709 | 0.02 | 0.00315 | 0.01 | | 1 |
| GLYCINE | Gly | 65.011 | 74.106 | 244.6 | 523.14 | 184.11 | 365.957 | 20 | 37 | 90 | 0.00727 | 0.02 | 0.00281 | 0.009 | 7 | 1 |
| HYPOXANTHINE | Hypoxanthine | 3.410 | 3.105 | 8.2703 | 9.59661 | 7.6871 | 9.55741 | 19 | 35 | 85 | 0.00905 | 0.024 | 0.01064 | 0.025 | | 1 |
| LEUCINE | Leu | 10.154 | 12.642 | 40.029 | 111.084 | 32.465 | 79.3207 | 20 | 37 | 90 | 0.00953 | 0.024 | 0.00198 | 0.007 | 7 | 1 |
| CROTONIC ACID | Crotonate | 3.972 | 4.571 | 13.987 | 16.6822 | 13.974 | 18.9801 | 13 | 32 | 75 | 0.00944 | 0.024 | 0.01306 | 0.03 | | 1 |
| ORNITHINE | Ornithine | 14.858 | 14.943 | 49.144 | 89.3732 | 38.014 | 61.7178 | 20 | 37 | 90 | 0.01051 | 0.026 | 0.00399 | 0.012 | 78 | 1 |
| ISOLEUCINE | Ile | 3.739 | 5.159 | 16.472 | 49.1296 | 13.323 | 34.8535 | 20 | 37 | 90 | 0.01076 | 0.026 | 0.00159 | 0.006 | 7 | 1 |
| TRYPTOPHAN | Trp | 1.021 | 1.114 | 2.3621 | 3.28089 | 2.3166 | 4.00336 | 16 | 36 | 88 | 0.01237 | 0.03 | 0.00849 | 0.02 | | 1 |
| CITRULLINE | Citrulline | 12.637 | 20.416 | 23.047 | 32.1049 | 19.889 | 24.4814 | 20 | 37 | 90 | 0.01463 | 0.034 | 0.00566 | 0.015 | | 1 |
| GLUTAMINE | Gln | 17.582 | 20.941 | 52.563 | 125.806 | 48.734 | 118.722 | 20 | 37 | 90 | 0.0164 | 0.038 | 0.0207 | 0.043 | | 1 |
| N1-, N8-DIACETYLSPERMIDINE | N1,N8-Diacetylspermidine | 0.097 | 0.090 | 0.2579 | 0.35117 | 0.2501 | 0.38753 | 15 | 32 | 76 | 0.01899 | 0.043 | 0.0671 | 0.118 | 8 | 1 |
| PROLINE | Pro | 41.381 | 58.124 | 172.84 | 450.311 | 117.48 | 298.65 | 20 | 37 | 90 | 0.0201 | 0.045 | 0.0066 | 0.017 | | 1 |
| 2-OXOISOPENTANOIC ACID | 2-Oxoisopentanoate | 0.783 | 0.465 | 1.3519 | 1.12646 | 1.3467 | 1.33154 | 17 | 34 | 81 | 0.02087 | 0.046 | 0.0153 | 0.033 | | 1 |
| GLUTAMIC ACID | Glu | 34.682 | 40.278 | 73.856 | 138.564 | 63.928 | 110.975 | 20 | 37 | 90 | 0.02196 | 0.047 | 0.02156 | 0.043 | 7 | 1 |
| 4-METHYLBENZOATE | 4-Methylbenzoate | 13.815 | 15.388 | 34.85 | 50.4018 | 30.807 | 39.5322 | 20 | 35 | 87 | 0.02558 | 0.054 | 0.00974 | 0.023 | | 1 |
| 3-(4-HYDROXYPHENYL)PROPIONIC ACID | 3-(4-Hydroxyphenyl)propionate | 5.764 | 6.256 | 18.082 | 23.9319 | 17.157 | 24.1955 | 19 | 35 | 86 | 0.02669 | 0.056 | 0.00776 | 0.019 | | 1 |
| CYSTEIC ACID | Cysteate | 0.140 | 0.129 | 0.4086 | 0.67652 | 0.3194 | 0.5148 | 13 | 27 | 60 | 0.02938 | 0.06 | 0.13727 | 0.209 | | 1 |
| AZELAIC ACID | Azelate | 0.037 | 0.129 | 0.0975 | 0.19163 | 0.0913 | 0.15074 | 2 | 15 | 39 | 0.03075 | 0.062 | 0.01386 | 0.03 | | 1 |
| RIBULOSE-5-PHOSPHORIC ACID | Ru5P | 3.152 | 2.063 | 4.9381 | 3.74484 | 4.7181 | 3.89669 | 20 | 36 | 88 | 0.03961 | 0.079 | 0.04804 | 0.09 | | 1 |
| PICOLINIC ACID | Pipecolate | 0.622 | 0.813 | 0.9077 | 0.96916 | 0.8528 | 0.87247 | 17 | 34 | 83 | 0.0403 | 0.079 | 0.0329 | 0.063 | | 1 |
| PHENYLALANINE | Phe | 14.694 | 12.454 | 28.133 | 35.1399 | 25.384 | 29.0587 | 20 | 37 | 90 | 0.04301 | 0.083 | 0.02133 | 0.043 | | 1 |

Table 7-3

| NAME OF SUBSTANCE | | CONCENTRATION (μM) | | | | | | DETECTION RATIO(%) | | | MANN-WHITNEY TEST | | | | SIGNIFICANT PUBLICLY KNOWN |
|---|---|---|---|---|---|---|---|---|---|---|---|---|---|---|---|
| | | HEALTHY SUBJECT | | BREAST CANCER ONLY BEFORE TREATMENT | | INCLUDING DURING TREATMENT | | HEALTHY SUBJECT | BREAST CANCER ONLY BEFORE TREATMENT | INCLUDING DURING TREATMENT | ONLY BEFORE TREATMENT vs HEALTHY SUBJECT | | INCLUDING DURING TREATMENT vs HEALTHY SUBJECT | | |
| | | AVERAGE | STANDARD DEVIATION | AVERAGE | STANDARD DEVIATION | AVERAGE | STANDARD DEVIATION | | BEFORE TREATMENT | INCLUDING DURING TREATMENT | P VALUE | Q VALUE | P VALUE | Q VALUE | |
| O-PHOSPHOSERINE | O-Phosphoserine | 0.708 | 0.930 | 1.5125 | 2.1099 | 1.317 | 1.73873 | 14 | 35 | 84 | 0.054 | 0.1023 | 0.047 | 0.08962 | 1 |
| MALONIC ACID | Malonate | 0.63945 | 0.329939 | 1.0177 | 0.7514 | 1.055 | 1.06444 | 18 | 36 | 88 | 0.056 | 0.10535 | 0.017 | 0.03548 | 1 |
| HEXANOIC ACID | Hexanoate | 13.3189 | 15.28551 | 28.902 | 34.775 | 31.21 | 37.8054 | 20 | 37 | 89 | 0.062 | 0.11442 | 0.007 | 0.01869 | 1 |
| 3-PHOSPHOGLYCERIC ACID | 3PG | 3.04736 | 3.338531 | 4.1156 | 4.6142 | 3.806 | 3.88737 | 20 | 36 | 89 | 0.066 | 0.1192 | 0.082 | 0.14011 | 0 |
| N-ACETYLGLUTAMIC ACID | N-Acetylglutamate | 0.13999 | 0.127875 | 0.3581 | 0.5293 | 0.304 | 0.42572 | 15 | 30 | 78 | 0.071 | 0.12654 | 0.055 | 0.10058 | 0 |
| N-ACETYLGLUCOSAMINE-6-PHOSPHORIC ACID | N-Acetylglucosamine 6-phosphate | 0.17916 | 0.254872 | 0.3779 | 0.5735 | 0.355 | 0.66918 | 10 | 25 | 58 | 0.072 | 0.12654 | 0.145 | 0.21651 | 0 |
| 2-OXOBUTYRIC ACID | 2-Oxobutyrate | 3.12964 | 2.243161 | 5.9299 | 5.5128 | 6.043 | 6.8824 | 17 | 33 | 76 | 0.077 | 0.13429 | 0.102 | 0.17022 | 0 |
| GLYCYL-GLYCINE | Glu-Glu | 0.22668 | 0.421861 | 0.3931 | 0.5354 | 0.391 | 0.64314 | 6 | 23 | 52 | 0.084 | 0.1439 | 0.094 | 0.15826 | 0 |
| LYSINE | Lys | 36.0936 | 38.50907 | 120.17 | 311.97 | 86.9 | 213.649 | 20 | 36 | 89 | 0.088 | 0.14876 | 0.074 | 0.12852 | 0 |
| ASPARTIC ACID | Asp | 17.2927 | 16.49612 | 25.784 | 30.243 | 23.1 | 23.215 | 20 | 37 | 90 | 0.094 | 0.15744 | 0.056 | 0.10156 | 7 | 0 |
| METHIONINE | Met | 1.335 | 1.93562 | 2.2982 | 3.2471 | 2.556 | 4.53023 | 12 | 31 | 75 | 0.097 | 0.15951 | 0.058 | 0.10439 | 0 |
| P-HYDROXYPHENYLACETIC ACID | p-Hydroxyphenylacetate | 12.5798 | 24.01714 | 31.185 | 64.355 | 24 | 45.5421 | 12 | 29 | 75 | 0.101 | 0.1639 | 0.032 | 0.06241 | 1 |
| AGMATINE | Agmatine | 0.09511 | 0.084621 | 0.1607 | 0.1443 | 0.148 | 0.1435 | 13 | 28 | 66 | 0.106 | 0.16947 | 0.185 | 0.26216 | 0 |
| 2-DEOXYRIBOSE 1-PHOSPHORIC ACID | 2-Deoxyribose 1-phosphate | 0.39332 | 0.743072 | 0.8457 | 1.6264 | 0.682 | 1.22119 | 10 | 26 | 63 | 0.138 | 0.21673 | 0.107 | 0.17574 | 0 |
| PEPLOMYCIN | PEP | 0.60591 | 0.643731 | 0.9077 | 1.0752 | 0.801 | 0.88615 | 18 | 36 | 85 | 0.139 | 0.21673 | 0.162 | 0.23485 | 0 |
| DIHYDROXYACETONEPHOSPHORIC ACID | DHAP | 7.36598 | 3.854232 | 9.1677 | 5.3083 | 8.998 | 5.96742 | 20 | 37 | 90 | 0.146 | 0.22459 | 0.286 | 0.37699 | 0 |
| GLYCOLIC ACID | Glycolate | 9.57279 | 3.591728 | 12.61 | 8.9417 | 11.9 | 7.76016 | 20 | 36 | 85 | 0.16 | 0.24375 | 0.115 | 0.18355 | 0 |
| HISTAMINE | Histamine | 0.30073 | 0.439731 | 0.8814 | 1.9794 | 0.669 | 1.55616 | 13 | 30 | 77 | 0.169 | 0.25525 | 0.129 | 0.20202 | 0 |
| N-ACETYLLEUCINE | N-Acetylleucine | 0.03062 | 0.060836 | 0.1832 | 0.5739 | 0.099 | 0.38111 | 6 | 16 | 28 | 0.179 | 0.26598 | 0.67 | 0.73379 | 0 |
| CYTIDINE DISODIUM 5'-MONOPHOSPHATE | CMP | 0.25869 | 0.443067 | 1.0785 | 2.6912 | 0.973 | 2.38666 | 10 | 21 | 54 | 0.19 | 0.27997 | 0.202 | 0.28365 | 0 |
| GUANINE | Guanine | 0.71569 | 0.608172 | 1.492 | 1.7957 | 1.271 | 1.4044 | 14 | 32 | 78 | 0.217 | 0.31541 | 0.144 | 0.21651 | 0 |
| 4-METHYL-2-OXOPENTANOIC ACID | 4-Methyl-2-oxopentanoate | 1.58389 | 0.943891 | 2.3178 | 2.2787 | 2.289 | 2.07591 | 20 | 37 | 90 | 0.222 | 0.31859 | 0.113 | 0.18302 | 0 |
| N-ACETYLASPARTIC ACID | N-Acetylaspartate | 0.68824 | 0.381095 | 1.1339 | 1.3923 | 1.025 | 1.04935 | 20 | 37 | 90 | 0.225 | 0.31979 | 0.162 | 0.23485 | 0 |
| TYROSINE | Tyr | 21.4067 | 18.11086 | 35.63 | 43.578 | 31.4 | 38.7827 | 20 | 37 | 90 | 0.229 | 0.32104 | 0.227 | 0.31474 | 7 | 0 |
| SUCCINIC ACID | Succinate | 19.5264 | 17.89444 | 35.029 | 69.034 | 42.57 | 100.728 | 20 | 37 | 90 | 0.238 | 0.32735 | 0.24 | 0.32643 | 0 |
| GLYCEROPHOSPHORIC ACID | Glycerophosphate | 8.39508 | 4.672054 | 9.4613 | 4.481 | 10.02 | 7.03863 | 20 | 37 | 90 | 0.238 | 0.32735 | 0.287 | 0.37699 | 0 |
| ALANYL-ALANINE | Ala-Ala | 0.77481 | 0.970735 | 1.2475 | 1.6193 | 1.115 | 1.47621 | 13 | 27 | 62 | 0.253 | 0.34319 | 0.378 | 0.47192 | 0 |
| 1,3-DIAMINOPROPANE | 1,3-Diaminopropane | 1.50535 | 1.791791 | 2.2101 | 2.494 | 1.983 | 2.11129 | 12 | 29 | 71 | 0.275 | 0.3699 | 0.278 | 0.37322 | 8 | 0 |
| 3-PHENYLPROPIONIC ACID | 3-Phenylpropionate | 9.9887 | 10.32626 | 17.117 | 20.316 | 18.22 | 21.885 | 20 | 31 | 80 | 0.296 | 0.38057 | 0.126 | 0.19886 | 0 |
| CIS-ACONITATE | cis-Aconitate | 0.12953 | 0.117343 | 0.2169 | 0.3154 | 0.35 | 0.94761 | 12 | 30 | 68 | 0.298 | 0.38057 | 0.134 | 0.20719 | 0 |

EP 3 578 984 A2

Table 7-4

A substance for which "7" or "8" is indicated in the column labeled "Publicly Known" is a known substance

| NAME OF SUBSTANCE | | CONCENTRATION (μM) | | | | | | DETECTION RATIO (%) | | | MANN-WHITNEY TEST | | | | PUBLICLY KNOWN | SIGNIFICANT |
|---|---|---|---|---|---|---|---|---|---|---|---|---|---|---|---|---|
| | | HEALTHY SUBJECT | | ONLY BEFORE TREATMENT | | BREAST CANCER INCLUDING DURING TREATMENT | | HEALTHY SUBJECT | BREAST CANCER ONLY BEFORE TREATMENT | INCLUDING DURING TREATMENT | BEFORE TREATMENT vs HEALTHY SUBJECT | | INCLUDING DURING TREATMENT vs HEALTHY SUBJECT | | | |
| | | AVERAGE | STANDARD DEVIATION | AVERAGE | STANDARD DEVIATION | AVERAGE | STANDARD DEVIATION | | BEFORE TREATMENT | INCLUDING DURING TREATMENT | P VALUE | Q VALUE | P VALUE | Q VALUE | | |
| 3-HYDROXYBUTYRIC ACID | 3-Hydroxybutyrate | 6.65264 | 4.679435 | 8.1673 | 5.6145 | 9.119 | 8.0353 | 20 | 37 | 90 | 0.288 | 0.38057 | 0.179 | 0.2567 | | 0 |
| BENZOIC ACID | Benzoate | 11.5158 | 4.957046 | 9.6971 | 6.7622 | 10.98 | 8.65846 | 18 | 27 | 67 | 0.289 | 0.38057 | 0.54 | 0.64014 | | 0 |
| GUANOSINE | Guanosine | 0.18071 | 0.208922 | 0.3316 | 0.4262 | 0.313 | 0.52441 | 10 | 19 | 42 | 0.297 | 0.38057 | 0.675 | 0.73379 | | 0 |
| 6-PHOSPHOGLUCONIC ACID | 6-Phosphogluconate | 0.45188 | 0.420291 | 0.6064 | 0.5832 | 0.547 | 0.59845 | 16 | 33 | 77 | 0.319 | 0.40265 | 0.655 | 0.73117 | | 0 |
| URIDYLIC ACID | UMP | 0.12161 | 0.168081 | 0.2179 | 0.2884 | 0.313 | 0.63248 | 8 | 18 | 42 | 0.324 | 0.40524 | 0.341 | 0.43454 | | 0 |
| ADENOSINE | Adenosine | 0.14107 | 0.164335 | 0.1919 | 0.2021 | 0.163 | 0.18723 | 11 | 22 | 49 | 0.356 | 0.44105 | 0.613 | 0.70354 | | 0 |
| MUCIC ACID | Mucate | 0.24406 | 0.271429 | 0.1807 | 0.2273 | 0.283 | 0.54345 | 11 | 18 | 49 | 0.465 | 0.56994 | 0.687 | 0.74006 | | 0 |
| ETHANOLAMINEPHOSPHORIC ACID | Ethanolamine phosphate | 32.346 | 26.12692 | 60.343 | 94.201 | 54.75 | 82.4473 | 18 | 34 | 84 | 0.558 | 0.67738 | 0.543 | 0.64014 | | 0 |
| ADIPATE | Adipate | 0.49755 | 0.32343 | 0.5903 | 0.531 | 0.562 | 0.39362 | 19 | 35 | 87 | 0.581 | 0.69792 | 0.319 | 0.41063 | | 0 |
| 2-ISOPROPYLMALATE | 2-Isopropylmalate | 0.25413 | 0.194751 | 0.2663 | 0.1619 | 0.261 | 0.18967 | 20 | 36 | 87 | 0.586 | 0.69803 | 0.913 | 0.95163 | | 0 |
| PHOSPHORYLCHLORINE | Phosphorylcholine | 5.9658 | 5.079958 | 5.9868 | 6.9568 | 6.471 | 7.37476 | 20 | 31 | 81 | 0.604 | 0.71215 | 0.914 | 0.95163 | | 0 |
| NICOTINATE | Nicotinate | 1.84972 | 1.398171 | 2.2547 | 2.2648 | 2.606 | 2.66505 | 16 | 23 | 63 | 0.628 | 0.73378 | 0.386 | 0.47816 | | 0 |
| 1-METHYL-2-PYRROLIDINONE | 1-Methyl-2-pyrrolidinone | 23.0783 | 14.05832 | 30.672 | 33.782 | 27.93 | 28.4191 | 20 | 36 | 87 | 0.64 | 0.74026 | 0.978 | 0.98625 | | 0 |
| MALIC ACID | Malate | 1.63046 | 0.501086 | 2.4789 | 2.9025 | 2.689 | 2.85035 | 20 | 37 | 90 | 0.651 | 0.74644 | 0.23 | 0.31652 | | 0 |
| PANTOTHENIC ACID | Pantothenate | 0.15636 | 0.284826 | 0.2627 | 0.5432 | 0.276 | 0.49056 | 7 | 14 | 42 | 0.678 | 0.76994 | 0.313 | 0.40777 | | 0 |
| N-ACETYLNEURAMINATE | N-Acetylneuraminate | 49.3584 | 36.98463 | 43.496 | 30.441 | 42.27 | 34.3608 | 20 | 37 | 90 | 0.744 | 0.80908 | 0.464 | 0.56853 | | 0 |
| HISTAMINE | His | 11.4764 | 8.07934 | 16.58 | 20.947 | 15.95 | 19.4737 | 20 | 37 | 90 | 0.738 | 0.80908 | 0.551 | 0.64092 | | 0 |
| FUMARIC ACID | Fumarate | 0.35582 | 0.233544 | 0.4677 | 0.5926 | 0.57 | 0.68895 | 15 | 30 | 72 | 0.743 | 0.80908 | 0.554 | 0.64092 | | 0 |
| 2-DEOXYGLUCOSE-6-PHOSPHORIC ACID | 2-Deoxyglucose 6-phosphate | 0.35743 | 0.74316 | 0.4996 | 1.0832 | 0.37 | 0.83301 | 5 | 11 | 24 | 0.744 | 0.80908 | 0.972 | 0.98625 | | 0 |
| CITRIC ACID | Citrate | 4.60257 | 3.675132 | 4.8562 | 6.0137 | 8.051 | 19.8864 | 20 | 35 | 86 | 0.732 | 0.80908 | 1 | 1 | | 0 |
| 4-ACETYLBUTYRIC ACID | 4-Acetylbutyrate | 0.09595 | 0.160288 | 0.0775 | 0.1312 | 0.072 | 0.12937 | 6 | 11 | 25 | 0.772 | 0.82508 | 0.625 | 0.71055 | | 0 |
| O-ACETYLCARNITINE | o-Acetylcarnitine | 0.93204 | 0.530378 | 1.3934 | 1.8255 | 1.283 | 1.90322 | 20 | 37 | 89 | 0.77 | 0.82508 | 0.96 | 0.98625 | | 0 |
| N-ACETYLGLUCOSAMINE-1-PHOSPHORIC ACID | N-Acetylglucosamine 1-phosphate | 0.28012 | 0.263447 | 0.265 | 0.3282 | 0.239 | 0.25934 | 17 | 26 | 63 | 0.781 | 0.82726 | 0.675 | 0.73379 | | 0 |
| SEDOHEPTULOSE-7-PHOSPHORIC ACID | S7P | 0.49477 | 0.412779 | 0.5007 | 0.3721 | 0.564 | 0.45267 | 16 | 29 | 72 | 0.814 | 0.85505 | 0.518 | 0.62283 | | 0 |
| HEPTANOIC ACID | Heptanoate | 0.23138 | 0.200911 | 0.2286 | 0.1788 | 0.215 | 0.22231 | 15 | 25 | 56 | 0.826 | 0.85996 | 0.654 | 0.73117 | | 0 |
| CREATININE | Creatinine | 3.47024 | 1.223945 | 4.3163 | 3.7725 | 4.805 | 8.00241 | 20 | 37 | 90 | 0.867 | 0.89576 | 0.877 | 0.92877 | | 0 |
| 2,5-DIHYDROXYBENZONATE | 2,5-Dihydroxybenzoate | 0.67706 | 1.220304 | 0.6312 | 1.125 | 0.563 | 0.97834 | 9 | 17 | 38 | 0.898 | 0.92041 | 0.767 | 0.81912 | | 0 |
| CYTIDINE | Cytidine | 0.2924 | 0.261356 | 0.4567 | 0.6785 | 0.386 | 0.62379 | 14 | 22 | 49 | 0.959 | 0.96668 | 0.508 | 0.6165 | | 0 |
| ARGININE | Arg | 12.0359 | 6.289379 | 15.501 | 15.934 | 14.71 | 14.7857 | 20 | 37 | 90 | 0.953 | 0.96668 | 0.972 | 0.98625 | | 0 |
| SYRINGIC ACID | Syringate | 1.13065 | 0.376083 | 1.2108 | 0.7313 | 1.106 | 0.67811 | 20 | 34 | 82 | 0.993 | 0.99331 | 0.374 | 0.47183 | | 0 |

disclosed in Non-Patent Literature 7 or 8.

Next, a network diagram in which a line is drawn between metabolites exhibiting a correlation between metabolites in the patients with breast cancer before initiation of treatment (37 cases) and metabolites in the healthy subjects (20 cases) of $R^2 > 0.92$ shown in FIG. 10 is shown. Among the substances, a substance that formed bonding lines to many substances and could be detected in all of the samples, or glutamine (Gln), was selected as a concentration-correcting substance. In the drawing, the substance is circled.

Substances in which a relative concentration exhibits a statistical significant difference ($p < 0.05$ in the Mann-Whitney test) between the patients with breast cancer and the healthy subjects are shown in Tables 8-1, 8-2, 8-3, and 8-4. In calculating the relative concentration, the concentration of each substance was divided by the concentration of glutamine, and the value was expressed with no units.

At that time, many of the metabolites included in saliva were measured. In order to calculate the significant difference of each substance, independent statistics (for example, Mann-Whitney test) needs to be repeated. When the test is repeated at a level of significance $\alpha$ of 0.05, null hypothesis that is accidentally dismissed is increased. Therefore, the P value was corrected by the false discovery rate (FDR) method (Storey, J. D., & Tibshirani, R. (2003). Statistical significance for genomewide studies. Proceedings of the National academy of Sciences of the United States of America, 100, 9440-9445), and a Q value was calculated. For example, when the Q value is 0.5, true null hypothesis occupies a half of the null hypothesis that is dismissed at $P < 0.05$.

Table 8-1

Table 8-2

| NAME OF SUBSTANCE | | RELATIVE CONCENTRATION (NO UNIT) | | | | | | DETECTION RATIO (%) | | | MANN-WHITNEY TEST | | | | SIGNIFICANT |
|---|---|---|---|---|---|---|---|---|---|---|---|---|---|---|---|
| | | HEALTHY SUBJECT | | BREAST CANCER ONLY BEFORE TREATMENT | | INCLUDING DURING TREATMENT | | HEALTHY SUBJECT | BREAST CANCER | | ONLY BEFORE TREATMENT vs HEALTHY SUBJECT | | INCLUDING DURING TREATMENT vs HEALTHY SUBJECT | | PUBLICLY KNOWN |
| | | AVERAGE | STANDARD DEVIATION | AVERAGE | STANDARD DEVIATION | AVERAGE | STANDARD DEVIATION | | BEFORE TREATMENT | INCLUDING DURING TREATMENT | P VALUE | Q VALUE | P VALUE | Q VALUE | |
| CHOLINE | Choline | 0.535 | 0.365 | 0.6695 | 0.39647 | 0.7124 | 0.392 | 20 | 37 | 90 | 0.20875 | 0.42434 | 0.042705 | 0.129157 | 1 |
| 2-HYDROXYBUTYRIC ACID | 2-Hydroxybutyrate | 0.126 | 0.110 | 0.1339 | 0.14048 | 0.1438 | 0.132294 | 18 | 37 | 90 | 0.7955 | 0.90497 | 0.695525 | 0.821382 | 0 |
| β-ALANINE | beta-Ala | 0.102 | 0.081 | 0.1503 | 0.07955 | 0.1676 | 0.118533 | 17 | 36 | 87 | 0.02613 | 0.12032 | 0.010775 | 0.049363 | 1 |
| 3-METHYLHISTDINE | 3-Methylhistidine | 0.007 | 0.016 | 0.0294 | 0.03108 | 0.0331 | 0.038386 | 6 | 28 | 70 | 0.00022 | 0.00989 | 2.52E-05 | 0.001565 | 1 |
| α-AMINOBUTYRIC ACID | 2AB | 0.057 | 0.043 | 0.0989 | 0.05536 | 0.1191 | 0.102119 | 16 | 35 | 86 | 0.00841 | 0.0652 | 0.001245 | 0.02205 | 1 |
| CADAVERINE | Cadaverine | 0.602 | 0.539 | 1.2257 | 0.86809 | 1.6484 | 1.741134 | 19 | 37 | 89 | 0.00496 | 0.05 | 0.001936 | 0.025253 | 78 | 1 |
| N-ACETYL-β-ALANINE | N-Acetyl-beta-alanine | 0.021 | 0.031 | 0.0409 | 0.03495 | 0.0424 | 0.033048 | 9 | 32 | 76 | 0.01176 | 0.07673 | 0.003595 | 0.03184 | 1 |
| ISETHIONIC ACID | Isethionate | 0.017 | 0.016 | 0.0162 | 0.01528 | 0.0178 | 0.025157 | 14 | 32 | 77 | 0.88658 | 0.93166 | 0.891843 | 0.929316 | 0 |
| N-ACETYLPHENYLALANINE | N-Acetylphenylalanine | 0.007 | 0.011 | 0.0003 | 0.00108 | 0.0007 | 0.002282 | 10 | 3 | 10 | 0.00017 | 0.00989 | 2.41E-05 | 0.001565 | 1 |
| TRIMETHYLLYSINE | N6,N6,N6-Trimethyllysine | 0.001 | 0.004 | 0.0071 | 0.00828 | 0.0075 | 0.008624 | 3 | 25 | 57 | 0.00045 | 0.01366 | 0.000233 | 0.009643 | 1 |
| α-AMINOADIPIC ACID | alpha-Aminoadipate | 0.073 | 0.056 | 0.089 | 0.05484 | 0.095 | 0.065314 | 17 | 34 | 83 | 0.2996 | 0.53842 | 0.182378 | 0.337535 | 0 |
| SPERMINE | Spermine | 0.004 | 0.007 | 0.0207 | 0.02522 | 0.0199 | 0.030295 | 6 | 28 | 60 | 0.00077 | 0.01585 | 0.001713 | 0.025253 | 8 | 1 |
| N1-ACETYLSPERMIDINE | N1-Acetylspermidine | 0.025 | 0.026 | 0.0386 | 0.0295 | 0.0425 | 0.032472 | 15 | 35 | 87 | 0.07745 | 0.20434 | 0.019641 | 0.073801 | 8 | 1 |
| CREATINE | Creatine | 1.034 | 0.560 | 0.9723 | 0.44164 | 1.0219 | 0.578194 | 20 | 37 | 90 | 0.8749 | 0.92725 | 0.741877 | 0.824197 | 0 |
| γ-BUTYROBETAINE | gamma-Butyrobetaine | 0.154 | 0.089 | 0.2152 | 0.14508 | 0.232 | 0.161565 | 20 | 36 | 89 | 0.15403 | 0.34726 | 0.076587 | 0.202061 | 0 |
| SARCOSINE | Sarcosine | 0.416 | 0.199 | 0.4475 | 0.25949 | 0.4453 | 0.284517 | 20 | 37 | 90 | 0.78449 | 0.90322 | 0.990725 | 0.990725 | 0 |
| PYRUVIC ACID | Pyruvate | 3.853 | 2.269 | 4.1335 | 2.94794 | 4.6006 | 3.016964 | 20 | 37 | 90 | 1 | 1 | 0.459233 | 0.618967 | 0 |
| UROCANIC ACID | Urocanate | 0.070 | 0.083 | 0.1517 | 0.19424 | 0.1848 | 0.27465 | 13 | 32 | 82 | 0.05766 | 0.17023 | 0.004244 | 0.035084 | 1 |
| PIPERIDINE | Piperidine | 0.006 | 0.011 | 0.0159 | 0.02094 | 0.0348 | 0.107037 | 7 | 25 | 67 | 0.02259 | 0.11672 | 0.001153 | 0.02205 | 1 |
| SERINE | Ser | 0.708 | 0.279 | 0.9755 | 0.86402 | 1.0627 | 1.0582 | 20 | 37 | 90 | 0.39241 | 0.63739 | 0.152761 | 0.315079 | 0 |
| HOMOVANILLIC ACID | Homovanillate | 0.155 | 0.073 | 0.143 | 0.07656 | 0.1857 | 0.14732 | 20 | 37 | 88 | 0.63652 | 0.81967 | 0.97836 | 0.986315 | 0 |
| 5-OXOPROLINE | 5-Oxoproline | 0.568 | 0.887 | 0.556 | 0.62212 | 0.7212 | 1.230777 | 20 | 37 | 90 | 0.81009 | 0.91124 | 0.249793 | 0.407335 | 0 |
| GABA | GABA | 0.100 | 0.046 | 0.1004 | 0.06762 | 0.1752 | 0.460201 | 20 | 36 | 89 | 0.66039 | 0.8356 | 0.650285 | 0.77534 | 0 |
| 5-AMINOVALERIC ACID | 5-Aminovalerate | 13.413 | 11.107 | 23.718 | 19.2024 | 27.708 | 23.34253 | 20 | 37 | 89 | 0.03978 | 0.13186 | 0.008131 | 0.049363 | 1 |
| TRIMETHYLAMINE-N-OXIDE | Trimethylamine N-oxide | 0.014 | 0.030 | 0.0244 | 0.03508 | 0.0222 | 0.031137 | 4 | 24 | 61 | 0.01743 | 0.09637 | 0.006256 | 0.0431 | 1 |
| 2-HYDROXYVALERIC ACID | 2-Hydroxypentanoate | 0.503 | 0.284 | 0.5272 | 0.35447 | 0.6226 | 0.481419 | 20 | 37 | 90 | 0.91422 | 0.95264 | 0.579501 | 0.717992 | 0 |
| CARNITINE | Carnitine | 0.064 | 0.028 | 0.0598 | 0.0358 | 0.0589 | 0.030769 | 20 | 37 | 90 | 0.32275 | 0.56367 | 0.29367 | 0.444087 | 0 |
| ISOPROPANOLAMINE | Isopropanolamine | 0.040 | 0.053 | 0.061 | 0.07286 | 0.0734 | 0.084822 | 12 | 31 | 77 | 0.1805 | 0.37303 | 0.038945 | 0.120731 | 1 |
| THREONINE | Thr | 0.286 | 0.085 | 0.3742 | 0.24147 | 0.4102 | 0.288762 | 20 | 37 | 90 | 0.40171 | 0.63862 | 0.086054 | 0.213453 | 7 | 0 |
| HYPOTAURINE | Hypotaurine | 0.007 | 0.020 | 0.0487 | 0.06827 | 0.05 | 0.072847 | 2 | 19 | 42 | 0.00265 | 0.04689 | 0.002568 | 0.028374 | 1 |
| LACTIC ACID | Lactate | 13.476 | 7.556 | 13.847 | 10.6027 | 15.565 | 17.77329 | 20 | 37 | 90 | 0.75912 | 0.90322 | 0.736028 | 0.824197 | 0 |
| 2-HYDROXY-4-METHYLPENTANOIC ACID | 2-Hydroxy-4-methylpentanoate | 0.144 | 0.093 | 0.1792 | 0.1286 | 0.2112 | 0.174675 | 20 | 36 | 89 | 0.48063 | 0.70115 | 0.168946 | 0.331163 | 0 |
| HYDROXYPROLINE | Hydroxyproline | 0.025 | 0.041 | 0.048 | 0.08291 | 0.0459 | 0.059641 | 9 | 31 | 75 | 0.04115 | 0.13186 | 0.010739 | 0.049363 | 1 |
| ALANINE | Ala | 1.408 | 0.577 | 1.7516 | 0.78559 | 2.0262 | 1.3785 | 20 | 37 | 90 | 0.08353 | 0.2115 | 0.012718 | 0.053624 | 7 | 1 |

EP 3 578 984 A2

Table 8-3

| NAME OF SUBSTANCE | | RELATIVE CONCENTRATION (NO UNIT) | | | | | | DETECTION RATIO (%) | | | MANN-WHITNEY TEST | | | | | |
|---|---|---|---|---|---|---|---|---|---|---|---|---|---|---|---|---|
| | | HEALTHY SUBJECT | | BREAST CANCER | | | | HEALTHY SUBJECT | BREAST CANCER | | ONLY BEFORE TREATMENT vs HEALTHY SUBJECT | | INCLUDING DURING TREATMENT vs HEALTHY SUBJECT | | PUBLICLY KNOWN | SIGNIFICANT |
| | | | | ONLY BEFORE TREATMENT | | INCLUDING DURING TREATMENT | | | BEFORE TREATMENT | INCLUDING DURING TREATMENT | | | | | | |
| | | AVERAGE | STANDARD DEVIATION | AVERAGE | STANDARD DEVIATION | AVERAGE | STANDARD DEVIATION | | | | P VALUE | Q VALUE | P VALUE | Q VALUE | | |
| VALINE | Val | 0.655 | 0.505 | 0.92 | 0.4944 | 1.085 | 0.7477 | 20 | 37 | 90 | 0.01043 | 0.0719 | 0.0009 | 0.022 | 7 | 1 |
| BUTYRIC ACID | Butanoate | 5.412 | 4.454 | 8.34 | 9.2551 | 10.66 | 12.991 | 20 | 37 | 89 | 0.53387 | 0.7523 | 0.10781 | 0.2476 | | 0 |
| SPERMIDINE | Spermidine | 0.106 | 0.082 | 0.12 | 0.0928 | 0.12 | 0.0849 | 20 | 36 | 88 | 0.54486 | 0.7532 | 0.43606 | 0.6075 | 8 | 0 |
| N8-ACETYLSPERMIDINE | N8-Acetylspermidine | 0.001 | 0.001 | 0 | 0.0025 | 0.002 | 0.003 | 3 | 20 | 49 | 0.00706 | 0.0584 | 0.00275 | 0.0284 | 8 | 1 |
| ADENINE | Adenine | 0.055 | 0.046 | 0.05 | 0.0394 | 0.058 | 0.0424 | 16 | 34 | 81 | 1 | 1 | 0.72416 | 0.8242 | | 0 |
| PUTRESCINE | Putrescine(1,4-Butanediamine) | 2.585 | 2.076 | 3.86 | 2.4115 | 4.412 | 3.3521 | 20 | 37 | 89 | 0.03978 | 0.1319 | 0.01115 | 0.0494 | 78 | 1 |
| N6-ACETYLLYSINE | N-epsilon-Acetyllysine | 0.004 | 0.009 | 0.01 | 0.0113 | 0.01 | 0.0126 | 4 | 22 | 51 | 0.01409 | 0.0832 | 0.00812 | 0.0494 | | 1 |
| 6-HYDROXYHEXANOIC ACID | 6-Hydroxyhexanoate | 0.103 | 0.142 | 0.01 | 0.0439 | 0.027 | 0.0732 | 9 | 5 | 19 | 0.00361 | 0.05 | 0.00982 | 0.0494 | | 1 |
| PROPIONIC ACID | Propionate | 15.110 | 13.467 | 19.6 | 16.982 | 21.9 | 19.007 | 20 | 37 | 89 | 0.3653 | 0.6205 | 0.12208 | 0.2703 | | 0 |
| BETAINE | Betaine | 0.235 | 0.161 | 0.21 | 0.1575 | 0.367 | 0.9139 | 18 | 36 | 88 | 0.4568 | 0.6824 | 0.86766 | 0.9209 | | 0 |
| N-ACETYLPUTRESCINE | N-Acetylputrescine | 0.140 | 0.098 | 0.2 | 0.1397 | 0.227 | 0.1674 | 20 | 36 | 89 | 0.11859 | 0.2723 | 0.02693 | 0.092 | | 1 |
| GLYCINE | Gly | 3.852 | 1.481 | 4.95 | 2.8102 | 5.317 | 3.6606 | 20 | 37 | 90 | 0.17447 | 0.373 | 0.04598 | 0.1357 | 7 | 1 |
| HYPOXANTHINE | Hypoxanthine | 0.255 | 0.144 | 0.27 | 0.2042 | 0.279 | 0.2112 | 19 | 35 | 85 | 0.83443 | 0.9112 | 0.95673 | 0.9724 | | 0 |
| LEUCINE | Leu | 0.551 | 0.355 | 0.69 | 0.3443 | 0.823 | 0.5284 | 20 | 37 | 90 | 0.05291 | 0.164 | 0.00521 | 0.0404 | 7 | 1 |
| CROTONIC ACID | Crotonate | 0.323 | 0.408 | 0.77 | 1.2057 | 0.785 | 1.1358 | 13 | 32 | 75 | 0.08358 | 0.2115 | 0.03365 | 0.107 | | 1 |
| ORNITHINE | Ornithine | 1.004 | 0.517 | 1.34 | 1.0714 | 1.364 | 1.0031 | 20 | 37 | 90 | 0.48063 | 0.7012 | 0.23726 | 0.3974 | 78 | 0 |
| ISOLEUCINE | Ile | 0.187 | 0.134 | 0.25 | 0.1419 | 0.304 | 0.2163 | 20 | 37 | 90 | 0.04147 | 0.1319 | 0.00327 | 0.0312 | 7 | 1 |
| TRYPTOPHAN | Trp | 0.063 | 0.054 | 0.07 | 0.0479 | 0.08 | 0.0572 | 16 | 36 | 88 | 0.4367 | 0.6704 | 0.16891 | 0.3312 | | 0 |
| CITRULLINE | Citrulline | 0.631 | 0.477 | 0.66 | 0.4836 | 0.711 | 0.4288 | 20 | 37 | 90 | 0.69684 | 0.8555 | 0.20512 | 0.3634 | | 0 |
| GLUTAMINE | Gln | 1.000 | 0.000 | 1 | 0 | 1 | 0 | 20 | 37 | 90 | NA | NA | NA | NA | | 0 |
| N1-,N8-DIACETYLSPERMIDINE | N1,N8-Diacetylspermidine | 0.006 | 0.005 | 0.01 | 0.0074 | 0.008 | 0.008 | 15 | 32 | 76 | 0.56865 | 0.7664 | 0.43032 | 0.6064 | 8 | 0 |
| PROLINE | Pro | 2.119 | 0.813 | 3.11 | 3.2776 | 3.232 | 3.1517 | 20 | 37 | 90 | 0.77177 | 0.9032 | 0.253 | 0.4073 | | 0 |
| 2-OXOISOPENTANOIC ACID | 2-Oxoisopentanoate | 0.082 | 0.058 | 0.06 | 0.0537 | 0.066 | 0.0518 | 17 | 34 | 81 | 0.29187 | 0.535 | 0.22048 | 0.3797 | | 0 |
| GLUTAMIC ACID | Glu | 2.005 | 0.996 | 1.91 | 1.0563 | 2.005 | 1.1163 | 20 | 37 | 90 | 0.68461 | 0.8518 | 0.86767 | 0.9209 | 7 | 0 |
| 4-METHYLBENZOATE | 4-Methylbenzoate | 1.109 | 1.178 | 1.19 | 1.201 | 1.376 | 1.3512 | 20 | 35 | 87 | 0.71922 | 0.8743 | 0.34639 | 0.5053 | | 0 |
| 3-(4-HYDROXYPHENYL)PROPIONIC ACID | 3-(4-Hydroxyphenyl)propionate | 0.456 | 0.466 | 0.67 | 0.8923 | 0.711 | 0.7999 | 19 | 35 | 86 | 0.55276 | 0.7532 | 0.17378 | 0.3312 | | 0 |
| CYSTEIC ACID | Cysteate | 0.014 | 0.014 | 0.01 | 0.0115 | 0.011 | 0.0118 | 13 | 27 | 60 | 0.64119 | 0.8197 | 0.5094 | 0.6512 | | 0 |
| AZELAIC ACID | Azelate | 0.003 | 0.010 | 0 | 0.0096 | 0.006 | 0.0124 | 2 | 15 | 39 | 0.03779 | 0.1319 | 0.0165 | 0.0639 | | 1 |
| RIBULOSE-5-PHOSPHORIC ACID | Ru5P | 0.292 | 0.160 | 0.23 | 0.1685 | 0.245 | 0.1554 | 20 | 36 | 88 | 0.15896 | 0.352 | 0.25623 | 0.4073 | | 0 |
| PICOLINIC ACID | Pipecolate | 0.060 | 0.109 | 0.03 | 0.0234 | 0.037 | 0.0289 | 17 | 34 | 83 | 0.41234 | 0.6472 | 0.72137 | 0.8242 | | 0 |
| PHENYLALANINE | Phe | 1.085 | 0.645 | 0.88 | 0.4717 | 1.039 | 0.652 | 20 | 37 | 90 | 0.24078 | 0.4665 | 0.58482 | 0.718 | | 0 |

Table 8-4

| NAME OF SUBSTANCE | | RELATIVE CONCENTRATION (NO UNIT) | | | | | | DETECTION RATIO (%) | | | MANN-WHITNEY TEST | | | | PUBLICLY KNOWN | SIGNIFICANT |
| | | HEALTHY SUBJECT | | BREAST CANCER ONLY BEFORE TREATMENT | | INCLUDING DURING TREATMENT | | HEALTHY SUBJECT | BREAST CANCER BEFORE TREATMENT | INCLUDING DURING TREATMENT | ONLY BEFORE TREATMENT vs HEALTHY SUBJECT | | INCLUDING DURING TREATMENT vs HEALTHY SUBJECT | | | |
| | | AVERAGE | STANDARD DEVIATION | AVERAGE | STANDARD DEVIATION | AVERAGE | STANDARD DEVIATION | | | | P VALUE | Q VALUE | P VALUE | Q VALUE | | |
|---|---|---|---|---|---|---|---|---|---|---|---|---|---|---|---|---|
| O-PHOSPHOSERINE | O-Phosphoserine | 0.045 | 0.060 | 0.045 | 0.0378 | 0.0488 | 0.03918 | 14 | 35 | 84 | 0.2802 | 0.52635 | 0.17599 | 0.3312 | | 0 |
| MALONIC ACID | Malonate | 0.0721 | 0.05367 | 0.046 | 0.0335 | 0.0578 | 0.04882 | 18 | 36 | 88 | 0.0961 | 0.23376 | 0.24034 | 0.3974 | | 0 |
| HEXANOIC ACID | Hexanoate | 1.1716 | 1.30695 | 1.794 | 2.2027 | 2.0692 | 2.44959 | 20 | 37 | 89 | 0.7845 | 0.90322 | 0.17626 | 0.3312 | | 0 |
| 3-PHOSPHOGLYCERIC ACID | 3PG | 0.2068 | 0.12486 | 0.164 | 0.1395 | 0.1728 | 0.13155 | 20 | 36 | 89 | 0.0898 | 0.22266 | 0.155 | 0.3151 | | 0 |
| N-ACETYLGLUTAMIC ACID | N-Acetylglutamate | 0.0101 | 0.00855 | 0.01 | 0.008 | 0.0107 | 0.00868 | 15 | 30 | 78 | 0.9732 | 0.99073 | 0.74142 | 0.8242 | | 0 |
| N-ACETYLGLUCOSAMINE-6-PHOSPHORIC ACID | N-Acetylglucosamine 6-phosphate | 0.0135 | 0.01997 | 0.012 | 0.0129 | 0.0121 | 0.01325 | 10 | 25 | 58 | 0.5296 | 0.75227 | 0.57941 | 0.718 | | 0 |
| 2-OXOBUTYRIC ACID | 2-Oxobutyrate | 0.3398 | 0.32933 | 0.288 | 0.3022 | 0.2977 | 0.31856 | 17 | 33 | 76 | 0.5524 | 0.75321 | 0.48712 | 0.6406 | | 0 |
| GLYCYL-GLYCINE | Glu-Glu | 0.007 | 0.01345 | 0.011 | 0.0141 | 0.0119 | 0.01543 | 6 | 23 | 52 | 0.0764 | 0.20434 | 0.05891 | 0.1588 | | 0 |
| LYSINE | Lys | 2.37 | 1.17409 | 2.109 | 1.2091 | 2.2092 | 1.19698 | 20 | 36 | 89 | 0.3653 | 0.62051 | 0.62264 | 0.7496 | | 0 |
| ASPARTIC ACID | Asp | 1.2001 | 0.49598 | 0.926 | 0.6067 | 1.0226 | 0.60552 | 20 | 37 | 90 | 0.0295 | 0.1214 | 0.09959 | 0.233 | 7 | 1 |
| METHIONINE | Met | 0.063 | 0.07532 | 0.073 | 0.073 | 0.0827 | 0.10958 | 12 | 31 | 75 | 0.3856 | 0.63739 | 0.3171 | 0.4737 | | 0 |
| P-HYDROXYPHENYLACETIC ACID | p-Hydroxyphenylacetate | 0.8015 | 1.44237 | 0.77 | 0.9516 | 0.8143 | 0.8509 | 12 | 29 | 75 | 0.3063 | 0.54264 | 0.128 | 0.2784 | | 0 |
| AGMATINE | Agmatine | 0.0072 | 0.00696 | 0.007 | 0.0084 | 0.0072 | 0.00815 | 13 | 28 | 66 | 0.8525 | 0.91124 | 0.87852 | 0.9232 | | 0 |
| 2-DEOXYRIBOSE 1-PHOSPHORIC ACID | 2-Deoxyribose 1-phosphate | 0.0246 | 0.04973 | 0.022 | 0.0305 | 0.0235 | 0.03225 | 10 | 26 | 63 | 0.3958 | 0.63739 | 0.27727 | 0.428 | | 0 |
| PEPLOMYCIN | PEP | 0.0429 | 0.03478 | 0.03 | 0.0222 | 0.0328 | 0.02543 | 18 | 36 | 85 | 0.1626 | 0.35379 | 0.20506 | 0.3634 | | 0 |
| DIHYDROXYACETONEPHOSPHORIC ACID | DHAP | 0.6886 | 0.3374 | 0.445 | 0.3238 | 0.5023 | 0.36005 | 20 | 37 | 90 | 0.0052 | 0.05 | 0.01043 | 0.0494 | | 1 |
| GLYCOLIC ACID | Glycolate | 1.0617 | 0.75961 | 0.677 | 0.6006 | 0.7289 | 0.69866 | 20 | 36 | 85 | 0.0336 | 0.13014 | 0.02913 | 0.0951 | | 1 |
| HISTAMINE | Histamine | 0.0236 | 0.04078 | 0.02 | 0.035 | 0.0194 | 0.02611 | 13 | 30 | 77 | 0.8398 | 0.91124 | 0.42786 | 0.6064 | | 0 |
| N-ACETYLLEUCINE | N-Acetylleucine | 0.0018 | 0.0034 | 0.002 | 0.0029 | 0.0015 | 0.00294 | 6 | 16 | 28 | 0.4863 | 0.70123 | 0.91331 | 0.936 | | 0 |
| CYTIDINE DISODIUM 5'-MONOPHOSPHATE | CMP | 0.0228 | 0.0348 | 0.031 | 0.0476 | 0.0282 | 0.04103 | 10 | 21 | 54 | 0.6165 | 0.8132 | 0.50663 | 0.6512 | | 0 |
| GUANINE | Guanine | 0.0706 | 0.07096 | 0.061 | 0.0505 | 0.0678 | 0.0594 | 14 | 32 | 78 | 0.9532 | 0.98494 | 0.79472 | 0.8644 | | 0 |
| 4-METHYL-2-OXOPENTANOIC ACID | 4-Methyl-2-oxopentanoate | 0.1425 | 0.07182 | 0.102 | 0.075 | 0.1129 | 0.07547 | 20 | 37 | 90 | 0.0258 | 0.12032 | 0.04857 | 0.1401 | | 1 |
| N-ACETYLASPARTIC ACID | N-Acetylaspartate | 0.0622 | 0.03537 | 0.046 | 0.0434 | 0.0558 | 0.05725 | 20 | 37 | 90 | 0.027 | 0.12032 | 0.08465 | 0.2135 | | 1 |
| TYROSINE | Tyr | 1.5902 | 0.72068 | 1.093 | 0.6385 | 1.165 | 0.61484 | 20 | 37 | 90 | 0.004 | 0.05 | 0.00997 | 0.0494 | 7 | 1 |
| SUCCINIC ACID | Succinate | 1.61 | 1.54845 | 1.123 | 0.7861 | 1.6525 | 2.61266 | 20 | 37 | 90 | 0.2212 | 0.43533 | 0.45454 | 0.619 | | 0 |
| GLYCEROPHOSPHORIC ACID | Glycerophosphate | 0.7889 | 0.54774 | 0.522 | 0.4592 | 0.6148 | 0.53233 | 20 | 37 | 90 | 0.0179 | 0.09637 | 0.05316 | 0.1495 | | 1 |
| ALANYL-ALANINE | Ala-Ala | 0.0498 | 0.0542 | 0.035 | 0.0332 | 0.0388 | 0.03904 | 13 | 27 | 62 | 0.5935 | 0.79129 | 0.62247 | 0.7496 | | 0 |
| 1,3-DIAMINOPROPANE | 1,3-Diaminopropane | 0.1091 | 0.12897 | 0.096 | 0.107 | 0.097 | 0.10061 | 12 | 29 | 71 | 0.8458 | 0.91124 | 0.77566 | 0.8512 | 8 | 0 |
| 3-PHENYLPROPIONIC ACID | 3-Phenylpropionate | 0.9437 | 1.09581 | 1.178 | 1.659 | 1.395 | 1.91697 | 20 | 31 | 80 | 0.7316 | 0.88078 | 0.71847 | 0.8242 | | 0 |
| CIS-ACONITATE | cis-Aconitate | 0.0175 | 0.01899 | 0.013 | 0.0169 | 0.0212 | 0.06004 | 12 | 30 | 68 | 0.8461 | 0.91124 | 0.90341 | 0.9335 | | 0 |

Even when the whole concentration of saliva of elderly patient is increased, using glutamine that is a substance that correlates with the most metabolites and can be detected in all of the samples as a concentration-correcting marker

| NAME OF SUBSTANCE | | RELATIVE CONCENTRATION (NO UNIT) | | | | | | DETECTION RATIO (%) | | | MANN-WHITNEY TEST | | | | |
|---|---|---|---|---|---|---|---|---|---|---|---|---|---|---|---|
| | | HEALTHY SUBJECT | | BREAST CANCER ONLY BEFORE TREATMENT | | BREAST CANCER INCLUDING DURING TREATMENT | | HEALTHY SUBJECT | BREAST CANCER ONLY BEFORE TREATMENT | BREAST CANCER INCLUDING DURING TREATMENT | BREAST CANCER ONLY BEFORE TREATMENT vs HEALTHY SUBJECT | | BREAST CANCER INCLUDING DURING TREATMENT vs HEALTHY SUBJECT | | PUBLICLY KNOWN SIGNIFICANT |
| | | AVERAGE | STANDARD DEVIATION | AVERAGE | STANDARD DEVIATION | AVERAGE | STANDARD DEVIATION | | | | P VALUE | Q VALUE | P VALUE | Q VALUE | |
| 3-HYDROXYBUTYRIC ACID | 3-Hydroxybutyrate | 0.647 | 0.52696 | 0.369 | 0.2321 | 0.4759 | 0.39905 | 20 | 37 | 90 | 0.037 | 0.1319 | 0.14 | 0.294 | 1 |
| BENZOIC ACID | Benzoate | 1.332 | 0.9986 | 0.799 | 0.8965 | 1.04938 | 0.99907 | 18 | 27 | 67 | 0.027 | 0.1203 | 0.024 | 0.085 | 1 |
| GUANOSINE | Guanosine | 0.024 | 0.03472 | 0.015 | 0.0129 | 0.0182 | 0.0182 | 10 | 19 | 42 | 0.624 | 0.8149 | 0.345 | 0.505 | 0 |
| 6-PHOSPHOGLUCONIC ACID | 6-Phosphogluconate | 0.036 | 0.03115 | 0.031 | 0.0485 | 0.02888 | 0.03903 | 16 | 33 | 77 | 0.258 | 0.4929 | 0.227 | 0.386 | 0 |
| URIDYLIC ACID | UMP | 0.012 | 0.01873 | 0.01 | 0.0136 | 0.0122 | 0.01936 | 8 | 18 | 42 | 0.848 | 0.9112 | 0.744 | 0.824 | 0 |
| ADENOSINE | Adenosine | 0.019 | 0.02147 | 0.013 | 0.0212 | 0.0115 | 0.01764 | 11 | 22 | 49 | 0.455 | 0.6824 | 0.272 | 0.427 | 0 |
| MUCIC ACID | Mucate | 0.032 | 0.04565 | 0.018 | 0.0317 | 0.0178 | 0.02751 | 11 | 18 | 49 | 0.293 | 0.535 | 0.358 | 0.516 | 0 |
| ETHANOLAMINEPHOSPHORIC ACID | Ethanolamine phosphate | 4.256 | 4.75927 | 3.189 | 3.9208 | 3.5534 | 4.83037 | 18 | 34 | 84 | 0.219 | 0.4353 | 0.28 | 0.428 | 0 |
| ADIPATE | Adipate | 0.052 | 0.04601 | 0.03 | 0.0276 | 0.0385 | 0.04267 | 19 | 35 | 87 | 0.03 | 0.1214 | 0.093 | 0.221 | 0 |
| 2-ISOPROPYLMALATE | 2-Isopropylmalate | 0.025 | 0.0254 | 0.014 | 0.013 | 0.0175 | 0.02347 | 20 | 36 | 87 | 0.029 | 0.1214 | 0.02 | 0.074 | 0 |
| PHOSPHORYLCHLORINE | Phosphorylcholine | 0.547 | 0.35158 | 0.261 | 0.2648 | 0.3764 | 0.3664 | 20 | 31 | 81 | 6E-04 | 0.0137 | 0.013 | 0.054 | 0 |
| NICOTINATE | Nicotinate | 0.157 | 0.12343 | 0.098 | 0.1071 | 0.1272 | 0.13348 | 16 | 23 | 63 | 0.068 | 0.1926 | 0.211 | 0.369 | 0 |
| 1-METHYL-2-PYRROLIDINONE | 1-Methyl-2-pyrrolidinone | 2.577 | 2.74352 | 1.798 | 2.1837 | 1.739 | 1.95496 | 20 | 36 | 87 | 0.107 | 0.2553 | 0.092 | 0.221 | 0 |
| MALIC ACID | Malate | 0.181 | 0.1191 | 0.132 | 0.131 | 0.1531 | 0.13135 | 20 | 37 | 90 | 0.057 | 0.1702 | 0.189 | 0.345 | 0 |
| PANTOTHENIC ACID | Pantothenate | 0.007 | 0.00177 | 0.004 | 0.006 | 0.007 | 0.01098 | 7 | 14 | 42 | 0.787 | 0.9032 | 0.549 | 0.694 | 0 |
| N-ACETYLNEURAMINATE | N-Acetylneuraminate | 4.326 | 3.32649 | 2.13 | 1.8598 | 2.3366 | 1.94534 | 20 | 37 | 90 | 0.005 | 0.05 | 0.006 | 0.043 | 0 |
| HISTAMINE | His | 0.914 | 0.35757 | 0.542 | 0.309 | 0.609 | 0.33114 | 20 | 37 | 90 | 0.392 | 0.6374 | 0.491 | 0.641 | 0 |
| FUMARIC ACID | Fumarate | 0.016 | 0.03298 | 0.01 | 0.0192 | 0.0093 | 0.01812 | 15 | 30 | 72 | 0.975 | 0.9907 | 0.869 | 0.921 | 0 |
| 2-DEOXYGLUCOSE-6-PHOSPHORIC ACID | 2-Deoxyglucose 6-phosphate | 0.033 | 0.0321 | 0.019 | 0.0192 | 0.016 | 0.03294 | 5 | 11 | 24 | 2E-04 | 0.0099 | 9E-04 | 0.022 | 0 |
| CITRIC ACID | Citrate | 0.636 | 0.62144 | 0.449 | 0.672 | 0.5399 | 0.79022 | 20 | 35 | 86 | 0.075 | 0.2043 | 0.138 | 0.294 | 0 |
| 4-ACETYLBUTYRIC ACID | 4-Acetylbutyrate | 0.013 | 0.02228 | 0.007 | 0.0149 | 0.0051 | 0.01133 | 6 | 11 | 25 | 0.687 | 0.8518 | 0.449 | 0.619 | 0 |
| O-ACETYLCARNITINE | o-Acetylcarnitine | 0.092 | 0.0607 | 0.057 | 0.0544 | 0.0588 | 0.04852 | 20 | 37 | 89 | 0.01 | 0.0719 | 0.009 | 0.049 | 0 |
| N-ACETYLGLUCOSAMINE-1-PHOSPHORIC ACID | N-Acetylglucosamine 1-phosphate | 0.021 | 0.0156 | 0.01 | 0.0111 | 0.0103 | 0.01125 | 17 | 26 | 63 | 0.006 | 0.0508 | 0.002 | 0.025 | 0 |
| SEDOHEPTULOSE-7-PHOSPHORIC ACID | S7P | 0.038 | 0.0291 | 0.024 | 0.0242 | 0.0263 | 0.02339 | 16 | 29 | 72 | 0.068 | 0.1926 | 0.112 | 0.252 | 0 |
| HEPTANOIC ACID | Heptanoate | 0.032 | 0.03981 | 0.015 | 0.0218 | 0.0165 | 0.02305 | 15 | 25 | 56 | 0.117 | 0.2723 | 0.086 | 0.213 | 0 |
| CREATININE | Creatinine | 0.364 | 0.23931 | 0.221 | 0.2029 | 0.2462 | 0.22261 | 20 | 37 | 90 | 0.005 | 0.05 | 0.01 | 0.049 | 0 |
| 2,5-DIHYDROXYBENZOATE | 2,5-Dihydroxybenzoate | 0.04 | 0.05452 | 0.021 | 0.0352 | 0.0334 | 0.07638 | 9 | 17 | 38 | 0.438 | 0.6704 | 0.483 | 0.641 | 0 |
| CYTIDINE | Cytidine | 0.037 | 0.04016 | 0.022 | 0.0278 | 0.0192 | 0.02594 | 14 | 22 | 49 | 0.178 | 0.373 | 0.054 | 0.149 | 0 |
| ARGININE | Arg | 1.147 | 0.61391 | 0.775 | 0.7669 | 0.8378 | 0.7202 | 20 | 37 | 90 | 0.013 | 0.083 | 0.027 | 0.092 | 0 |
| SYRINGIC ACID | Syringate | 0.13 | 0.08544 | 0.085 | 0.0942 | 0.0843 | 0.08747 | 20 | 34 | 82 | 0.039 | 0.1319 | 0.015 | 0.062 | 1 |

makes it possible to distinguish a subject with cancer from a healthy subject by eliminating the influence of concentration variations by this method. On the other hand, people equal to or older than 70 years of age have a trend of increasing the whole concentration of saliva. Therefore, when the absolute concentration is used, the construction of a model using only data of people less than 70 years of age leads to a highly accurate separation.

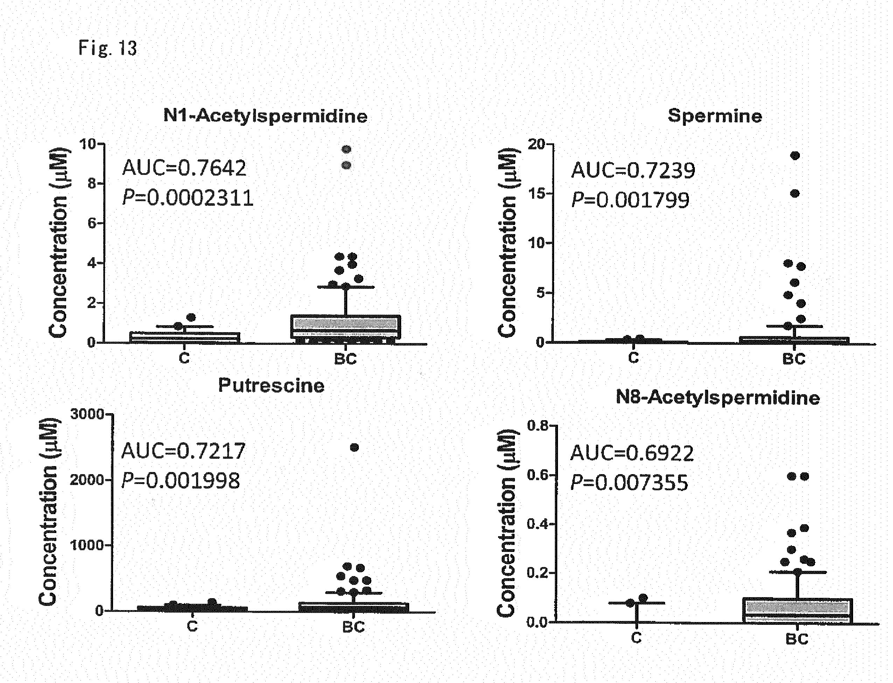

A substance belonging to polyamines among substances that give a significant difference between the healthy subjects (C, n = 20) and the patients with breast cancer (BC, all the cases including before treatment, n = 90) is shown in FIG. 13.

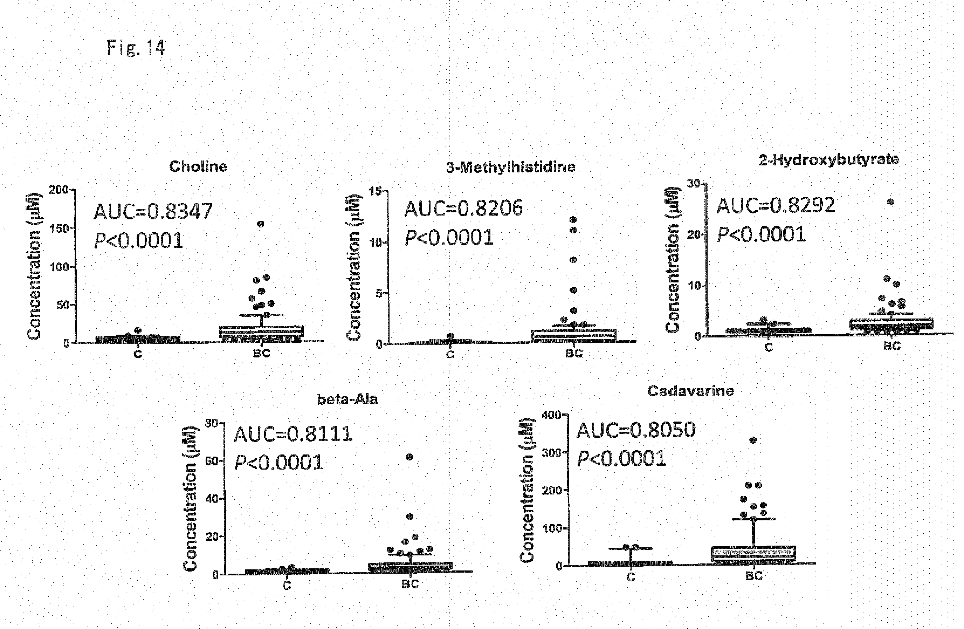

Examples (the top five substances with a smaller P value) of substances other than polyamines among the substances that give a significant difference between the healthy subjects (C, n = 20) and the patients with breast cancer (BC, all the cases including before treatment, n = 90) are shown in FIG. 14.

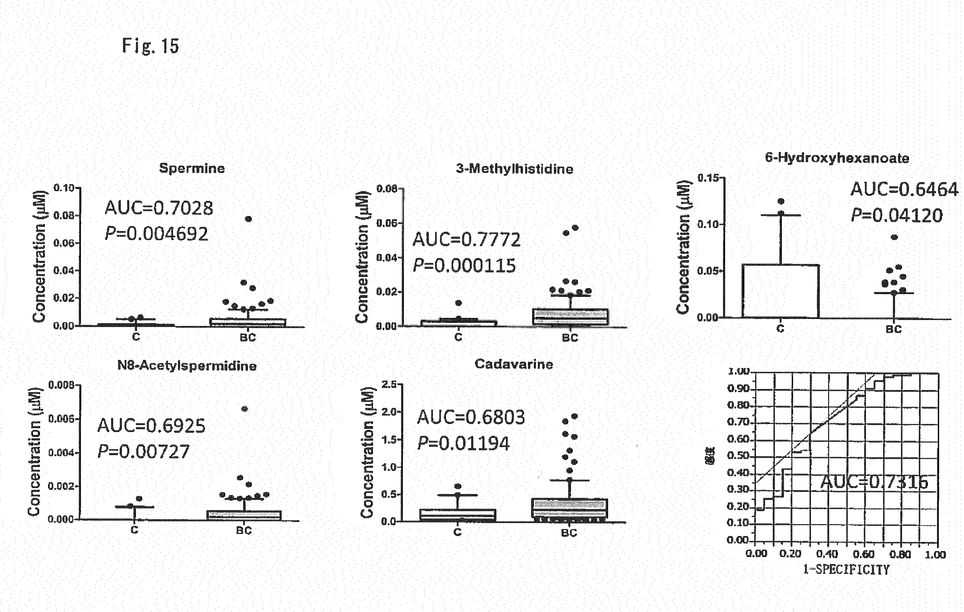

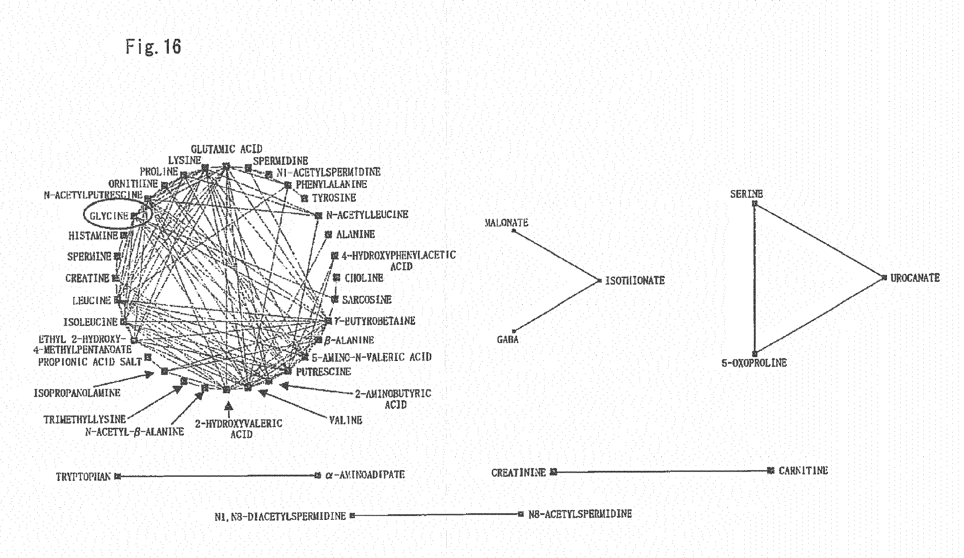

Substances that give a significant difference ($p < 0.05$) between the healthy subjects (C, 20 cases) and the patients with breast cancer (BC, 90 cases) regardless of the presence or absence of concentration correction are shown in FIG. 15. A network diagram (shown in FIG. 16) was formed using all the cases of all the samples (20 cases of C and 90 cases of BC). A concentration correction substance, or Gly (glycine, expressed in o in the drawing) was determined. When concentration correction with this concentration correction marker was not performed, 73 substances exhibited a significant difference. When concentration correction was performed (the concentration of metabolite of interest was divided by the concentration of Gly), 35 substances exhibited a significant difference. Among the substances, 11 substances exhibited a significant difference regardless of the presence or absence of concentration correction. The top five substances that had a smaller P value are shown. An ROC curve at which an MLR model was formed using two substances of spermine and 6-hydaroxyhexanoate is shown in the lower right.

Next, a biomarker for oral cancer will be described.

Substances in which the absolute concentrations of metabolites in saliva exhibit a difference between subjects with oral cancer and healthy subjects are shown in Tables 9-1 and 9-2. The healthy subjects were 20 cases, and patients with breast cancer were 20 cases. For the healthy subjects, saliva was collected 1.5 hours after eating, and for the patients with cancer, saliva was collected two times, before eating (in a fasting state from the previous night) and 1.5 hours after eating. By comparing either of them, a P value was calculated using the Mann-Whitney test, and a Q value was calculated using the false discovery rate (FDR). Substances of $Q < 0.05$ were listed.

Table 9-1

| COMPOUND | NAME OF SUBSTANCE | HEALTHY SUBJECT 1.5 HOURS AFTER DIET | | ORAL CANCER | | | | TEST (COMPARISON WITH HEALTHY SUBJECTS) | | | | PUBLICLY KNOWN |
|---|---|---|---|---|---|---|---|---|---|---|---|---|
| | | | | 1.5 HOURS AFTER DIET | | ON EMPTY STOMACH | | CANCER (1.5 HOURS AFTER DIET) | | CANCER (ON EMPTY STOMACH) | | |
| | | AVERAGE | S.D. | AVERAGE | S.D. | AVERAGE | S.D. | P-value | Q-value | P-value | Q-value | |
| Gly-Gly | GLYCYL-GLYCINE | 0.4069 | 0.9342 | 1.058 | 1.70904 | 2.6807 | 3.3424 | 0.01231 | 0.08136 | 7.3E-05 | 0.0028 | |
| Choline | CHOLINE | 9.8191 | 13.615 | 16.57 | 10.5053 | 23.982 | 18.487 | 0.00196 | 0.03719 | 9.5E-05 | 0.0029 | 9 |
| Citrulline | CITRULLINE | 15.509 | 17.115 | 32.87 | 33.9328 | 58.026 | 59.93 | 0.04298 | 0.16753 | 0.00013 | 0.0032 | |
| gamma-Butyrobetaine | γ-BUTYROBETAINE | 3.0834 | 3.8972 | 5.898 | 5.71459 | 8.116 | 5.7123 | 0.01809 | 0.10186 | 0.00015 | 0.0032 | |
| 3-Phenyllactate | 3-PHENYLLACTATE | 1.7552 | 3.8247 | 2.227 | 2.43789 | 4.436 | 5.4298 | 0.15394 | 0.2945 | 0.00024 | 0.004 | |
| Butanoate | BUTYRIC ACID | 63.135 | 53.789 | 368.9 | 572.732 | 276.63 | 290.83 | 0.00195 | 0.03719 | 0.00027 | 0.004 | |
| Hexanoate | HEXANOIC ACID | 13.695 | 13.216 | 38.52 | 65.1854 | 71.359 | 106.92 | 0.34078 | 0.45043 | 0.0003 | 0.004 | |
| Met | METHIONINE | 1.372 | 3.3692 | 2.48 | 4.46552 | 7.1068 | 10.922 | 0.12541 | 0.28034 | 0.00032 | 0.004 | |
| Hypoxanthine | HYPOXANTHINE | 5.4485 | 8.9829 | 12.46 | 16.7868 | 18.403 | 21.808 | 0.0239 | 0.11717 | 0.00034 | 0.004 | |
| Spermidine | SPERMIDINE | 1.813 | 1.7732 | 3.705 | 5.16895 | 4.6425 | 2.7304 | 0.31408 | 0.42626 | 0.00043 | 0.0042 | |
| Val | VALINE | 13.282 | 20.557 | 27.63 | 47.2029 | 47.09 | 63.858 | 0.1207 | 0.27586 | 0.00044 | 0.0042 | 7 |
| Glu | GLUTAMIC ACID | 43.09 | 90.355 | 54.12 | 65.7928 | 93.418 | 88.182 | 0.31408 | 0.42626 | 0.00044 | 0.0042 | 7 |
| Trp | TRYPTOPHAN | 1.6971 | 2.9918 | 2.367 | 2.83711 | 5.3687 | 6.4 | 0.10745 | 0.26082 | 0.00048 | 0.0042 | |
| Ala | ALANINE | 40.573 | 68.685 | 73.86 | 78.6906 | 137.25 | 145.91 | 0.00672 | 0.06109 | 0.0005 | 0.0042 | 7 |
| Asp | ASPARTIC ACID | 19 | 30.334 | 27.79 | 28.8413 | 45.057 | 41.162 | 0.1207 | 0.27586 | 0.00104 | 0.0083 | |
| Piperidine | PIPERIDINE | 0.1498 | 0.2574 | 1.321 | 2.36202 | 1.3152 | 2.5325 | 0.00501 | 0.06109 | 0.00121 | 0.0083 | 7 |
| Isopropanolamine | ISOPROPANOLAMINE | 0.6591 | 0.5352 | 1.688 | 1.72126 | 1.5766 | 0.9826 | 0.06715 | 0.20118 | 0.00121 | 0.0083 | |
| Ala-Ala | ALANYL-ALANINE | 0.8831 | 1.1716 | 1.732 | 2.46196 | 2.8654 | 3.1707 | 0.37911 | 0.48424 | 0.00123 | 0.0083 | |
| N,N-Dimethylglycine | N,N-DIMETHYLGLYCINE | 0.2266 | 0.5635 | 0.43 | 0.47283 | 0.5607 | 0.3983 | 0.00596 | 0.06109 | 0.00126 | 0.0083 | |
| N1-Acetylspermidine | N1-ACETYLSPERMIDINE | 0.7048 | 0.9459 | 1.337 | 1.27463 | 2.0556 | 2.4166 | 0.01327 | 0.08387 | 0.00143 | 0.0087 | |
| N1,N8-Diacetylspermidine | N1-,N8-DIACETYLSPERMIDINE | 0.5098 | 1.6679 | 0.336 | 0.33971 | 0.5473 | 0.5625 | 0.03538 | 0.14152 | 0.00145 | 0.0087 | |
| N8-Acetylspermidine | N8-ACETYLSPERMIDINE | 0.0524 | 0.0882 | 0.111 | 0.1197 | 0.1356 | 0.1038 | 0.06919 | 0.20192 | 0.00148 | 0.0087 | |
| 2AB | α-AMINOBUTYRIC ACID | 1.6132 | 1.5683 | 3.175 | 4.32823 | 6.9715 | 13.599 | 0.14171 | 0.2945 | 0.00166 | 0.0088 | |
| Trimethylamine N-oxide | TRIMETHYLAMINE-N-OXIDE | 0.1483 | 0.2186 | 0.596 | 0.6416 | 0.8705 | 1.2581 | 0.00337 | 0.05691 | 0.00175 | 0.0088 | |
| N-Acetylaspartate | N-ACETYLASPARTIC ACID | 0.9277 | 0.817 | 2.256 | 2.45157 | 2.2515 | 1.7815 | 0.01435 | 0.08387 | 0.00185 | 0.0088 | |
| Adenine | ADENINE | 0.6068 | 0.6117 | 0.845 | 0.73556 | 1.2708 | 0.8954 | 0.06377 | 0.20118 | 0.00185 | 0.0088 | |
| Thr | THREONINE | 7.3662 | 12.091 | 12.76 | 14.0435 | 19.827 | 18.913 | 0.02831 | 0.12656 | 0.00185 | 0.0088 | 7 |
| 2-Hydroxypentanoate | 2-HYDROXYVALERIC ACID | 10.065 | 22.066 | 12.13 | 13.4737 | 16.866 | 20.227 | 0.08023 | 0.21396 | 0.00193 | 0.0089 | |
| Putrescine(1,4-Butanediamine) | PUTRESCINE | 55.793 | 65.975 | 139.1 | 212.235 | 140.18 | 122.54 | 0.04909 | 0.18199 | 0.00207 | 0.009 | |
| Ile | ISOLEUCINE | 5.4605 | 9.7987 | 10.74 | 19.3536 | 17.599 | 23.355 | 0.0675 | 0.20118 | 0.00207 | 0.009 | 7 |
| 3PG | 3-PHOSPHOGLYCERIC ACID BARIUM SALT | 2.9479 | 4.4668 | 5.387 | 4.81451 | 5.5895 | 3.7831 | 0.00733 | 0.06109 | 0.00231 | 0.0092 | |
| Gln | GLUTAMINE | 21.627 | 22.327 | 38.27 | 36.3946 | 66.62 | 65.148 | 0.07178 | 0.20192 | 0.00231 | 0.0092 | 7 |
| beta-Ala | β-ALANINE | 2.2944 | 2.0961 | 3.48 | 3.29484 | 4.9516 | 3.9527 | 0.10216 | 0.25881 | 0.00231 | 0.0092 | 7 |
| 3-Phenylpropionate | 3-PHENYLPROPIONIC ACID | 8.6553 | 9.8714 | 29.14 | 48.6612 | 53.281 | 74.39 | 0.22064 | 0.36454 | 0.00256 | 0.0094 | |
| Ser | SERINE | 16.853 | 20.939 | 34.11 | 35.0987 | 45.686 | 40.867 | 0.0143 | 0.08387 | 0.00257 | 0.0094 | |

Table 9-2

| COMPOUND | NAME OF SUBSTANCE | HEALTHY SUBJECT 1.5 HOURS AFTER DIET | | ORAL CANCER 1.5 HOURS AFTER DIET | | ON EMPTY STOMACH | | TEST (COMPARISON WITH HEALTHY SUBJECTS) CANCER (1.5 HOURS AFTER DIET) | | CANCER (ON EMPTY STOMACH) | | PUBLICLY KNOWN |
|---|---|---|---|---|---|---|---|---|---|---|---|---|
| | | AVERAGE | S.D. | AVERAGE | S.D. | AVERAGE | S.D. | P-value | Q-value | P-value | Q-value | |
| 1-Methylnicotinamide | 1-METHYLNICOTINAMIDE | 0 | 0 | 0.0863 | 0.1482 | 0.0447 | 0.061 | 0.00093 | 0.0283 | 0.002658 | 0.0093969 | |
| 3-Hydroxy-3-methylglutarate | 3-HYDROXY-3-METHYLGLUTARIC ACID | 0 | 0 | 0.1326 | 0.2051 | 0.1545 | 0.263 | 0.00093 | 0.0283 | 0.002658 | 0.0093969 | |
| Guanine | GUANINE | 0.8532 | 0.8635 | 2.0106 | 2.3806 | 2.1938 | 2.504 | 0.08023 | 0.214 | 0.002701 | 0.0093969 | |
| 3-(4-Hydroxyphenyl)propionate | 3-(4-HYDROXYPHENYL)PROPIONIC ACID | 5.7327 | 5.3987 | 16.824 | 25.171 | 26.897 | 26.52 | 0.22686 | 0.3665 | 0.002948 | 0.0099587 | |
| 4-Methylbenzoate | 4-METHYLBENZOATE | 13.693 | 16.348 | 29.523 | 44.709 | 40.875 | 35.76 | 0.15324 | 0.2945 | 0.003242 | 0.0107135 | |
| Ru5P | RIBULOSE-5-PHOSPHORIC ACID | 3.6439 | 3.1654 | 6.2273 | 3.6308 | 6.4447 | 3.509 | 0.00427 | 0.0611 | 0.003529 | 0.0111736 | |
| Cadaverine | CADAVERINE | 17.118 | 26.089 | 64.655 | 111.69 | 70.095 | 93.24 | 0.03264 | 0.1341 | 0.003529 | 0.0111736 | 7 |
| alpha-Aminoadipate | α-AMINOADIPIC ACID | 2.2491 | 4.0828 | 2.9071 | 1.7281 | 3.6911 | 4.177 | 0.00511 | 0.0611 | 0.00362 | 0.011228 | |
| N-epsilon-Acetyllysine | N6-ACETYLLYSINE | 0.2091 | 0.532 | 0.3292 | 0.4221 | 0.6021 | 0.626 | 0.07152 | 0.2019 | 0.004421 | 0.0131769 | |
| Glucosamine | GLUCOSAMINE | 0.0982 | 0.2061 | 0.4062 | 0.7291 | 0.7409 | 1.101 | 0.15617 | 0.2945 | 0.004612 | 0.0133543 | |
| Pipecolate | PICOLINIC ACID | 0.5181 | 0.5391 | 0.7482 | 0.7248 | 1.5685 | 1.624 | 0.27202 | 0.4195 | 0.004656 | 0.0133543 | 79 |
| Cystine | CYSTINE | 0.1372 | 0.4283 | 0.2625 | 0.288 | 0.4851 | 0.588 | 0.01123 | 0.0813 | 0.005031 | 0.0141617 | |
| Leu | LEUCINE | 15.883 | 26.949 | 27.312 | 49.448 | 47.753 | 73.1 | 0.14171 | 0.2945 | 0.005288 | 0.0146144 | 7 |
| Carnosine | CARNOSINE | 0.2446 | 0.406 | 0.092 | 0.1094 | 0.0635 | 0.201 | 0.30422 | 0.4242 | 0.005549 | 0.0150626 | |
| Urocanate | UROCANIC ACID | 3.3151 | 4.3875 | 5.4696 | 8.6626 | 6.7838 | 7.392 | 0.14171 | 0.2945 | 0.005833 | 0.0152872 | |
| Phe | PHENYLALANINE | 22.976 | 38.395 | 24.975 | 26.739 | 43.982 | 42.62 | 0.34078 | 0.4504 | 0.005833 | 0.0152872 | |
| 2-Deoxyribose 1-phosphate | 2-DEOXYRIBOSE-1-PHOSPHORIC ACID | 0 | 0 | 0.4503 | 1.456 | 1.3412 | 3.671 | 0.0198 | 0.1075 | 0.006141 | 0.015558 | |
| CMP | CYTIDINE DISODIUM 5'-MONOPHOSPHATE | 0 | 0 | 0.0262 | 0.0806 | 0.6152 | 1.603 | 0.16259 | 0.3014 | 0.006141 | 0.015558 | |
| p-Hydroxyphenylacetate | p-HYDROXYPHENYLACETIC ACID | 9.3775 | 15.199 | 23.122 | 26.812 | 32.256 | 31.62 | 0.02914 | 0.1266 | 0.006704 | 0.0167057 | |
| 3-Hydroxybutyrate | POLYHYDROXYBUTYRIC ACID | 5.649 | 7.9585 | 9.1822 | 7.9574 | 9.7319 | 5.422 | 0.06574 | 0.2012 | 0.008441 | 0.0205966 | |
| N-Acetylputrescine | N-ACETYLPUTRESCINE | 3.7027 | 4.2148 | 6.7508 | 9.5333 | 8.5994 | 8.382 | 0.14928 | 0.2945 | 0.008537 | 0.0205966 | |
| 7-Methylguanine | 7-METHYLGUANINE | 0.0973 | 0.169 | 0.1594 | 0.1784 | 0.2467 | 0.17 | 0.15081 | 0.2945 | 0.008981 | 0.021304 | |
| Inosine | INOSINE | 1.1818 | 5.2853 | 1.1392 | 2.1327 | 0.932 | 1.333 | 0.00764 | 0.0611 | 0.00911 | 0.021304 | |
| Lys | LYSINE | 54.872 | 77.058 | 59.839 | 61.894 | 107.36 | 92.02 | 0.35465 | 0.4647 | 0.010257 | 0.0232693 | |
| DHAP | DIHYDROXYACETONEPHOSPHORIC ACID | 9.8189 | 12.418 | 14.832 | 12.189 | 15.183 | 10.62 | 0.02633 | 0.1213 | 0.011224 | 0.0250892 | |
| 3-Methylhistidine | 3-METHYLHISTIDINE | 0.269 | 0.3248 | 0.3945 | 0.198 | 0.6527 | 0.531 | 0.10982 | 0.2608 | 0.011398 | 0.0251085 | |
| Carbamoylaspartate | CARBAMOYLASPARTIC ACID | 0.1314 | 0.3286 | 0.3321 | 0.6735 | 0.5884 | 0.684 | 0.27515 | 0.4195 | 0.012219 | 0.0259014 | |
| Creatinine | CREATINE | 4.5834 | 2.8466 | 5.6163 | 2.1009 | 6.9995 | 4.008 | 0.05589 | 0.1976 | 0.012269 | 0.0259014 | |
| 1-Methyl-2-pyrrolidinone | N-METHYL-2-PYRROLIDONE | 0 | 0 | 3.393 | 4.145 | 1.6277 | 2.739 | 0.00093 | 0.0283 | 0.013779 | 0.0286895 | |
| Pyruvate | PYRUVIC ACID | 71.74 | 129.01 | 95.867 | 72.494 | 100.95 | 72.28 | 0.00733 | 0.0611 | 0.014053 | 0.0288663 | |
| Carnitine | CARNITINE | 1.3784 | 1.4646 | 1.6847 | 1.0058 | 2.2939 | 1.956 | 0.0524 | 0.1896 | 0.014613 | 0.0292252 | 79 |
| Propionate | PROPIONIC ACID | 212.01 | 162.5 | 503.43 | 443.17 | 444.31 | 328.5 | 0.00733 | 0.0611 | 0.01733 | 0.0333437 | |
| 5-Aminovalerate | 5-AMINOVALERIC ACID | 353.84 | 383.45 | 680.09 | 697.61 | 681.35 | 530.6 | 0.0675 | 0.2012 | 0.01733 | 0.0333437 | |
| N-Acetylornithine | N-ACETYLORNITHINE | 0.15 | 0.3066 | 0.3977 | 0.6845 | 0.5015 | 0.628 | 0.15694 | 0.2945 | 0.020103 | 0.0377233 | |
| Tyr | TYROSINE | 29.353 | 30.264 | 39.195 | 38.122 | 49.956 | 32.98 | 0.30125 | 0.4242 | 0.020467 | 0.0379391 | 7 |
| 5-Oxoproline | 5-OXOPROLINE | 11.522 | 26.849 | 11.424 | 13.484 | 14.236 | 20.71 | 0.06343 | 0.2012 | 0.022209 | 0.0406711 | |
| Creatine | CREATININE | 30.546 | 53.384 | 26.173 | 13.642 | 33.368 | 35.71 | 0.04595 | 0.1746 | 0.024074 | 0.0434287 | |
| Homoserine | HOMOSERINE | 0.3548 | 0.5828 | 0.657 | 0.7476 | 0.6454 | 0.537 | 0.07306 | 0.2019 | 0.024286 | 0.0434287 | |
| Fumarate | FUMARIC ACID | 0.4679 | 1.4637 | 1.5633 | 2.3957 | 0.703 | 0.883 | 0.01212 | 0.0814 | 0.025797 | 0.0455463 | |
| Gly | GLYCINE | 130.77 | 167.9 | 157.76 | 117.97 | 222.74 | 193.5 | 0.14928 | 0.2945 | 0.026069 | 0.0455463 | |

EP 3 578 984 A2

A substance in which "7" or "9" is described in the column labeled "Publicly Known" is a known substance disclosed in Non-Patent Literature 7 or 9.

Herein, the patients with oral cancer included stages I to IVa, and include oral squamous cell carcinoma (17 cases), malignant melanoma (2 cases), and adenoid cystic carcinoma (1 case). Spermine, spermidine, or acetylated spermine or spermidine consistently have a high concentration in comparison of an oral cancer tissue sample obtained during surgery and a healthy part in a vicinity of the oral cancer tissue.

For example, choline (second substance from the top in Table) among the substances is a known substance in Non-Patent Literatures 7 and 9, and an increase in the concentration of the substance in saliva has been confirmed. However, oral cancer can be identified with high accuracy by a mathematical model combined with a plurality of novel markers by the same procedure as those in pancreatic cancer and breast cancer. The substance is increased in oral cancer, but is not increased in breast cancer. Therefore, when the substance is included as a variable of the mathematical model, the specific type of cancer can be expressed.

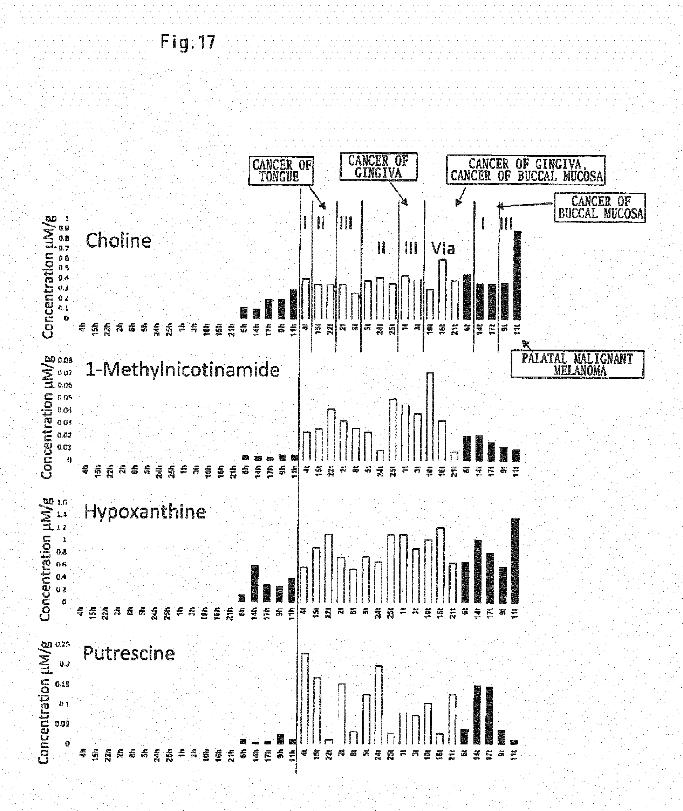

The concentrations of metabolites in the cancer tissue sample obtained during surgery of oral cancer and the healthy tissue sample near the cancer tissue sample (herein, the concentration corrected with the weight of the tissue in $\mu$M/g is used) are shown in FIG. 17. In the drawing, the healthy tissue is at a left part and the cancer tissue is at a right part. An extent of progression (grade) of cancer is represented by I, II, III, and Via. The drawing shows some substances that have a significant difference between the healthy part and the cancer part.

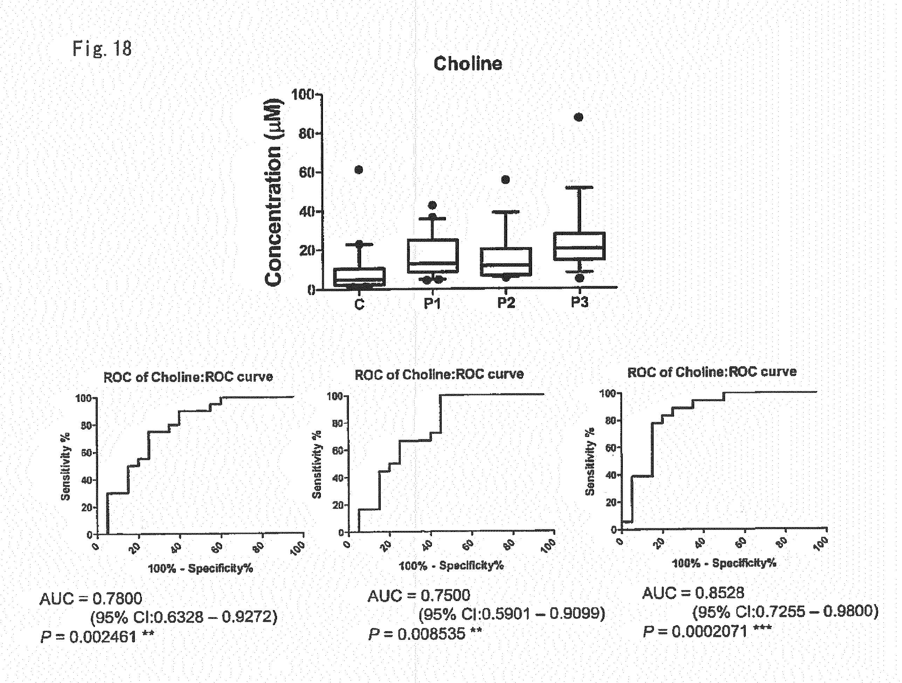

A difference in the concentration of saliva between the patients with oral cancer and the healthy subjects (C) when a method of collecting saliva in the patients with oral cancer was changed is shown in FIG. 18. For the healthy subjects, choline (Choline) that had the smallest P value in comparison of saliva from the patients with oral cancer is expressed as an example. For the healthy subjects (C), saliva was collected 1.5 hours after eating. For oral cancer, saliva was collected from the same patients, and saliva was collected 1.5 hours after eating as P1, collected 3.5 hours after eating as P2, and collected during fasting (before breakfast) as P3.

Table 10 shows results in which the absolute concentrations of polyamines and hypoxanthine were measured using saliva collected from 17 healthy subjects, 21 patients with pancreatic cancer, 16 patents with breast cancer, and 20 patients with oral cancer during fasting (hungry from 9:00 of previous night, no eating on the collection day) by liquid chromatography-mass spectrometer (LC-MS). A P value for evaluation of difference in average was calculated using the Student's t-test as a parametric test because the number of cases was small.

Results of determination of polyamines and hypoxanthine (Hypoxanthine) as a metabolite other than the polyamines are shown in Table 10. Among the polyamines, when N1,N12-diacetylspermine (N1,N12-diacetylspermine) was measured using CE-TOFMS, the peak thereof overlapped the peak of another substance. When LC-qTOFMS was used, the peak of N1,N12-diacetylspermine and the peak of the other substance could be separately measured. Herein, only the samples that were collected during fasting were used. The quantitative values determined for 17 cases of healthy subjects, 21 cases of pancreatic cancer, 18 cases of oral cancer, and 16 cases of breast cancer are described (the unit of quantitative value is $\mu$M).

Table 10

| COMPOUND | JAPANESE NAME | HEALTHY SUBJECT | | PANCREATIC CANCER | | | ORAL CANCER | | | BREAST CANCER | | |
|---|---|---|---|---|---|---|---|---|---|---|---|---|
| | | AVERAGE | SD | AVERAGE | SD | P-value | AVERAGE | SD | P-value | AVERAGE | SD | P-value |
| Hypoxanthine | HYPOXANTHINE | 1.088 | 1.421 | 3.245 | 3.166 | 0.00914 | 6. 369 | 8.410 | 0.01718 | 1.496 | 1.643 | 0.452478 |
| Spermidine | SPERMIDINE | 1.571 | 1.553 | 3.943 | 2.957 | 0.003346 | 4.756 | 3.689 | 0.00269 | 5.119 | 6.958 | 0.063076 |
| N8-Acetylspermidine | N8-ACETYLSPERMIDINE | 0.017 | 0.030 | 0.047 | 0.056 | 0.046547 | 0.088 | 0.114 | 0.01942 | 0.065 | 0.146 | 0.211951 |
| N1-Acetylspermidine | N1-ACETYLSPERMIDINE | 0.039 | 0.052 | 0.132 | 0.124 | 0.004252 | 0.482 | 0.689 | 0.01453 | 0.224 | 0.372 | 0.06753 |
| Spermine | SPERMINE | 0.147 | 0.175 | 1.328 | 1.937 | 0.011404 | 2.526 | 3.351 | 0.00788 | 0.536 | 0.539 | 0.013041 |
| N1,N8-Diacetylspermidine | N1-,N8-DIACETYLSPERMIDINE | 0.090 | 0.118 | 0.184 | 0.153 | 0.038241 | 0.223 | 0.336 | 0.12931 | 0.219 | 0.292 | 0.11577 |
| N1-Acetyl spermine | N1-ACETYLSPERMINE | 0.024 | 0.033 | 0.114 | 0.101 | 0.000769 | 0.242 | 0.379 | 0.02649 | 0.099 | 0.133 | 0.042624 |
| N1,N12-Diacetylspermine | N1,N12-DIACETYLSPERMINE | 0.068 | 0.103 | 0.189 | 0.169 | 0.010603 | 0.404 | 0.521 | 0.01502 | 0.173 | 0.243 | 0.127459 |

Saliva for LC-MS is treated as follows.

1) In 270 μL of MeOH and NH₄OH adjusted to 2 μM MES, saliva stored at -80°C is dissolved, and 30 μL thereof is added and stirred.

2) The mixture is centrifuged at 4°C and 15,000 rpm for 10 minutes, and the entire upper layer is transferred to another tube.

3) The whole amount of the liquid is subjected to centrifugal concentration, and added to the liquid are 18 μL of 90% MeOH and 12 μL of BorateBuffer, resulting in redissolution.

4) 5 μL of the liquid is used for LC-MS analysis, and 20 μL of the liquid is used for ELISA analysis.

5) In the LC-MS analysis, 10 μL of MilliQ water containing 4 μM Methionine-sulfone is added to 5 μL of the afore-mentioned solution to obtain a dilution as a sample.

Measurement conditions of LC-MS are as follows.

LC system: Agilent Technologies 1290 infinity
Mobile phase: Solvent A; Water containing 1% Formic acid: Solvent B; Acetonitrile containing 0.1% formic acid
Flow rate: 0.5 mL/min
Gradient [min. (%B)]: 0(98)-1(98)-3(55)-5(5)
Stop time: 7 min
Post time: 3 min
Column: CAPCELL CORE PC (Shiseido: 2.1 mm × 50 mm, 2.7 mm)
Column temp.: 50°C
Injection volume: 1 ·L
MS: Agilent Technologies G6230A
Gas temp: 350°C
Gas flow: 13 L/min
Neblizer Gas: 55 psig
Fragmentor: 150
Skimmer: 90
OCT1 RF Vpp: 200
VCap: 3500
Reference: 121.050873, 922.009798
Mode: Positive

According to the present invention, when the concentration of saliva is corrected (normalized), using data analysis of a correlation network reduces the influence of the concentration. Even in saliva in which concentrations vary greatly, a subject with pancreatic cancer can be distinguished from a healthy subject. The present method makes prediction of chronic pancreatitis, IPMN, breast cancer, and oral cancer possible.

A range in which a test can be performed using the marker of the present invention is determined by the value of concentration-correcting marker that reflects the saliva concentration, and saliva whose overall concentration is outside shoulde be treated as outliers. In saliva within the range, a patient with each cancer can be distinguished from a healthy subject by a mathematical model that combines the markers of absolute concentrations or corrected relative concentrations.

Industrial Applicability

Even by using saliva in which the concentration largely varies, pancreatic cancer, breast cancer, and oral cancer can be early detected in a healthy subject.

The present invention is further directed to the following embodiments:

1. A salivary biomarker for cancer, comprising any of low-molecular-weight compounds being metabolites in a saliva sample or a combination thereof.

2. The salivary biomarker for cancer according to embodiment 1, wherein the cancer is any of pancreatic cancer, intraductal papillary mucinous neoplasm (IPMN), breast cancer, and oral cancer.

3. The salivary biomarker for cancer according to embodiment 1 or 2, wherein the salivary biomarker is used for detection of pancreatic disease, and is any of N-acetylputrescine (N-Acetylputrescine), adenosine (Adenosine), 3-phospho-D-glyceric acid (3PG), urea (Urea), o-acetylcarnitine (o-Acetylcarnitine), citric acid (Citrate), glycyl-glycine (Gly-Gly), 5-aminovaleric acid (5-Aminovalerate), methyl 2-oxopentanoate (2-Oxoisopentanoate), malic acid (Malate), benzoate ester (Benzoate), fumaric acid (Fumarate), N-acetylaspartic acid (N-Acetylaspartate), inosine (Inosine), 3-methylhistidine (3-Methylhistidine), N1-acetylspermine (N1-Acetylspermine), creatine (Creatine), α-aminoadipic acid (alpha-Aminoadipate), phosphorylcholine (Phosphorylcholine), 2-hydroxypentanoate (2-Hydroxypentanoate), xanthine (Xanthine), succinic acid (Succinate), 6-phosphogluconic acid (6-Phosphogluconate), butanoic acid (Butanoate), homovanillic acid (Homovanillate), O-phosphoserine (O-Phosphoserine), trimethylamine-N-oxide (Trimethylamine N-oxide), piperidine (Piperidine), cystine (Cystine), 2-isopropylmalic acid (2-Isopropylmalate), N8-acetylspermidine (N8-Acetylspermidine), N1-acetylspermidine (N1-Acetylspermidine), N-acetylneuraminic acid (N-Acetylneuraminate), glucosamine (Glucosamine), spermine (Spermine), agmatine (Agmatine), N-acetylhistamine (N-Acetylhistamine), methionine (Met), p-4-hydroxyphenylacetic acid (p-4-Hydroxyphenylacetate), N,N-dimethylglycine (N,N-Dimethylglycine), hypotaurine (Hypotaurine), glutamyl-glutamic acid (Glu-Glu), and N1,N12-diacetylspermine (N1,N12-Diacetylspermine), or a combination thereof.

4. The salivary biomarker for cancer according to embodiment 1 or 2, wherein the salivary biomarker is used for detection of pancreatic disease, and is any of N8-acetylspermidine (N8-Acetylspermidine), creatinine (Creatinine), spermine (Spermine), aspartic acid (Asp), N1-acetylspermidine (N1-Acetylspermidine), N1-acetylspermine (N1-Acetylspermine), cytidine (Cytidine), α-aminoadipic acid (alpha-Aminoadipate), cytosine (Cytosine), betaine (Betaine), urea (Urea), homovanillic acid (Homovanillate), N-acetylneuraminic acid (N-Acetylneuraminate), cystine (Cys), urocanic acid (Urocanate), fumaric acid (Fumarate), 1,3-diaminopropane (1,3-Diaminopropane), hypotaurine (Hypotaurine), nicotinic acid (Nicotinate), agmatine (Agmatine), valine (Val), 2-hydroxy-4-methylpentanoic acid (2-Hydroxy-4-methylpentanoate), alanyl-alanine (Ala-Ala), citric acid (Citrate), glucosamine (Glucosamine), carnosine (Carnosine), glycyl-glycine (Gly-Gly), 2-aminobutyric acid (2AB), arginine (Arg), an N-acylglutamic acid (N-Acetylglutamate), glycerophosphoric acid (Glycerophosphate), phosphoenolpyruvic acid (PEP), isoleucine (Ile), adenosine (Adenosine), guanine (Guanine), dihydroxyacetonephosphoric acid (DHAP), and cadaverine (Cadaverine), or a combination thereof.

5. The salivary biomarker for cancer according to embodiment 4, wherein the salivary biomarker is a combination of creatinine, N1-acetylspermidine, α-aminoadipic acid, N-acetylneuraminic acid, and 1,3-diaminopropane.

6. The salivary biomarker for cancer according to embodiment 2, wherein the salivary biomarker is used for detection of breast cancer, and is any of choline (Choline), 2-hydroxybutyric acid (2-Hydroxybutyrate), β-alanine (beta-Ala), 3-methylhisdine (3-Methylhistidine), α-aminobutyric acid (2AB), N-acetyl-β-alanine (N-Acetyl-beta-alanine), isethionic acid (Isethionate), N-acetylphenylalanine (N-Acetylphenylalanine), trimethyllysine (N6,N6,N6-Trimethyllysine), α-aminoadipic acid (alpha-Aminoadipate), creatine (Creatine), γ-butyrobetaine (gamma-Butyrobetaine), sarcosine (Sarcosine), pyruvic acid (Pyruvate), urocanic acid (Urocanate), piperidine (Piperidine), serine (Ser), homovanillic acid (Homovanillate), 5-oxoproline (5-Oxoproline), GABA (GABA), 5-aminovaleric acid (5-Aminovalerate), trimethylamine-N-oxide (Trimethylamine N-oxide), 2-hydroxyvaleric acid (2-Hydroxyppentanoate), carnitine (Carnitine), isopropanolamine (Isopropanolamine), hypotaurine (Hypotaurine), lactic acid (Lactate), 2-hydroxy-4-methylpentanoic acid (2-Hydroxy-4-methylpentanoate), hydroxyproline (Hydroxyproline), butyric acid (Butanoate), adenine (Adenine), N6-acetyllysine (N-epsilon-Acetyllysine), 6-hydroxyhexanoic acid (6-Hydroxyhexanoate), propionic acid (Propionate), betaine (Betaine), N-acetylputrescine (N-Acetylputrescine), hypoxanthine (hypoxanthine), crotonic acid (Crotonate), tryptophan (Trp), citrulline (Citrulline), glutamine (Gln), proline (Pro), 2-oxoisopentanoic acid (2-Oxoisopentanoate), 4-methylbenzoate (4-Methylbenzoate), 3-(4-hydroxyphenyl)propionic acid (3-(4-Hydroxyphenyl)propionate), cysteic acid (Cysteate), azelaic acid (Azelate), ribulose-5-phosphoric acid (Ru5P), pipecolinic acid (Pipecolate), phenylalanine (Phe), O-phosphoserine (O-Phosphoserine), malonic acid (Malonate), hexanoic acid (Hexanoate), and p-hydroxyphenylacetic acid (p-Hydroxyphenylacetate), or a combination thereof.

7. The salivary biomarker for cancer according to embodiment 2, wherein the salivary biomarker is used for detection of breast cancer, and is a combination of β-alanine, N-acetylphenylalanine, and citrulline.

8. The salivary biomarker for cancer according to embodiment 2, wherein the salivary biomarker is used for detection of breast cancer, and is any of choline (Choline), β-alanine (beta-Ala), 3-methylhisdine (3-Methylhistidine), α-aminobutyric acid (2AB), N-acetyl-β-alanine (N-Acetyl-beta-alanine), isethionic acid (Isethionate), N-acetylphenylalanine (N-Acetylphenylalanine), trimethyllysine (N6,N6,N6-Trimethyllysine), urocanic acid (Urocanate), piperidine (Piperidine), 5-aminovaleric acid (5-Aminovalerate), trimethylamine-N-oxide (Trimethylamine N-oxide), isopropanolamine (Isopropanolamine), hypotaurine (Hypotaurine), hydroxyproline (Hydroxyproline), N6-acetyllysine (N-epsilon-Acetyllysine), 6-hydroxyhexanoic acid (6-Hydroxyhexanoate), N-acetylputrescine (N-Acetylputrescine), azelaic acid (Azelate), dihydroxyacetonephosphoric acid (DHAP), glycolic acid (Glycolate), 4-methyl-2-oxopentanoic acid (4-Methyl-2-oxopentanoate), N-acetylaspartic acid (N-Acetylaspartate), glycerophosphoric acid (Glycerophosphate), 3-hydroxybutyric acid (3-Hydroxybutyrate), benzoic acid (Benzoate), adipic acid (Adipate), 2-isopropylmalate (2-Isopropylmalate), phosphorylchlorine (Phosphorylcholine), N-acetylneuraminic acid (N-Acetylneuraminate), histamine (His), o-acetylcarnitine (o-Acetylcarnitine), N-acetylglucosamine 1-phosphate (N-Acetylglucosamine 1-phosphate), creatinine (Creatinine), arginine (Arg), and syringic acid (Syringate), or a combination thereof.

9. The salivary biomarker for cancer according to embodiment 2, wherein the salivary biomarker is used for detection of breast cancer, and is a combination of N-acetylphenylalanine, N-acetylspermidine, and creatine.

10. The salivary biomarker for cancer according to embodiment 2, wherein the salivary biomarker is used for detection of oral cancer, and is any of glycyl-glycine (Gly-Gly), citrulline (Citrulline), γ-butyrobetaine (gamma-Butyrobetaine), 3-phenyllactate (3-Phenyllactate), butyric acid (Butanoate), hexanoic acid (Hexanoate), methionine (Met), hypoxanthine (Hypoxanthine), spermidine (Spermidine), tryptophan (Trp), aspartic acid (Asp), isopropanolamine (Isopropanolamine), alanyl-alanine (Ala-Ala), N,N-dimethylglycine (N,N-Dimethylglycine), N1-acetylspermidine (N1-Acetylspermidine), N1,N8-diacetylspermidine (N1,N8-Diacetylspermidine), N8-acetylspermidine (N8-Acetylspermidine), α-aminobutyric acid (2AB), trimethylamine-N-oxide (Trimethylamine N-oxide), N-acetylaspartic acid (N-Acetylaspartate), adenine (Adenine), 2-hydroxyvaleric acid (2-Hydroxyppentanoate), putrescine (Putrescine (1,4-Butanediamine)), 3-phosphoglycerate (3PG), 3-phenylpropionic acid (3-Phenylpropionate), serine (Ser), 1-methylnicotinamide (1-Methylnicotineamide), 3-hydroxy-3-methylglutaric acid (3-Hydroxy-3-methylglutarate), guanine (guanine), 3-(4-hydroxyphenyl)propionic acid (3-(4-Hydroxyphenyl)propionate), 4-methylbenzoate (4-Methylbenzoate), ribulose-5-phosphoric acid (Ru5P), α-aminoadipic acid (alpha-Aminoadipate), N6-acetyllysine (N-epsilon-Acetyllysine), glucosamine (Glucosamine), cystine (Cys), carnosine (Carnosine), urocanic acid (Urocanate), phenylalanine (Phe), 2-deoxyribose-1-phosphoric acid (2-Deoxyribose 1-phosphate), cytidine disodium 5'-monophosphate (CMP), p-hydroxyphenylacetic acid (p-Hydroxyphenylacetate), polyhydroxybutyric acid (3-Hydroxybutyrate), N-acetylputrescine (N-Acetylputrescine), 7-methylguanine (7-Methylguanine), inosine (Inosine), lysine (Lys), dihydroxyacetonephosphoric acid (DHAP), 3-methylhisdine (3-Methylhistidine), carbamoylaspartic acid (Carbamoylaspartate), creatinine (Creatinine), N-methyl-2-pyrrolidone (1-Methyl-2-pyrrolidinone), pyruvic acid (Pyruvate), propionic acid (Propionate), 5-aminovaleric acid (5-Aminovalerate), N-acetylornithine (N-Acetylornithine), 5-oxoproline (5-Oxoproline), creatine (Creatine), homoserine (Homoserine), fumaric acid (Fumarate), glycine (Gly), and N1,N12-diacetylspermine (N1,N12-Diacetylspermine), or a combination thereof.

11. A method for assaying a salivary biomarker for cancer comprising the steps of:

collecting a saliva sample; and
detecting the salivary biomarker for cancer according to any one of embodiments 1 to 10 in the collected saliva sample.

12. A device for assaying a salivary biomarker for cancer comprising:

means for collecting a saliva sample; and
means for detecting the salivary biomarker for cancer according to any one of embodiments 1 to 10 in the collected saliva sample.

13. A method for determining a salivary biomarker for cancer comprising steps of:

performing ultrafiltration of a saliva sample;
means for cyclopedically measuring ionic metabolites in the saliva sample after the ultrafiltration; and
selecting a substance having high ability of distinguishing a patient with a pancreatic disease from a healthy subject according to concentrations of the measured metabolites.

14. The method for determining a salivary biomarker for cancer according to embodiment 13, wherein during selection of the salivary biomarker for cancer, a correlation value between the measured metabolites is calculated, and a concentration of each substance is normalized with a concentration of a substance correlated with the most substances.

15. The method for determining a salivary biomarker for cancer according to embodiment 13 or 14, wherein a combination of the salivary biomarkers for cancer is determined using a mathematical model.

**Claims**

1. A salivary biomarker for use in detecting oral cancer, wherein the salivary biomarker is any of glycyl-glycine (Gly-Gly), citrulline (Citrulline), γ-butyrobetaine (gamma-Butyrobetaine), 3-phenyllactate (3-Phenyllactate), butyric acid (Butanoate), hexanoic acid (Hexanoate), methionine (Met), hypoxanthine (Hypoxanthine), spermidine (Spermidine), tryptophan (Trp), aspartic acid (Asp), isopropanolamine (Isopropanolamine), alanyl-alanine (Ala-Ala), N,N-dimethylglycine (N,N-Dimethylglycine), N1-acetylspermidine (N1-Acetylspermidine), N1,N8-diacetylspermidine (N1,N8-Diacetylspermidine), N8-acetylspermidine (N8-Acetylspermidine), α-aminobutyric acid (2AB), trimethylamine-N-oxide (Trimethylamine N-oxide), N-acetylaspartic acid (N-Acetylaspartate), adenine (Adenine), 2-hydroxyvaleric acid (2-Hydroxyppentanoate), putrescine (Putrescine (1,4-Butanediamine)), 3-phosphoglycerate (3PG), 3-phenylpropionic acid (3-Phenylpropionate), serine (Ser), 1-methylnicotinamide (1-Methylnicotineamide), 3-hydroxy-3-methylglutaric acid (3-Hydroxy-3-methylglutarate), guanine (guanine), 3-(4-hydroxyphenyl)propionic acid (3-(4-Hydroxyphenyl)propionate), 4-methylbenzoate (4-Methylbenzoate), ribulose-5-phosphoric acid (Ru5P), $\alpha$-aminoadipic acid (alpha-Aminoadipate), N6-acetyllysine (N-epsilon-Acetyllysine), glucosamine (Glucosamine), cystine (Cystine), carnosine (Carnosine), urocanic acid (Urocanate), phenylalanine (Phe), 2-deoxyribose-1-phosphoric acid (2-Deoxyribose 1-phosphate), cytidine disodium 5'-monophosphate (CMP), p-hydroxyphenylacetic acid (p-Hydroxyphenylacetate), polyhydroxybutyric acid (3-Hydroxybutyrate), N-acetylputrescine (N-Acetylputrescine), 7-methylguanine (7-Methylguanine), inosine (Inosine), lysine (Lys), dihydroxyacetonephosphoric acid (DHAP), 3-methylhisdine (3-Methylhistidine), carbamoylaspartic acid (Carbamoylaspartate), creatinine (Creatinine), N-methyl-2-pyrrolidone (1-Methyl-2-pyrrolidinone), pyruvic acid (Pyruvate), propionic acid (Propionate), 5-aminovaleric acid (5-Aminovalerate), N-acetylornithine (N-Acetylornithine), 5-oxoproline (5-Oxoproline), creatine (Creatine), homoserine (Homoserine), fumaric acid (Fumarate), glycine (Gly), and N1,N12-diacetylspermine (N1,N12-Diacetylspermine), or a combination thereof.

Fig.1

START

100
COLLECTION OF SALIVA

110
PRETREATMENT FOR MEASUREMENT
OF METABOLITES

120
MEASUREMENT OF ABSOLUTE CONCENTRATIONS
OF METABOLITES IN SALIVA

130
REMOVAL OF NOISE

140
SELECTION OF SUBSTANCE IN WHICH THE NUMBER
OF DETECTION IS LARGE IN EACH GROUP

150
SELECTION OF SUBSTANCE HAVING
STATISTICALLY SIGNIFICANT
DIFFERENCE BETWEEN GROUPS

142
SELECTION OF SUBSTANCE FOR
CONCENTRATION CORRECTION

152
SELECTION OF SUBSTANCE HAVING
STATISTICALLY SIGNIFICANT DIFFERENCE
AMONG SUBSTANCES AFTER
CONCENTRATION CORRECTION

END

Fig. 2

Fig.3

```
        ┌─────────────────────────┐
        │  PROCEDURE OF DEVELOPING │
        │    MATHEMATICAL MODEL    │
        └─────────────────────────┘
                     │
                                              200
        ┌─────────────────────────────────────┐
        │         START CONSTRUCTION OF        │
        │  MODEL FROM STATE WITHOUT VARIABLE   │
        └─────────────────────────────────────┘
                     │
        ┌───────────────────────────────────────┐
        └───────────────────────────────────────┘
                                              210
        ┌─────────────────────────────────────┐
        │     SELECT SMALL NUMBER OF MARKER    │
        │    BY VARIABLE SELECTION METHOD      │
        └─────────────────────────────────────┘
                     │
        ┌───────────────────────────────────────┐
        └───────────────────────────────────────┘
                                              220
        ┌─────────────────────────────────────┐
        │      DIVIDE DATA INTO LEARNING DATA  │
        │          AND EVALUATION DATA         │
        └─────────────────────────────────────┘
                     │                          230
        ┌─────────────────────────────────────┐
        │       FORM MODEL FROM LEARNING DATA  │
        │    AND EVALUATE WITH EVALUATION DATA │
        └─────────────────────────────────────┘
                     │
        ┌───────────────────────────────────────┐
        └───────────────────────────────────────┘
                                              240
        ┌─────────────────────────────────────┐
        │    COLLECT ALL PREDICTION RESULTS OF │
        │            EVALUATION DATA,          │
        │  AND CALCULATE AREA UNDER ROC CURVE  │
        │          ──► EVALUATE MODEL          │
        └─────────────────────────────────────┘
                     │
        ┌───────────────────────────────────────┐
        └───────────────────────────────────────┘
                                              250
        ┌─────────────────────────────────────┐
        │       SELECT MODEL HAVING THE        │
        │           HIGHEST ACCURACY           │
        │    AS RESULT OF CROSS VALIDATION     │
        └─────────────────────────────────────┘
```

LOOP 1

LOOP 2

Fig. 4

Fig.5

Fig.6

Fig.7

Fig.8

Fig.9

Fig.10

MLR MODEL USING $\beta$-ALA,
N-ACETYLPHENILALANINE,
AND CITRULLINE

Fig.11

MLR MODEL USING N-ACETYLPHENILALANINE,
N1-ACETYLPPERMIDINE,
AND CREATINE

Fig. 12

Ornithine

Succinate

2-Hydroxybutyrate

Pro

2AB

Lys

beta-Ala

Glu

Creatine

Ile

Gln

2-Hydroxy-4-methylpentanoate

Leu

2-Hydroxypentanoate

N-Acetylputrescine

Val

5-Aminovalerate

Ala

Sarcosine

gamma-Butyrobetaine

Putrescine(1,4-Butanediamine)

Gly

Carnitine

alpha-Aminoadipate

5-Oxoproline

Urocanate

Fig. 13

Fig. 14

Fig. 15

Fig. 16

Fig.17

Fig. 18

## Choline

AUC = 0.7800
(95% CI:0.6328 – 0.9272)
*P* = 0.002461 **

AUC = 0.7500
(95% CI:0.5901 – 0.9099)
*P* = 0.008535 **

AUC = 0.8528
(95% CI:0.7255 – 0.9800)
*P* = 0.0002071 ***

EP 3 578 984 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2011058863 A
- JP 2011232164 A
- WO 2011158590 A1
- JP 2011247869 A
- JP 2009508493 W
- JP 2013521763 A

### Non-patent literature cited in the description

- **HAMADA S ; SHIMOSEGAWA T.** *Biomarkers of pancreatic cancer, Pancreatology,* 2011, vol. 11, 14-9
- **SODA K.** The mechanisms by which polyamines accelerate tumor spread. *Journal of Experimental & Clinical Cancer Research,* 2011, vol. 30 (1), 95
- **JIN HT ; LAMSA T ; MERENTIE M ; HYVONEN MT ; SAND J ; RATY S ; HERZIG KH ; ALHONEN L ; NORDBACK I.** Polyamine levels in the pancreas and the blood change according to the severity of pancreatitis. *Pancreatology,* 2008, vol. 8 (1), 15-24
- **ZHANG L ; FARRELL JJ ; ZHOU H ; ELASHOFF D ; AKIN D ; PARK NH ; CHIA D ; WONG DT.** Salivary transcriptomic biomarkers for detection of resectable pancreatic cancer. *Gastroenterology,* 2010, vol. 138 (3), 949-57
- **SUGIMOTO M ; WONG DT ; HIRAYAMA A ; SOGA T ; TOMITA M.** Capillary electrophoresis mass spectrometry-based saliva metabolomics identified oral, breast and pancreatic cancer-specific profiles. *Metabolomics,* 2010, vol. 6, 78-95
- **SOGA, T. ; BARAN, R. ; SUEMATSU M. ; UENO, Y. ; IKEDA, S. ; SAKURAKAWA T. ; KAKAZU, Y. ; ISHIKAWA, T. ; ROBERT, M. ; NISHIOKA, T.** Differential methabolomics reveals ophthalmic acid as an oxidative stress biomarker indicating hepatic glutathione sonsumption. *Journal of Biological Chemistry,* 2006, vol. 281 (24), 16768-16776
- **SUGIMOTO et al.** Capillary electrophoresis mass spectrometry-based saliva metabolomics identified oral, breast and pancreatic cancer-specific profiles. *Metabolomics,* 2010, vol. 6, 78-95
- **TSUTSUI et al.** High-throughput LC-MS/MS based simultaneous determination of polyamines including N-acetylated forms in human saliva and the diagnostic approach to breast cancer patients. *Anal Chem,* 2013, vol. 85, 11835-42
- **WANG Q et al.** Investigation and identification of potential biomarkers in human saliva for the early diagnosis of oral squamous cell carcinoma. *Clin Chim Acta.,* 2014, vol. 427, 79-85
- **M. A. HALL.** *Correlation-based Feature Subset Selection for Machine Learning,* 1998
- An adaptation of Relief for attribute estimation in regression. **MARKO ROBNIK-SIKONJA ; IGOR KONONENKO.** International Conference on Machine Learning. 1997, 296-304
- **I. GUYON ; J. WESTON ; S. BARNHILL ; V. VAPNIK.** Gene selection for cancer classification using support vector machines. *Machine Learning,* 2002, vol. 46 (1-3), 389-422
- Statistical Decision Theory and Bayesian Analysis. **BERGER, JAMES O.** Springer Series in Statistics. Springer-Verlag, 1985
- **D. E. RUMELHART ; G. E. HINTON ; R. J. WILLIAMS.** Learning representaions by back-propagating errors. *Nature,* 1986, vol. 323-9, 533-536
- Fast Training of Support Vector Machines using Sequential Minimal Optimization. **J. PLATT.** Advances in Kernel Methods-Support Vector Learning. 1998
- **YOAV FREUND ; LLEW MASON.** The Alternating Decision Tree Algorithm. *Proceedings of the 16th International Conference on Machine Learning,* 1999, 124-133
- **FREUND, Y. ; MASON, L.** The alternating decision tree learning algorithm. *Proceeding of the Sixteenth International Conference on Machine Learning,* 1999, 124-133
- **ROSS QUINLAN.** C4.5: Programs for Machine Learning. Morgan Kaufmann Publishers, 1993
- **EIBE FRANK ; IAN H. WITTEN.** Generating Accurate Rule Sets Without Global Optimization. *Fifteenth International Conference on Machine Learning,* 1998, 144-151
- **LINDGREN, F ; GELADI, P ; WOLD, S.** The kernel algorithm for PLS. *J. Chemometrics,* 1993, vol. 7, 45-59
- **TRYGG, J. ; WOLD, S.** Orthogonal projections to latent structures (O-PLS). *Journal of Chemometrics,* 2002, vol. 16 (3), 119-128

- **BREIMAN, LEO.** Random Forests. *Machine Learning,* 2001, vol. 45 (1), 5-32
- **BREIMAN, LEO.** Bagging predictors. *Machine, Learning,* 1996, vol. 24 (2), 123-140
- **HOTELLING, H.** Analysis of a complex of statistical variables into principal components. *Journal of Educational Psychology,* 1933, vol. 24, 417-441
- **FU NN ; ZHANG HS ; MAM ; WANG H.** Quantification of polyamines in human erythrocytes using a new near-infrared cyanine 1-(epsilon-succinimidyl-hexanoate)-1'-methyl-3,3,3',3'-tetramethyl-indocarbocyanine-5,5'-dis ulfonate potassium with CE-LIF detection. *Electrophoresis,* 2007, vol. 28 (5), 822-9
- **BYUN JA ; LEE SH ; JUNG BH ; CHOI MH ; MOON MH ; CHUNG BC.** Analysis of polyamines as carbamoyl derivatives in urine and serum by liquid chromatography-tandem mass spectrometry. *Biomed Chromatogr.,* 2008, vol. 22 (1), 73-80
- **STOREY, J. D. ; TIBSHIRANI, R.** Statistical significance for genomewide studies. *Proceedings of the National academy of Sciences of the United States of America,* 2003, vol. 100, 9440-9445